(19) Europäisches Patentamt
European Patent Office
Office européen des brevets

(11) **EP 3 658 913 B1**

(12) **FASCICULE DE BREVET EUROPEEN**

(45) Date de publication et mention de la délivrance du brevet:
**03.04.2024 Bulletin 2024/14**

(21) Numéro de dépôt: **18766314.1**

(22) Date de dépôt: **27.07.2018**

(51) Classification Internationale des Brevets (IPC):
***G01N 33/542*** *(2006.01)* ***G01N 33/566*** *(2006.01)*

(52) Classification Coopérative des Brevets (CPC):
**G01N 33/542; G01N 33/566;** G01N 2333/4719; G01N 2333/726; G01N 2500/02

(86) Numéro de dépôt international:
**PCT/FR2018/051948**

(87) Numéro de publication internationale:
**WO 2019/020964 (31.01.2019 Gazette 2019/05)**

(54) **MÉTHODE POUR MESURER LA MODULATION DE L'ACTIVATION D'UN RÉCEPTEUR COUPLÉ À UNE PROTÉINE G**

VERFAHREN ZUR MESSUNG DER MODULATION DER AKTIVITÄT EINES G-PROTEIN-GEKOPPELTEN REZEPTORS

METHOD FOR MEASURING MODULATION IN THE ACTIVITY OF A G PROTEIN-COUPLED RECEPTOR

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **28.07.2017 FR 1757263**

(43) Date de publication de la demande:
**03.06.2020 Bulletin 2020/23**

(73) Titulaires:
- **CISBIO BIOASSAYS**
  **30200 Codolet (FR)**
- **Centre National de la Recherche Scientifique**
  **75016 Paris (FR)**

(72) Inventeurs:
- **ROUX, Thomas, Fabien, Michel**
  **30900 Nimes (FR)**
- **DA SILVA, Mélanie**
  **34090 Montpellier (FR)**
- **DUPUIS, Elodie, Julie**
  **30132 Caissargues (FR)**
- **TRINQUET, Eric, Jacques, Christian**
  **30130 Pont-Saint-Esprit (FR)**
- **SOULE, Julien, Jean-Marius**
  **34090 Montpellier (FR)**
- **RONDARD, Philippe**
  **34980 Saint-Gely-Du-Fesc (FR)**
- **PIN, Jean-Philippe, R.**
  **34000 Montpellier (FR)**

(74) Mandataire: **Cabinet Beau de Loménie**
**158, rue de l'Université**
**75340 Paris Cedex 07 (FR)**

(56) Documents cités:
**WO-A1-2004/035614**

- **CHRISTOPHER A. JOHNSTON ET AL: "Minimal Determinants for Binding Activated G[alpha] from the Structure of a G[alpha] i1 -Peptide Dimer", BIOCHEMISTRY, vol. 45, no. 38, 1 septembre 2006 (2006-09-01), pages 11390-11400, XP055457282, US ISSN: 0006-2960, DOI: 10.1021/bi0613832**
- **Pauline Scholler ET AL: "Time-Resolved Förster Resonance Energy Transfer-Based Technologies to Investigate G Protein-Coupled Receptor Machinery" In: "Progress in molecular biology and translational science", 1 janvier 2013 (2013-01-01), Elsevier, Netherlands, XP055456461, ISSN: 1877-1173 vol. 113, pages 275-312, DOI: 10.1016/B978-0-12-386932-6.00007-7, le document en entier**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

- **YU B ET AL: "Inhibition of subsets of G protein-coupled receptors by empty mutants of G protein alpha subunits in g(o), G(11), and G(16).", THE JOURNAL OF BIOLOGICAL CHEMISTRY 07 JAN 2000, vol. 275, no. 1, 7 January 2000 (2000-01-07), pages 71-76, ISSN: 0021-9258**
- **ANDHIRKA SAI KRISHNA ET AL: "The nucleotide-free state of heterotrimeric G proteins [alpha]-subunit adopts a highly stable conformation.", THE FEBS JOURNAL 08 2017, vol. 284, no. 15, August 2017 (2017-08), pages 2464-2481, ISSN: 1742-4658**


Remarques:

Le document complet y compris le(s) table(s) de référence et le(s) listage(s) de séquence sont téléchargeables sur le site internet de l’OEB

## Description

### Domaine technique

L'invention concerne un nouveau procédé pour mesurer la modulation de l'activation d'un récepteur couplé à une protéine G (RCPG ou GPCR en anglais), par exemple un procédé pour déterminer la capacité d'une molécule à moduler l'activation d'un RCPG. Le procédé selon l'invention permet notamment de détecter l'apparition ou la disparition d'une protéine G vide ou d'une protéine G pleine dans une préparation de RCPG.

### Arrière-plan technologique

Les récepteurs couplés aux protéines G (RCPG ou GPCR en anglais) sont une famille de récepteurs membranaires chez les mammifères et dans tout le règne animal. Les protéines G sont des protéines hétérotrimériques (3 sous unités : alpha, bêta et gamma) qui sont activées par les RCPG. Au travers des RCPG, les protéines G ont un rôle de transduction d'un signal de l'extérieur de la cellule vers l'intérieur de la cellule (i.e. réponse cellulaire à un stimulus externe). Leur mécanisme d'action communément décrit est présenté dans la Figure 1 et résumé ci-après :

- dans son état inactif, de repos, la sous unité alpha de la protéine G est liée au nucléotide GDP (protéine G pleine liée au GDP) ;
- après activation du RCPG, ce dernier se lie à la sous unité alpha de la protéine G et enclenche un processus d'activation de la protéine G constitué de deux étapes : 1) la sortie du GDP de la protéine G pour donner une protéine G vide, et la formation d'un complexe RCPG / protéine-G-vide inactif, et 2) la fixation du GTP qui conduit à la formation d'une protéine G active, sous forme GTP (protéine G pleine liée au GTP). Dans la première étape, la protéine G liée au récepteur est sous une forme appelée « forme vide ». Cet état est décrit dans la littérature comme étant transitoire puisqu'il est décrit que le nucléotide GTP se lie rapidement à la sous unité alpha de la protéine G. Par ailleurs, les sous unités bêta/gamma de la protéine G activée se dissocient de la sous unité alpha ;
- la sous unité alpha de la protéine G pleine liée au GTP se fixe alors aux effecteurs pour les activer. Les effecteurs activent à leur tour des voies de signalisation entraînant une réponse cellulaire ;
- le GTP est ensuite hydrolysé en GDP par la sous unité alpha de la protéine G et la sous unité alpha se réassocie aux sous unité bêta/gamma pour reformer la protéine G pleine liée au GDP (état inactif).

Il existe plusieurs sous types de protéines G alpha présentant des profils de sélectivité différents pour les différents effecteurs et induisant ainsi l'activation de voies de signalisation préférentielles.

Les RCPG sont associés à de nombreuses fonctions physiologiques importantes et sont considérés comme l'une des cibles thérapeutiques privilégiées pour un grand nombre de pathologies. Ainsi, de nombreux tests de criblage *in vitro* ont été développés pour identifier des molécules capables de moduler les RCPG. Les tests mis au point exploitent différents mécanismes d'activation des protéines G et mettent en oeuvre des technologies variées (Zhang et al. ; Tools for GPCR drug discovery ; (2012) Acta Pharmacologica Sinica).

On peut citer notamment les tests d'affinité qui utilisent des ligands radiomarqués pour mesurer l'affinité du ligand au RCPG, les tests de scintigraphie de proximité qui utilisent des billes de scintigraphie sur lesquelles des RCPG ont été fixés ou les tests fonctionnels utilisant du GTP faiblement ou non hydrolysable comme le GTP$\gamma$S. Ces tests sont néanmoins difficiles à mettre en oeuvre et nécessitent parfois des étapes de filtration sur membrane qui peut limiter leur utilisation comme tests de criblage à haut débit (HTS).

D'autres tests ont été développés pour mettre en évidence l'activation des RCPG. Ces tests se basent notamment sur les techniques de transfert d'énergie (RET - acronyme anglais de « Résonance Energy Transfer »), comme le FRET (acronyme anglais de « Fluorescence Resonance Energy Transfer ») ou le BRET (acronyme anglais de

« Bioluminescence Resonance Energy Transfer »). On peut citer par exemple les techniques de transfert d'énergie mettant en évidence l'interaction entre un RCPG et la protéine G en utilisant soit un donneur fusionné au RCPG et un accepteur fusionné à la protéine G (WO 2006/086883 et WO 2003/008435) soit un accepteur fusionné à la sous-unité alpha de la protéine G et un donneur fusionné à la sous-unité bêta et/ou gamme de la protéine G (Bunemann et al. Proc. Natl. Acad. Sci., 2003, 26, 16077-16082). Ces techniques sont néanmoins contraignantes puisqu'elles nécessitent la préparation de protéines de fusion et elles ne permettent pas d'étudier les RCPG et les protéines G exprimés de façon endogène par les cellules (i.e. non modifiés et non sur-exprimés). D'autre part, afin de discriminer les différents sous types de protéines G alpha pouvant être activées par le récepteur, ces techniques nécessitent la préparation d'échantillons membranaires multiples (une préparation spécifique pour chaque sous type de protéine G alpha).

Les techniques de transfert d'énergie ont également été utilisées pour la mise au point de tests visant à visualiser la modulation de la forme GTP (active) de la protéine G ou de la forme GDP (inactive) de la protéine G. On peut tout d'abord citer par exemple l'utilisation d'un format avec la protéine G fusionnée à une étiquette biotine ainsi liée à un

donneur lui-même couplé à une protéine streptavidine et un accepteur lié à un analogue GTP non hydrolysable ou lentement hydrolysable (WO 2006/035208). D'autre part, un autre format utilise des peptides BioKey® biotinylé (KaroBio) discriminant la forme GTP de la forme GDP et qui sont liés à un donneur grâce à une protéine streptavidine couplée au donneur. L'accepteur est lié au RCPG, qui est fusionné avec une étiquette 6HIS, à l'aide d'un anticorps anti 6HIS (WO 2004/035614). Ces techniques sont également contraignantes puisqu'elles nécessitent aussi la préparation de protéines de fusion et elles ne permettent pas d'étudier les RCPG et les protéines G exprimés de façon endogène par les cellules. De même, afin de discriminer les différents sous type de protéines G alpha pouvant être activées par le récepteur, ces techniques nécessitent la préparation d'échantillons membranaires multiples.

Enfin, un dernier format à base de transfert d'énergie utilise les peptides BioKey® biotinylés (Karobio) décrits ci-dessus avec de la streptavidine couplée à un donneur et un analogue GTPγS lié à un accepteur (Frang et al., https://shop.perkinelmer.com/Content/relatedmaterials/posters/sps_006943gtplance.pdf). Ce dernier format ne permet toutefois pas de discriminer la forme de la protéine G non associée (protéine G liée à un nucléotide GDP ou GTP) de la forme associée au RCPG lorsque celui est activé par un composé (protéine G vide).

Il existe donc un réel besoin de disposer d'une méthode sensible et fiable qui permet de déterminer facilement la modulation de l'activation d'un RCPG, par exemple pour déterminer facilement la capacité d'une molécule à moduler l'activation d'un RCPG, et/ou de déterminer quelle sous type de protéine G est activé par le RCPG.

**Résumé de l'invention**

La présente invention vise à proposer une nouvelle méthode pour le criblage *in vitro* de molécules capables de moduler les RCPG. Cette nouvelle méthode est notamment basée sur la capacité à discriminer une forme pleine de protéine G (Protéine G pleine liée au GTP ou protéine G pleine liée au GDP) et une forme vide de protéine G, approche qui à la connaissance des inventeurs n'a jamais été utilisée pour mesurer l'activation d'un RCPG.

En particulier la présente invention a comme avantages 1) d'utiliser une méthode de détection à base de fluorescence donc non radioactive ; 2) de ne nécessiter aucune étape de lavage et ainsi de simplifier sa mise en oeuvre notamment pour des activités de criblage de composés à haut débit ; 3) de permettre de travailler notamment sur des protéines G et RCPG non modifiés ; 4) de permettre la discrimination de différents sous-types de protéines Galpha activées par le RCPG dans une même préparation membranaire comportant ces différents sous types (la discrimination étant apportée par l'utilisation de ligands de détection discriminant les sous-types de protéines Galpha).

Selon un premier aspect, l'invention concerne une méthode pour déterminer la capacité d'une molécule à moduler l'activation d'un récepteur couplé à une protéine G (RCPG), ladite méthode comprenant les étapes suivantes :

a) introduction, dans un premier contenant :

- d'une préparation membranaire portant un ou plusieurs RCPG et une ou plusieurs protéine(s) G,
- d'une source de GDP ou d'une source de GTP,
- d'un premier ligand de la sous-unité alpha d'une protéine G (protéine Galpha) marqué par un premier membre d'un couple de partenaires de RET, et d'un second ligand de la protéine Galpha marqué par un second membre du couple de partenaires de RET, lesdits ligands étant capables de se lier spécifiquement, soit individuellement soit en association, à la protéine Galpha qui n'est pas liée au GTP ou au GDP ou au GTP non hydrolysable ou lentement hydrolysable (protéine Galpha vide) ;

b) mesure du signal de RET émis dans le premier contenant ;
c) introduction (i) dans un second contenant, des mêmes réactifs qu'à l'étape a) et de la molécule à tester ou (ii) dans le premier contenant, de la molécule à tester ;
d) mesure du signal de RET émis dans le second contenant ou dans le premier contenant obtenu à l'étape c) ;
e) comparaison des signaux obtenus aux étapes b) et d) ;

- une augmentation du signal quand on utilise une source de GDP et des ligands capables de se lier spécifiquement à la protéine Galpha vide indiquant que la molécule est un agoniste du RCPG ;
- une diminution du signal quand on utilise une source de GDP et des ligands capables de se lier spécifiquement à la protéine Galpha vide indiquant que la molécule est un agoniste inverse du RCPG ;
- une augmentation du signal quand on utilise une source de GTP et des ligands capables de se lier spécifiquement à la protéine Galpha vide indiquant que la molécule est un agoniste du RCPG ;
- une diminution du signal quand on utilise une source de GTP et des ligands capables de se lier spécifiquement à la protéine Galpha vide indiquant que la molécule est un agoniste inverse du RCPG ;

caractérisée en ce que le premier ligand est choisi parmi un anticorps, un fragment d'anticorps, un peptide ou un

aptamère et le second ligand est choisi parmi un anticorps, un fragment d'anticorps ou un aptamère.

Selon un deuxième aspect, l'invention concerne une méthode pour déterminer la capacité d'une molécule à moduler l'activation d'un récepteur couplé à une protéine G (RCPG), ladite méthode comprenant les étapes suivantes :

a) introduction, dans un premier contenant :

- d'une préparation membranaire portant un ou plusieurs RCPG et une ou plusieurs protéine(s) G,
- d'une source de GDP ou d'une source de GTP,
- d'un premier ligand de la sous-unité alpha d'une protéine G (protéine Galpha) marqué par un premier membre d'un couple de partenaires de RET, et d'un second ligand de la protéine Galpha marqué par un second membre du couple de partenaires de RET, lesdits ligands étant capables de se lier spécifiquement, soit individuellement soit en association, à la protéine Galpha qui n'est pas liée au GTP ou au GDP ou au GTP non hydrolysable ou lentement hydrolysable (protéine Galpha vide) ;
- d'un agoniste du RCPG ;

b) mesure du signal de RET émis dans le premier contenant ;
c) introduction dans un second contenant, des mêmes réactifs qu'à l'étape a) et de la molécule à tester ;
d) mesure du signal de RET émis dans le second contenant obtenu à l'étape c) ;
e) comparaison des signaux obtenus aux étapes b) et d);

- une augmentation du signal quand on utilise une source de GDP et des ligands capables de se lier spécifiquement à la protéine Galpha vide indiquant que la molécule est un agoniste ou un modulateur allostérique positif du RCPG ;
- une augmentation du signal quand on utilise une source de GTP et des ligands capables de se lier spécifiquement à la protéine Galpha vide indiquant que la molécule est un agoniste ou un modulateur allostérique positif du RCPG ;
- une diminution du signal quand on utilise une source de GDP et des ligands capables de se lier spécifiquement à la protéine Galpha vide indiquant que la molécule est un antagoniste ou un modulateur allostérique négatif du RCPG ;
- une diminution du signal quand on utilise une source de GTP et des ligands capables de se lier spécifiquement à la protéine Galpha vide indiquant que la molécule est un antagoniste ou un modulateur allostérique négatif du RCPG ;

caractérisée en ce que le premier ligand est choisi parmi un anticorps, un fragment d'anticorps, un peptide ou un aptamère et le second ligand est choisi parmi un anticorps, un fragment d'anticorps ou un aptamère.

Selon un troisième aspect, l'invention concerne un kit pour la mise en oeuvre de la méthode selon l'invention comprenant :

- un premier ligand de la sous-unité alpha d'une protéine G (protéine Galpha) marqué par un premier membre d'un couple de partenaires de RET et un second ligand de la protéine Galpha marqué par un second membre du couple de partenaires de RET, lesdits ligands étant capables de se lier spécifiquement, soit individuellement soit en association, à la protéine Galpha qui n'est pas liée au GTP ou au GDP ou au GTP non hydrolysable ou lentement hydrolysable (protéine Galpha vide) ; et
- une notice d'instructions ;
- éventuellement un ou des tampon(s) de dilution pour les réactifs ;
- une source de GDP ou une source de GTP ;
- éventuellement une préparation membranaire portant un ou plusieurs RCPG et/ou une ou plusieurs protéine(s) ; et
- éventuellement une protéine Galpha ;

caractérisé en ce que le premier ligand est choisi parmi un anticorps, un fragment d'anticorps, un peptide ou un aptamère et le second ligand est choisi parmi un anticorps, un fragment d'anticorps ou un aptamère.

**Brève description des figures**

La **figure 1** représente le mécanisme d'action d'activation d'un RCPG et de la protéine G.

La **figure 2** représente le concept de détection de l'activation du RCPG et de la protéine G associée par la discrimination d'une forme initiale de la protéine G pleine (liée au GDP ou au GTP ou au GTP non hydrolysable / lentement hydrolysable) d'une forme finale vide (pas de nucléotide).

Les **figures 3A à 3D** illustrent 2 formats d'essai selon l'invention (format 1A et 2A) et 2 formats d'essai qui ne sont pas selon l'invention (format 1B et 2B).

Les **figures 4A et 4B** illustrent un test d'activation selon le format 2B, qui n'est pas selon l'invention, avec le couple de ligands de détection : Peptide Biotin-KB1753/SA-XL + anticorps anti Galphai SC13533-Lumi4Tb.

Les **figures 5A et 5B** illustrent un test d'activation selon le format 2B, qui n'est pas selon l'invention, avec le couple de ligands de détection : anticorps anti Galphai DSV36S-d2 + anticorps anti Galphai SC13533-Lumi4Tb.

Les **figures 6A et 6B** illustrent un test d'activation selon le format 2B, qui n'est pas selon l'invention, avec le couple de ligands de détection : anticorps anti Galphai DSV39S-d2 + anticorps anti Galphai SC13533-Lumi4Tb.

Les **figures 7A et 7B** illustrent un test d'activation selon le format 2B, qui n'est pas selon l'invention, avec le couple de ligands de détection : anticorps anti Galphai DSV3S-d2 + anticorps anti Galphai DSV36S-Lumi4Tb.

Les **figures 8A et 8B** illustrent un test d'activation selon le format 1A avec le couple de ligands de détection : anticorps anti Galphai DSV39S-d2 + anticorps anti Galphai DSV26S-Lumi4Tb.

Les **figures 9A et 9B** illustrent un test d'activation selon le format 2A avec le couple de ligands de détection : anticorps anti Galphai DSV39S-d2 + anticorps anti Galphai DSV26S-Lumi4Tb.

La **figure 10** illustre un test d'activation selon le format 2B, qui n'est pas selon l'invention, avec le couple de ligands de détection : peptide Biotin-KB1753/SA-XL + anticorps anti Galphai SC13533-Lumi4Tb, avec validation du format d'essai sur quatre RCPG différents (Delta Opioïde, GalanineR1, NOP, 5HT1A) et sur deux fonds cellulaires différents (HEK293 et CHO-K1).

La **figure 11** illustre un test d'activation selon le format 2A avec le couple de ligands de détection : anticorps anti Galphai DSV39S-d2 + anticorps anti Galpai DSV26S-Lumi4Tb, avec validation du format d'essai avec du GTPγS ou du GTP.

Les **figures 12A et 12B** illustrent un test d'activation selon le format 2B, qui n'est pas selon l'invention, avec le couple de ligands de détection : anticorps anti Galphai DSV36S-Lumi4Tb + anticorps anti Galphai SC13533-d2.

Les **figures 13A et 13B** illustrent un test d'activation selon le format 2B, qui n'est pas selon l'invention, avec le couple de ligands de détection : anticorps anti Galphai sdAb F11-d2 + anticorps anti Galphai SC13533-Lumi4Tb.

Les **figures 14A et 14B** illustrent un test d'activation selon le format 2B, qui n'est pas selon l'invention, avec le couple de ligands de détection : anticorps anti Galphai sdAb F4-d2 + anticorps anti Galphai SC13533-Lumi4Tb.

Les **figures 15A et 15B** illustrent un test d'activation selon le format 2B, qui n'est pas selon l'invention, avec le couple de ligands de détection : anticorps anti Galphai sdAb G6-d2 + anticorps anti Galphai SC13533-Lumi4Tb.

Les **figures 16A et 16B** illustrent un test d'activation selon le format 2B, qui n'est pas selon l'invention, avec le couple de ligands de détection : anticorps anti Galphai sdAb B11-d2 + anticorps anti Galphai SC13533-Lumi4Tb.

Les **figures 17A et 17B** illustrent un test d'activation selon le format 2B, qui n'est pas selon l'invention, avec le couple de ligands de détection : anticorps anti Galphai sdAb F9-d2 + anticorps anti Galphai SC13533-Lumi4Tb.

Les **figures 18A et 18B** illustrent un test d'activation selon le format 2B, qui n'est pas selon l'invention, avec le couple de ligands de détection : anticorps anti Galphai sdAb G7-d2 + anticorps anti Galphai SC13533-Lumi4Tb.

Les **figures 19A et 19B** illustrent un test d'activation selon le format 2B, qui n'est pas selon l'invention, avec le

couple de ligands de détection : anticorps anti Galphai sdAb A10-d2 + anticorps anti Galphai DSV36S-Lumi4Tb.

La **figure 20A** illustre la capacité du couple de ligands de détection anticorps anti Galphai sdAb E2-d2 + anticorps anti Galphai SC13533-Lumi4Tb à détecter spécifiquement la forme pleine GTPgS de la protéine Galpha par rapport à la forme vide de la protéine Galpha.

**Description détaillée de l'invention**

Définitions

Au sens de l'invention, le terme « protéine G » désigne une protéine hétéro-trimérique composée de trois sous-unités appelées protéine Galpha, protéine Gbêta et protéine Ggamma.

Au sens de l'invention, le terme « Protéine Galpha » ou « Galpha » désigne la sous-unité alpha de la protéine G. La protéine Galpha possèdent deux domaines, le domaine GTPase, et le domaine hélice alpha. Il existe au moins 20 protéines Galpha différentes, qui peuvent se classer parmi les principales familles de protéines suivantes : Galphas (connu pour activer l'adénylate cyclase afin d'augmenter la synthèse de l'AMPc), Galphai (connu pour inhiber l'adénylate cyclase), Galphaolf (associé aux récepteurs olfactifs), Galphat (connu pour la transduction des signaux visuels dans la rétine en conjonction avec la rhodopsine), Galphaq (connu pour stimuler la phospholipase C) ou la famille Galpha12/13 (connue pour réguler le cytosquelette, les jonctions cellulaires, et d'autres processus liés au mouvement de la cellule). Dans un mode de réalisation préféré de l'invention, la protéine Galpha est choisie parmi la protéine Galphai1, Galphai2, Galphai3, Galphao1, Galphao2, Galphaq, Galpha12, Galpha13, Galphas, Galphaz, Galphat1, Galphat2, Galpha11, Galpha14, Galpha15, Galpha16 et Galphagus, de préférence choisie parmi la protéine Galphai1, Galphai2 et Galphai3.

Au sens de l'invention, le terme « Protéine Galpha pleine » désigne une protéine Galpha liée au GTP ou au GTP non hydrolysable ou lentement hydrolysable ou au GDP. Ce terme désigne donc à la fois la protéine Galpha liée au GDP (« Protéine Galpha pleine liée au GDP ») et la protéine Galpha liée au GTP ou au GTP non hydrolysable ou lentement hydrolysable (« Protéine Galpha pleine liée au GTP »). La protéine Galpha pleine (liée au GDP ou au GTP) est représentée à la Figure 1.

Le terme « GDP » désigne la guanosine diphosphate.

Le terme « GTP » désigne la guanosine triphosphate.

Le terme « source de GTP » désigne un composé qui apporte du GTP et/ou un GTP non-hydrolysable ou lentement hydrolysable, par exemple le GTP en tant que tel et/ou un GTP non-hydrolysable ou lentement hydrolysable en tant que tel.

Le terme « source de GDP » désigne un composé qui apporte du GDP, par exemple le GDP en tant que tel.

Le terme « GTP non hydrolysable ou lentement hydrolysable » désigne un analogue du GTP qui n'est pas hydrolysé ou peu hydrolysé en GDP. On peut citer par exemple le GTPgammaS (CAS no. 37589-80-3), le GppNHp (CAS no. 148892-91-5) ou le GppCp (CAS no. 10470-57-2). Les termes « GTPgS » ou « GTPγS » sont des abréviations du terme « GTPgammaS ».

Au sens de l'invention, le terme « Protéine Galpha vide » désigne une protéine Galpha qui n'est pas liée au GTP ou au GDP ou au GTP non hydrolysable ou lentement hydrolysable. La protéine Galpha vide est décrite dans la littérature comme un état transitoire entre la forme pleine liée au GDP et la forme pleine liée au GTP ou au GTP non hydrolysable ou lentement hydrolysable. La protéine Galpha vide est représentée à la Figure 1.

Au sens de l'invention, le terme « préparation membranaire » désigne une préparation comprenant des membranes cellulaires ou des fragments de membranes cellulaires ou des systèmes artificiels mimant des membranes cellulaires qui portent (ou qui expriment à leur surface) un ou plusieurs RCPG et une ou plusieurs protéine(s) Galpha. Ainsi, le terme « préparation membranaire » englobe les cellules entières, les cellules entières perméabilisées, les cellules lysées, les membranes cellulaires purifiées et les complexes RCPG / Protéine Galpha purifiés et reconstitués dans des nanodisques (aussi appelés « nanoscale phospholipid bilayers ») ou des mélanges de détergents qui portent (ou qui expriment à leur surface) un ou plusieurs RCPG et une ou plusieurs protéine(s) Galpha.

Un « anticorps » selon l'invention peut être d'origine mammifère (ex. humain ou de souris ou de camélidé), humanisé, chimérique, recombinant. Il s'agit de préférence d'un anticorps monoclonal produit de manière recombinante par des cellules génétiquement modifiées selon des techniques largement connues de l'homme de l'art. L'anticorps peut être de n'importe quel isotype, par exemple IgG, IgM, IgA, IgD ou IgE.

Un « fragment d'anticorps » selon l'invention peut par exemple être choisi parmi les fragments Fv, Fab, Fab', Fab'-SH, $F(ab')^2$, les diabodies, les « Single Domain Antibodies », les anticorps avec une seule chaine (ex. les scFv).

Les termes « anticorps à domaine unique », « Single domain Antibody » et « sdAb » sont interchangeables et font référence à un anticorps dans lequel la liaison à l'antigène est effectuée par un seul domaine variable. Un sdAb peut être i) un anticorps comprenant ou consistant en un domaine variable de chaîne lourde (VH) ou un fragment de celui-ci capable de se lier à l'antigène, qui se lie à l'antigène indépendamment de tout autre domaine variable, ii) un

anticorps comprenant ou consistant en un domaine variable de chaîne légère (VL) ou un fragment de celui-ci capable de se lier à l'antigène, qui se lie à l'antigène indépendamment de tout autre domaine variable, ou ii) un anticorps comprenant ou consistant en un domaine variable de chaîne lourde de type $V_HH$ ($V_HH$) ou un fragment de celui-ci capable de se lier à l'antigène, qui se lie à l'antigène indépendamment de tout autre domaine variable.

Au sens de l'invention, les termes « ligand capable de se lier spécifiquement à la forme vide » et « ligand capable de se lier spécifiquement à la forme pleine » désignent respectivement un ligand capable de se lier préférentiellement à la forme vide ou à la forme pleine de la protéine G. C'est à dire un ligand présentant respectivement une meilleure affinité (i.e un Kd plus petit) pour la forme vide ou la forme pleine de la protéine G vis-à-vis respectivement de la forme pleine ou de la forme vide de la protéine G. Le facteur de sélectivité (rapport du Kd pour la forme préférentiellement reconnue sur le Kd pour l'autre forme) est, par exemple, au moins d'un facteur deux.

Au sens de l'invention, les termes « ligands capables de se lier spécifiquement, en association, à la protéine Galpha vide » et « ligands capables de se lier spécifiquement, en association, à la protéine Galpha pleine » désignent respectivement :

1) une paire de ligands capable de se lier préférentiellement à une forme vide de la protéine Galpha, c'est à dire une paire de ligands dont au moins un des deux ligands présente, seul ou en association avec le deuxième ligand, une meilleure affinité (i.e un Kd plus petit) pour la forme vide de la protéine Galpha vis-à-vis de la forme pleine de la protéine Galpha. Le facteur de sélectivité (rapport du Kd pour la forme vide sur le Kd pour la forme pleine) est, par exemple, au moins d'un facteur deux.

2) une paire de ligands capable de se lier préférentiellement à une forme pleine de la protéine Galpha, c'est à dire une paire de ligands dont au moins un des deux ligands présente, seul ou en association avec le deuxième ligand, une meilleure affinité (i.e un Kd plus petit) pour la forme pleine de la protéine Galpha vis-à-vis de la forme vide de la protéine Galpha. Le facteur de sélectivité (rapport du Kd pour la forme pleine sur le Kd pour la forme vide) est, par exemple, au moins d'un facteur deux.

Il peut également s'agir d'une paire de ligands capable de générer un signal de RET plus élevé avec une forme donnée de la protéine Galpha vis-à-vis de l'autre forme de la protéine Galpha. Le rapport de signal de RET (rapport du signal de RET pour la forme préférentiellement reconnue sur le signal de RET pour l'autre forme) est, par exemple, au moins d'un facteur 1.3.

Au sens de l'invention, le terme « molécule capable de moduler l'activation du RCPG » désigne une molécule capable d'activer ou d'inhiber un RCPG, et donc d'induire la transduction ou d'empêcher la transduction d'un signal de l'extérieur de la cellule vers l'intérieur de la cellule via le RCPG. Il peut s'agir d'un agoniste, d'un antagoniste, d'un agoniste inverse, d'un modulateur allostérique positif ou d'un modulateur allostérique négatif.

Au sens de l'invention, le terme « molécule à tester » est une molécule susceptible de moduler l'activation du RCPG.

Dans la présente description, le terme « molécule », sans autre précision, désigne à la fois les termes « molécule capable de moduler l'activation du GPCR » et « molécule à tester ».

Le terme « RET » (de l'anglais « Resonance Energy Transfer ») désigne les techniques de transfert d'énergie.

Le terme « FRET » (de l'anglais « Fluorescence Resonance Energy Transfer ») désigne le transfert d'énergie entre deux molécules fluorescentes. Le FRET est défini comme un transfert d'énergie non radiatif résultant d'une interaction dipôle-dipôle entre un donneur et un accepteur d'énergie. Ce phénomène physique nécessite une compatibilité énergétique entre ces molécules. Cela signifie que le spectre d'émission du donneur doit recouvrir, au moins partiellement, le spectre d'absorption de l'accepteur. En accord avec la théorie de Förster, le FRET est un processus qui dépend de la distance séparant les deux molécules, donneur et accepteur : lorsque ces molécules sont à proximité l'une de l'autre, un signal de FRET sera émis.

Le terme « BRET » (de l'anglais « Bioluminescence Resonance Energy Transfer ») désigne le transfert d'énergie entre une molécule bioluminescente et une molécule fluorescente.

Au sens de l'invention, le terme « ligand » désigne une molécule capable de se lier spécifiquement et de manière réversible à une molécule cible. Dans le cadre de l'invention, la molécule cible est la protéine Galpha vide ou la protéine Galpha pleine. Le ligand peut être de nature protéique (ex. une protéine ou un peptide) ou de nature nucléotidique (ex. un ADN ou un ARN). Dans le cadre de l'invention, chaque ligand est avantageusement choisi parmi un anticorps, un fragment d'anticorps, un peptide ou un aptamère.

Au sens de l'invention, le ligand est marqué par un membre d'un couple de partenaires de RET. Le ligand peut être marqué de manière directe ou indirecte selon des méthodes bien connues de l'homme du métier, par exemple comme décrit ci-après, mais de manière préférée, le ligand est marqué de manière directe, par liaison covalente avec un membre d'un couple de partenaires de RET.

Le terme « couple de partenaires de RET » désigne un couple constitué d'un composé donneur d'énergie (ci-après « composé donneur ») et d'un composé accepteur d'énergie (ci-après « composé accepteur »); lorsqu'ils sont à

proximité l'un de l'autre et lorsqu'ils sont excités à la longueur d'onde d'excitation du composé donneur, ces composés émettent un signal de RET. Il est connu que pour que deux composés soient partenaires de RET, le spectre d'émission du composé donneur doit recouvrir partiellement le spectre d'excitation du composé accepteur. Par exemple, on parle de « couples de partenaires de FRET » lorsqu'on utilise un composé fluorescent donneur et un composé accepteur ou de « couple de partenaires de BRET » lorsqu'on utilise un composé bioluminescent donneur et un composé accepteur.

Le terme « signal de RET » désigne tout signal mesurable représentatif d'un RET entre un composé donneur et un composé accepteur. Par exemple, un signal de FRET peut donc être une variation de l'intensité ou de la durée de vie de luminescence du composé fluorescent donneur ou du composé accepteur lorsque ce dernier est fluorescent.

Le terme « contenant » désigne un puits d'une plaque, un tube à essai ou de tout autre contenant approprié au mélange d'une préparation membranaire avec les réactifs nécessaires à la mise en oeuvre de la méthode selon l'invention.

Méthodes

Selon un premier aspect, l'invention concerne une méthode pour déterminer la capacité d'une molécule à moduler l'activation d'un récepteur couplé à une protéine G (RCPG), ladite méthode comprenant les étapes suivantes :

a) introduction, dans un premier contenant :

- d'une préparation membranaire portant un ou plusieurs RCPG et une ou plusieurs protéine(s) G,
- d'une source de GDP ou d'une source de GTP,
- d'un premier ligand de la sous-unité alpha d'une protéine G (protéine Galpha) marqué par un premier membre d'un couple de partenaires de RET, et d'un second ligand de la protéine Galpha marqué par un second membre du couple de partenaires de RET, lesdits ligands étant capables de se lier spécifiquement, soit individuellement soit en association, à la protéine Galpha qui n'est pas liée au GTP ou au GDP ou au GTP non hydrolysable ou lentement hydrolysable (protéine Galpha vide) ;

b) mesure du signal de RET émis dans le premier contenant ;
c) introduction (i) dans un second contenant, des mêmes réactifs qu'à l'étape a) et de la molécule à tester ou (ii) dans le premier contenant, de la molécule à tester ;
d) mesure du signal de RET émis dans le second contenant ou dans le premier contenant obtenu à l'étape c) ;
e) comparaison des signaux obtenus aux étapes b) et d) ;

- une augmentation du signal quand on utilise une source de GDP et des ligands capables de se lier spécifiquement à la protéine Galpha vide indiquant que la molécule est un agoniste du RCPG ;
- une diminution du signal quand on utilise une source de GDP et des ligands capables de se lier spécifiquement à la protéine Galpha vide indiquant que la molécule est un agoniste inverse du RCPG ;
- une augmentation du signal quand on utilise une source de GTP et des ligands capables de se lier spécifiquement à la protéine Galpha vide indiquant que la molécule est un agoniste du RCPG ;
- une diminution du signal quand on utilise une source de GTP et des ligands capables de se lier spécifiquement à la protéine Galpha vide indiquant que la molécule est un agoniste inverse du RCPG ;

caractérisée en ce que le premier ligand est choisi parmi un anticorps, un fragment d'anticorps, un peptide ou un aptamère et le second ligand est choisi parmi un anticorps, un fragment d'anticorps ou un aptamère.

Selon un deuxième aspect, l'invention concerne une méthode pour déterminer la capacité d'une molécule à moduler l'activation d'un récepteur couplé à une protéine G (RCPG), ladite méthode comprenant les étapes suivantes :

a) introduction, dans un premier contenant :

- d'une préparation membranaire portant un ou plusieurs RCPG et une ou plusieurs protéine(s) G,
- d'une source de GDP ou d'une source de GTP,
- d'un premier ligand de la sous-unité alpha d'une protéine G (protéine Galpha) marqué par un premier membre d'un couple de partenaires de RET, et d'un second ligand de la protéine Galpha marqué par un second membre du couple de partenaires de RET, lesdits ligands étant capables de se lier spécifiquement, soit individuellement soit en association, à la protéine Galpha qui n'est pas liée au GTP ou au GDP ou au GTP non hydrolysable ou lentement hydrolysable (protéine Galpha vide) ;
- d'un agoniste du RCPG ;

b) mesure du signal de RET émis dans le premier contenant ;
c) introduction dans un second contenant, des mêmes réactifs qu'à l'étape a) et de la molécule à tester;
d) mesure du signal de RET émis dans le second contenant obtenu à l'étape c) ;
e) comparaison des signaux obtenus aux étapes b) et d);

- une augmentation du signal quand on utilise une source de GDP et des ligands capables de se lier spécifiquement à la protéine Galpha vide indiquant que la molécule est un agoniste ou un modulateur allostérique positif du RCPG ;
- une augmentation du signal quand on utilise une source de GTP et des ligands capables de se lier spécifiquement à la protéine Galpha vide indiquant que la molécule est un agoniste ou un modulateur allostérique positif du RCPG ;
- une diminution du signal quand on utilise une source de GDP et des ligands capables de se lier spécifiquement à la protéine Galpha vide indiquant que la molécule est un antagoniste ou un modulateur allostérique négatif du RCPG ;
- une diminution du signal quand on utilise une source de GTP et des ligands capables de se lier spécifiquement à la protéine Galpha vide indiquant que la molécule est un antagoniste ou un modulateur allostérique négatif du RCPG ;

caractérisée en ce que le premier ligand est choisi parmi un anticorps, un fragment d'anticorps, un peptide ou un aptamère et le second ligand est choisi parmi un anticorps, un fragment d'anticorps ou un aptamère.

La description concerne également, mais qui ne concerne pas la présente invention, une méthode pour mesurer l'activation d'un récepteur couplé à une protéine G (RCPG), ladite méthode comprenant les étapes suivantes :

a) introduction, dans un premier contenant :

- d'une préparation membranaire portant un ou plusieurs RCPG et une ou plusieurs protéine(s) G,
- d'une source de GDP ou d'une source de GTP,
- d'un premier ligand de la sous-unité alpha d'une protéine G (protéine Galpha) marqué par un premier membre d'un couple de partenaires de RET, et d'un second ligand de la protéine Galpha marqué par un second membre du couple de partenaires de RET, lesdits ligands étant capables de se lier spécifiquement, soit individuellement soit en association, à la protéine Galpha vide ou à la protéine Galpha pleine ;

b) mesure du signal de RET émis dans le premier contenant ;
c) introduction (i) dans un second contenant, des mêmes réactifs qu'à l'étape a) et d'une molécule capable de moduler l'activation du RCPG ou (ii) dans le premier contenant, d'une molécule capable de moduler l'activation du RCPG ;
d) mesure du signal de RET émis dans le second contenant ou dans le premier contenant obtenu à l'étape c) ;
e) comparaison des signaux obtenus aux étapes b) et d), une augmentation ou une diminution du signal obtenu à l'étape d) par rapport à celui obtenu à l'étape b) indiquant une modulation de l'activation du RCPG.

La description concerne également, mais qui ne correspond pas à la présente invention, une méthode pour détecter l'apparition ou la disparition d'une protéine G vide ou d'une protéine G pleine dans une préparation de récepteur couplé à une protéine G (RCPG), ladite méthode comprenant les étapes suivantes :

a) introduction, dans un premier contenant :

- d'une préparation membranaire portant un ou plusieurs RCPG et une ou plusieurs protéine(s) Galpha,
- d'une source de GDP ou d'une source de GTP,
- d'un premier ligand de la sous-unité alpha d'une protéine G (protéine Galpha) marqué par un premier membre d'un couple de partenaires de RET, et d'un second ligand de la protéine Galpha marqué par un second membre du couple de partenaires de RET, lesdits ligands étant capables de se lier spécifiquement, soit individuellement soit en association, à la protéine Galpha vide ou à la protéine Galpha pleine ;

b) mesure du signal de RET émis dans le premier contenant ;
c) introduction (i) dans un second contenant, des mêmes réactifs qu'à l'étape a) et d'une molécule capable de moduler l'activation du RCPG ou (ii) dans le premier contenant, d'une molécule capable de moduler l'activation du RCPG ;

d) mesure du signal de RET émis dans le second contenant ou dans le premier contenant obtenu à l'étape c) ;
e) comparaison des signaux obtenus aux étapes b) et d), une augmentation ou une diminution du signal obtenu à l'étape d) par rapport à celui obtenu à l'étape b) indiquant une apparition ou une disparition de la protéine G vide ou de la protéine G pleine.

La description concerne également, mais qui ne correspond pas à la présente invention, une méthode pour déterminer la capacité d'une molécule à moduler l'activation d'un récepteur couplé à une protéine G (RCPG), ladite méthode comprenant les étapes suivantes :

a) introduction, dans un premier contenant :

- d'une préparation membranaire portant un ou plusieurs RCPG et une ou plusieurs protéine(s) G,
- d'une source de GDP et d'une source de GTP,
- d'un premier ligand de la sous-unité alpha d'une protéine G (protéine Galpha) marqué par un premier membre d'un couple de partenaires de RET, et d'un second ligand de la protéine Galpha marqué par un second membre du couple de partenaires de RET, lesdits ligands étant capables de se lier spécifiquement, soit individuellement soit en association, à la protéine Galpha vide ou à la protéine Galpha pleine ;

b) mesure du signal de RET émis dans le premier contenant ;
c) introduction (i) dans un second contenant, des mêmes réactifs qu'à l'étape a) et de la molécule à tester ou (ii) dans le premier contenant, de la molécule à tester ;
d) mesure du signal de RET émis dans le second contenant ou dans le premier contenant obtenu à l'étape c) ;
e) comparaison des signaux obtenus aux étapes b) et d) ;

- une augmentation du signal quand on utilise une source de GDP et des ligands capables de se lier spécifiquement à la protéine Galpha vide indiquant que la molécule est un agoniste du RCPG ;
- une diminution du signal quand on utilise une source de GDP et des ligands capables de se lier spécifiquement à la protéine Galpha vide indiquant que la molécule est un agoniste inverse du RCPG ;
- une diminution du signal quand on utilise une source de GDP et des ligands capables de se lier spécifiquement à la protéine Galpha pleine indiquant que la molécule est un agoniste du RCPG ;
- une augmentation du signal quand on utilise une source de GDP et des ligands capables de se lier spécifiquement à la protéine Galpha pleine indiquant que la molécule est un agoniste inverse du RCPG ;
- une augmentation du signal quand on utilise une source de GTP et des ligands capables de se lier spécifiquement à la protéine Galpha vide indiquant que la molécule est un agoniste du RCPG ;
- une diminution du signal quand on utilise une source de GTP et des ligands capables de se lier spécifiquement à la protéine Galpha vide indiquant que la molécule est un agoniste inverse du RCPG ;
- une diminution du signal quand on utilise une source de GTP et des ligands capables de se lier spécifiquement à la protéine Galpha pleine indiquant que la molécule est un agoniste du RCPG ;
- une augmentation du signal quand on utilise une source de GTP et des ligands capables de se lier spécifiquement à la protéine Galpha pleine indiquant que la molécule est un agoniste inverse du RCPG.

La description concerne également, mais qui ne correspond pas à la présente invention, une méthode pour déterminer la capacité d'une molécule à moduler l'activation d'un récepteur couplé à une protéine G (RCPG), ladite méthode comprenant les étapes suivantes :

a) introduction, dans un premier contenant :

- d'une préparation membranaire portant un ou plusieurs RCPG et une ou plusieurs protéine(s) G,
- d'une source de GDP et d'une source de GTP,
- d'un premier ligand de la sous-unité alpha d'une protéine G (protéine Galpha) marqué par un premier membre d'un couple de partenaires de RET, et d'un second ligand de la protéine Galpha marqué par un second membre du couple de partenaires de RET, lesdits ligands étant capables de se lier spécifiquement, soit individuellement soit en association, à la protéine Galpha vide ou à la protéine Galpha pleine ;
- d'un agoniste du RCPG ;

b) mesure du signal de RET émis dans le premier contenant ;
c) introduction dans un second contenant, des mêmes réactifs qu'à l'étape a) et de la molécule à tester;
d) mesure du signal de RET émis dans le second contenant obtenu à l'étape c) ;

e) comparaison des signaux obtenus aux étapes b) et d);

- une augmentation du signal quand on utilise une source de GDP et des ligands capables de se lier spécifiquement à la protéine Galpha vide indiquant que la molécule est un agoniste ou un modulateur allostérique positif du RCPG ;
- une diminution du signal quand on utilise une source de GDP et des ligands capables de se lier spécifiquement à la protéine Galpha pleine indiquant que la molécule est un agoniste ou un modulateur allostérique positif du RCPG ;
- une augmentation du signal quand on utilise une source de GTP et des ligands capables de se lier spécifiquement à la protéine Galpha vide indiquant que la molécule est un agoniste ou un modulateur allostérique positif du RCPG ;
- une diminution du signal quand on utilise une source de GTP et des ligands capables de se lier spécifiquement à la protéine Galpha pleine indiquant que la molécule est un agoniste ou un modulateur allostérique positif du RCPG.
- une diminution du signal quand on utilise une source de GDP et des ligands capables de se lier spécifiquement à la protéine Galpha vide indiquant que la molécule est un antagoniste ou un modulateur allostérique négatif du RCPG ;
- une augmentation du signal quand on utilise une source de GDP et des ligands capables de se lier spécifiquement à la protéine Galpha pleine indiquant que la molécule est un antagoniste ou un modulateur allostérique négatif du RCPG ;
- une diminution du signal quand on utilise une source de GTP et des ligands capables de se lier spécifiquement à la protéine Galpha vide indiquant que la molécule est un antagoniste ou un modulateur allostérique négatif du RCPG ;
- une augmentation du signal quand on utilise une source de GTP et des ligands capables de se lier spécifiquement à la protéine Galpha pleine indiquant que la molécule est un antagoniste ou un modulateur allostérique négatif du RCPG.

Etape a)

Selon le premier aspect de la présente invention, l'étape a) consiste à introduire, dans un premier contenant les trois éléments suivants :

- une préparation membranaire portant un ou plusieurs RCPG et une ou plusieurs protéine(s) G,
- une source de GDP ou d'une source de GTP,
- un premier ligand de la sous-unité alpha d'une protéine G (protéine Galpha) marqué par un premier membre d'un couple de partenaires de RET, et un second ligand de la protéine Galpha marqué par un second membre du couple de partenaires de RET, lesdits ligands étant capables de se lier spécifiquement, soit individuellement soit en association, à la protéine Galpha vide.

Dans un mode de réalisation du premier aspect, à l'étape a), le premier contenant ne contient pas la molécule à tester. Il permet de mesurer le niveau basal du signal de RET et/ou le bruit de fond du signal de RET.

Selon le deuxième aspect de la présente invention, l'étape a) consiste à introduire, dans un premier contenant les quatre éléments suivants :

- une préparation membranaire portant un ou plusieurs RCPG et une ou plusieurs protéine(s) G,
- une source de GDP ou d'une source de GTP,
- un premier ligand de la sous-unité alpha d'une protéine G (protéine Galpha) marqué par un premier membre d'un couple de partenaires de RET, et un second ligand de la protéine Galpha marqué par un second membre du couple de partenaires de RET, lesdits ligands étant capables de se lier spécifiquement, soit individuellement soit en association, à la protéine Galpha vide,
- un agoniste du RCPG.

Dans un mode de réalisation préféré, la source de GTP est un GTP non-hydrolysable ou lentement hydrolysable, de préférence choisi parmi le GTPgammaS (GTP$\gamma$S), GppNHp et GppCp, de préférence le GTPgammaS (GTP$\gamma$S).

Dans un mode de réalisation préféré, la source de GDP est le GDP.

On doit comprendre que la méthode selon l'invention ne prévoit pas d'introduire à la fois une source de GTP et une source de GDP.

Les différents éléments peuvent être introduits dans le contenant de manière séquentielle dans n'importe quel

ordre, ou de manière simultanée ou quasi-simultanée. Le mélange des éléments permet d'obtenir une solution réactionnelle adaptée à la mise en oeuvre d'un RET. D'autres éléments peuvent être ajoutés dans le contenant afin d'adapter la solution à la mise en oeuvre du RET. Par exemple, on peut ajouter la coelentérazine h (benzyl-coelentérazine) ou la bisdésoxycoelentérazine (DeepBlueC™) ou didéshydrocoelenterazine (coelentérazine-400a) ou la D-luciférine.

Les ligands sont capables de se lier spécifiquement, soit individuellement soit en association, à la protéine Galpha vide. Dans un premier mode de réalisation, chacun des deux ligands peut se lier spécifiquement et individuellement à la protéine Galpha vide, c'est à dire qu'un ligand peut se lier spécifiquement à la protéine Galpha vide en présence et en l'absence de l'autre ligand. Dans un second mode de réalisation particulier, les deux ligands en association peuvent se lier spécifiquement à la protéine Galpha vide, c'est à dire qu'au moins un des deux ligands ne peut se lier spécifiquement à la protéine Galpha vide qu'en présence de l'autre ligand.

Dans un mode de réalisation particulier, on utilise une source de GDP et des ligands capables de se lier spécifiquement, soit individuellement soit en association, à la protéine Galpha vide. Ce mode de réalisation permet de discriminer la protéine Galpha vide de la protéine Galpha pleine liée au GDP.

Dans un autre mode de réalisation particulier, on utilise une source de GTP et des ligands capables de se lier spécifiquement, soit individuellement soit en association, à la protéine Galpha vide. Ce mode de réalisation permet de discriminer la protéine Galpha vide de la protéine Galpha pleine liée au GTP et/ou au GTP non-hydrolysable/lentement hydrolysable.

Avantageusement, la protéine Galpha est choisie parmi la protéine Galphai1, Galphai2, Galphai3, Galphao1, Galphao2, Galphaq, Galpha12, Galpha13, Galphas, Galphaz, Galphat1, Galphat2, Galpha11, Galpha14, Galpha15, Galpha16 et Galphagus, de préférence choisie parmi la protéine Galphai1, Galphai2 et Galphai3.

Dans un mode de réalisation particulier, le premier ligand est un peptide et le second ligand est un anticorps ou un fragment d'anticorps. Par exemple, l'anticorps anti-Galphai est l'anticorps du fournisseur Santa Cruz Biotechnologies de référence #SC13533 (clone R4, qui reconnait les formes vide et pleine de la protéine G).

Dans un autre mode de réalisation particulier, le premier ligand et le second ligand sont des anticorps ou des fragments d'anticorps.

Dans un mode de réalisation particulier, le ou les anticorps utilisés pour la mise en œuvre du procédé selon l'invention est/sont choisi(s) parmi DSV3S, DSV39S et DSV26S (anticorps DSV disponibles auprès de Cisbio Bioassays sur demande).

Bien entendu, le premier ligand et le deuxième ligand ne doivent pas entrer en compétition quand ils se lient à la protéine Galpha, par exemple le premier ligand et le deuxième ligand ne lient pas le même épitope sur la protéine Galpha.

L'homme du métier n'aura aucune difficulté à adapter la concentration de l'agoniste du RCPG et la concentration de la molécule à tester, par exemple en fonction de l'agoniste choisi, de la molécule à tester et/ou en fonction des caractéristiques recherchées.

<u>Etape b)</u>

Selon les trois aspects de la présente invention, l'étape b) consiste à mesurer le signal de RET émis dans le premier contenant, c'est-à-dire le contenant obtenu à l'étape a). Le signal mesuré correspond au signal obtenu dans le contenant en absence de la molécule à tester. La mesure peut se faire par des méthodes conventionnelles largement connues de l'homme du métier et ne pose pas de problème particulier. On utilise généralement un appareil qui permet de détecter et de mesurer le signal de RET, comme par exemple le lecteur de microplaques PHERAstar FS (BMG Labtech) avec le mode de lecture TR-FRET ou bioluminescence.

<u>Etape c)</u>

Selon le premier aspect de l'invention :

- Dans un mode de réalisation, l'étape c) consiste à introduire dans un second contenant les mêmes réactifs qu'à l'étape a) et la molécule à tester. Avantageusement, le second contenant est préparé de la même manière que le premier contenant, la seule différence étant la présence dans le deuxième contenant de la molécule à tester. Ce mode de réalisation est avantageux car il permet d'effectuer la mesure du signal de RET émis dans le premier contenant et dans le deuxième contenant de manière simultanée. Ce mode de réalisation permet également d'effectuer la mesure simultanée du signal de RET émis dans un ou plusieurs seconds contenants. Ainsi, ce mode de réalisation est particulièrement avantageux puisqu'il permet de tester plusieurs molécules différentes en parallèle.
- Dans un autre mode de réalisation, l'étape c) consiste à introduire dans le premier contenant la molécule à tester. Ce mode de réalisation présente l'avantage de n'utiliser qu'un seul contenant pour la mise en oeuvre de la méthode selon l'invention.

Dans un mode de réalisation particulier, on introduit la molécule à tester en concentration croissante pour faire varier l'amplitude de la diminution ou de l'augmentation du signal.

Selon le deuxième aspect de l'invention, l'étape c) consiste à introduire dans un second contenant les mêmes réactifs qu'à l'étape a) et la molécule à tester. Avantageusement, le second contenant est préparé de la même manière que le premier contenant, la seule différence étant la présence dans le deuxième contenant de la molécule à tester. Ainsi, la mesure du signal de RET émis dans le premier contenant et dans le deuxième contenant est effectuée de manière simultanée. Cela permet également d'effectuer la mesure simultanée du signal de RET émis dans un ou plusieurs seconds contenants. Cela permet de tester plusieurs molécules différentes en parallèle.

Dans un mode de réalisation particulier, on introduit la molécule à tester ou l'agoniste du RCPG en concentration croissante pour faire varier l'amplitude de la diminution ou de l'augmentation du signal.

Etape d)

Selon les trois aspects de la présente invention, l'étape d) consiste à mesurer le signal de RET émis dans le second contenant ou dans le premier contenant obtenu à l'étape c). Le signal mesuré correspond au signal obtenu dans le contenant en présence de la molécule à tester (+/- un agoniste pour le deuxième aspect). Comme à l'étape b), la mesure peut se faire par des méthodes conventionnelles largement connues de l'homme du métier et ne pose pas de problème particulier. On utilise généralement un appareil qui permet de détecter et de mesurer le signal de RET, comme par exemple le lecteur de microplaques PHERAstar FS (BMG Labtech) avec le mode de lecture TR-FRET ou bioluminescence.

Etape e)

Selon les trois aspects de la présente invention, l'étape e) consiste à comparer les signaux obtenus aux étapes b) et d).

L'homme du métier peut aisément comparer les signaux des étapes b) et d) et définir un seuil lui permettant de qualifier l'augmentation ou la diminution, par exemple un écart entre les signaux supérieur à 5%, supérieur à 10%, supérieur à 15%, supérieur à 20% ou supérieur à 25%. On peut par exemple calculer le ratio entre les signaux des étapes b) et d). De manière générale, pour un couple de ligands donné, plus l'écart entre les signaux est important, plus le ratio entre les signaux sera important et plus la modulation de l'activation du RCPG (e.g. activation ou inhibition) est importante. L'écart entre les signaux est toutefois susceptible de varier en fonction du couple de ligands utilisés pour la mise en oeuvre du procédé selon l'invention. Le niveau de modulation de l'activation du RCPG permet d'identifier des molécules plus ou moins agonistes, antagonistes, agonistes inverse, modulateur allostérique positif ou modulateur allostérique négatif.

Selon le premier aspect :

- une augmentation du signal quand on utilise une source de GDP et des ligands capables de se lier spécifiquement à la protéine Galpha vide indiquant que la molécule est un agoniste du RCPG ;
- une diminution du signal quand on utilise une source de GDP et des ligands capables de se lier spécifiquement à la protéine Galpha vide indiquant que la molécule est un agoniste inverse du RCPG ;
- une augmentation du signal quand on utilise une source de GTP et des ligands capables de se lier spécifiquement à la protéine Galpha vide indiquant que la molécule est un agoniste du RCPG ;
- une diminution du signal quand on utilise une source de GTP et des ligands capables de se lier spécifiquement à la protéine Galpha vide indiquant que la molécule est un agoniste inverse du RCPG.

Selon le deuxième aspect :

- une augmentation du signal quand on utilise une source de GDP et des ligands capables de se lier spécifiquement à la protéine Galpha vide indiquant que la molécule est un agoniste ou un modulateur allostérique positif du RCPG ;
- une augmentation du signal quand on utilise une source de GTP et des ligands capables de se lier spécifiquement à la protéine Galpha vide indiquant que la molécule est un agoniste ou un modulateur allostérique positif du RCPG ;
- une diminution du signal quand on utilise une source de GDP et des ligands capables de se lier spécifiquement à la protéine Galpha vide indiquant que la molécule est un antagoniste ou un modulateur allostérique négatif du RCPG ;
- une diminution du signal quand on utilise une source de GTP et des ligands capables de se lier spécifiquement à la protéine Galpha vide indiquant que la molécule est un antagoniste ou un modulateur allostérique négatif du RCPG.

Marguage du ligand par un membre d'un couple de partenaires de RET

Un ligand peut être marqué de manière directe ou indirecte.

Le marquage direct du ligand par un membre d'un couple de partenaires de RET, par exemple un composé fluorescent quand on met en oeuvre un FRET, peut être effectué par les méthodes classiques connues de l'homme du métier, reposant sur la présence de groupes réactifs sur le ligand. Par exemple, lorsque le ligand est un anticorps ou un fragment d'anticorps, les groupes réactifs suivants peuvent être utilisés : le groupe amino terminal, les groupes carboxylates des acides aspartique et glutamique, les groupes amines des lysines, les groupes guanidines des arginines, les groupes thiols des cystéines, les groupes phénols des tyrosines, les cycles indoles des tryptophanes, les groupes thioéthers des méthionines, les groupes imidazoles des histidines.

Les groupes réactifs peuvent former une liaison covalente avec un groupe réactif porté par un membre d'un couple de partenaires de RET. Les groupes réactifs appropriés, portés par le membre d'un couple de partenaires de RET, sont bien connus de l'homme du métier, par exemple un composé donneur ou un composé accepteur fonctionnalisé par un groupe maléimide sera par exemple capable de se lier de manière covalente avec les groupes thiols portés par les cystéines portées par une protéine ou un peptide, par exemple un anticorps ou un fragment d'anticorps. De même, un composé donneur/accepteur portant un ester de N-hydroxysuccinimide sera capable de se fixer de manière covalente à une amine présente une protéine ou un peptide.

Le ligand peut également être marqué par un composé fluorescent ou bioluminescent de manière indirecte, par exemple en introduisant dans le milieu de mesure un anticorps ou un fragment d'anticorps, lui-même lié de manière covalente à un composé accepteur/donneur, ce deuxième anticorps ou fragment d'anticorps reconnaissant de manière spécifique le ligand.

Un autre moyen de marquage indirect très classique consiste à fixer de la biotine sur le ligand à marquer, puis d'incuber ce ligand biotinylé en présence de streptavidine marquée par un composé accepteur/donneur. Des ligands biotinylés appropriés peuvent être préparés par des techniques bien connues de l'homme du métier ; la société Cisbio Bioassays commercialise par exemple de la streptavidine marquée avec un fluorophore dont la dénomination commerciale est « d2 » (réf. 610SADLA).

Dans le cadre de l'invention, le premier ligand et le second ligand sont chacun marqués par un membre d'un couple de partenaires de RET, l'un des membres du couple étant un composé fluorescent donneur ou luminescent donneur et l'autre membre du couple étant un composé fluorescent accepteur ou un composé accepteur non fluorescent (quencher).

Marquage pour la mise en oeuvre d'un FRET

Dans un mode de réalisation particulier, le premier ligand et le deuxième ligand sont chacun marqués par un membre d'un couple de partenaires de FRET, c'est à dire un composé fluorescent donneur ou un composé fluorescent accepteur d'énergie.

La sélection du couple de partenaires de FRET pour obtenir un signal de FRET est à la portée de l'homme du métier. Par exemple, des couples donneur-accepteur utilisables pour étudier les phénomènes de FRET sont notamment décrits dans l'ouvrage de Joseph R. Lakowicz (Principles of fluorescence spectroscopy, $2^{nd}$ édition 338), auquel l'homme du métier pourra se référer.

*Composés fluorescents donneurs*

Les composés fluorescents donneurs d'énergie à durée de vie longue (> 0,1 ms, de préférence comprise dans la gamme allant de 0,5 à 6 ms), en particulier les chélates ou cryptates de terres rares sont avantageux puisqu'ils permettent d'effectuer des mesures en temps résolu, c'est-à-dire de mesurer des signaux de TR-FRET (en langue anglaise, « Time Resolved FRET ») en s'affranchissant d'une grande partie du bruit de fond émis par le milieu de mesure. Ils sont pour cette raison et de manière générale préférés pour la mise en oeuvre du procédé selon l'invention. Avantageusement, ces composés sont des complexes de lanthanides. Ces complexes (tels que chélates ou cryptates) sont particulièrement appropriés en tant que membre du couple de FRET donneur d'énergie. Les complexes d'europium (Eu3+), de terbium (Tb3+), de dysprosium (Dy3+), de samarium (Sm3+), de neodymium (Nd3+), d'ytterbium (Yb3+) ou encore d'erbium (Er3+) sont des complexes de terres rares également appropriés aux fins de l'invention, les complexes d'europium (Eu3+) et de terbium (Tb3+) étant particulièrement préférés. De très nombreux complexes de terres rares ont été décrits et plusieurs sont actuellement commercialisés par les sociétés Perkin Elmer, Invitrogen et Cisbio Bioassays.

Des exemples de chélates ou cryptates de terres rares appropriés aux fins de l'invention sont :

- Les cryptates de lanthanides, comportant un ou plusieurs motifs pyridine. De tels cryptates de terre rare sont décrits

par exemple dans les brevets EP 0 180 492, EP 0 321 353, EP 0 601 113 et dans la demande internationale WO 01/96 877. Les cryptates de terbium (Tb3+) et d'europium (Eu3+) sont particulièrement appropriés aux fins de la présente invention. Des cryptates de lanthanides sont commercialisés par la société Cisbio Bioassays. On peut citer à titre d'exemple non limitatif les cryptates d'europium de formules ci-dessous (qui peuvent être couplés au composé à marquer via un groupe réactif, ici par exemple un groupe $NH_2$) :

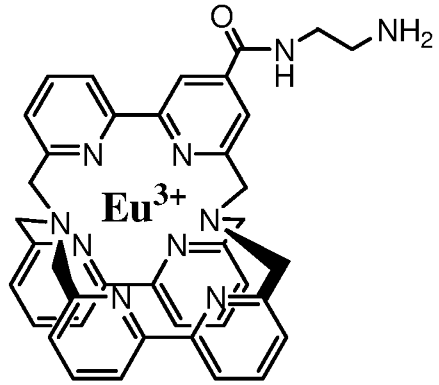

**TrisBiPy-Eu**

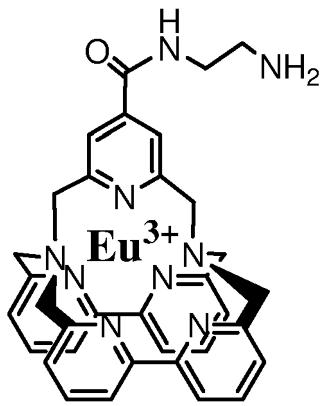

**Py-BiPy-Eu**

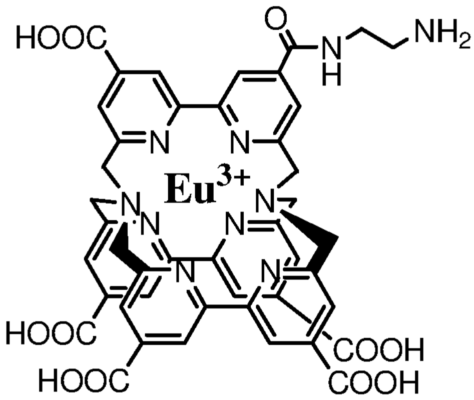

**TrisBiPy-tetraacide-Eu**

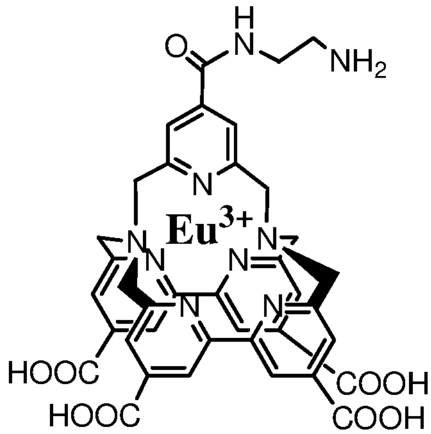

**Py-BiPy-tetraacide-Eu**

- Les chélates de lanthanides décrits notamment dans les brevets US 4 761 481, US 5 032 677, US 5 055 578, US 5 106 957, US 5 116 989, US 4 761 481, US 4 801 722, US 4 794 191, US 4 637 988, US 4 670 572, US 4 837 169, US 4 859 777. Les brevets EP 0 403 593, US 5 324 825, US 5 202 423, US 5 316 909 décrivent des chélates composés d'un ligand nonadenté tel que la terpyridine. Des chélates de lanthanides sont commercialisés par la société Perkin Elmer.
- Des complexes de lanthanides constitués d'un agent chélatant, tel que le tétraazacyclododécane, substitué par un chromophore comportant des cycles aromatiques, tels que ceux décrits par Poole R. et al. dans Biomol. Chem, 2005, 3, 1013-1024 "Synthesis and characterisation of highly emissive and kinetically stable lanthanide complexes suitable for usage in cellulo", peuvent également être utilisés. On peut aussi utiliser les complexes décrits dans la demande WO 2009/10580.
- Les cryptates de lanthanides décrits dans les brevets EP 1 154 991 et EP 1 154 990 sont également utilisables.
  - le cryptate de terbium de formule ci-dessous (qui peut être couplé à un composé à marquer via un groupe réactif, ici par exemple un groupe $NH_2$) :

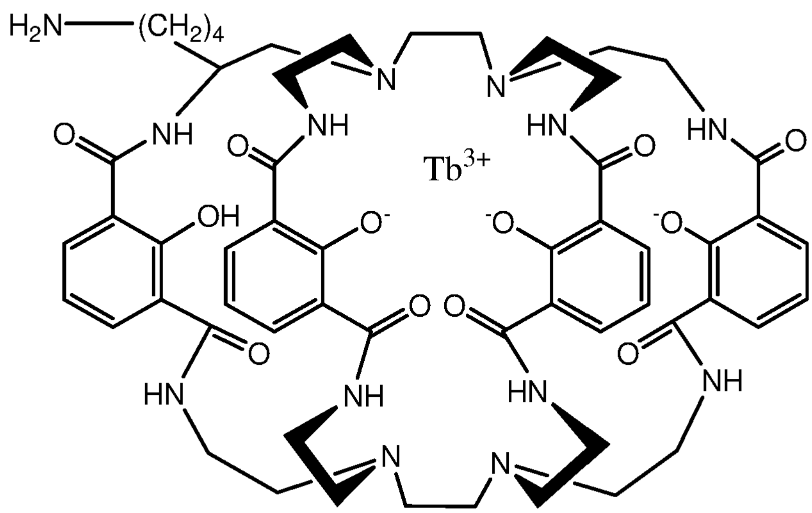

et dont la synthèse est décrite dans la demande internationale WO 2008/063721 (composé 6a page 89).

- Le cryptate de terbium Lumi4-Tb de la société Lumiphore, commercialisé par Cisbio Bioassays.
- Le quantum dye de la société Research Organics, de formule ci-dessous (qui peut être couplé au composé à marquer via un groupe réactif, ici NCS) :

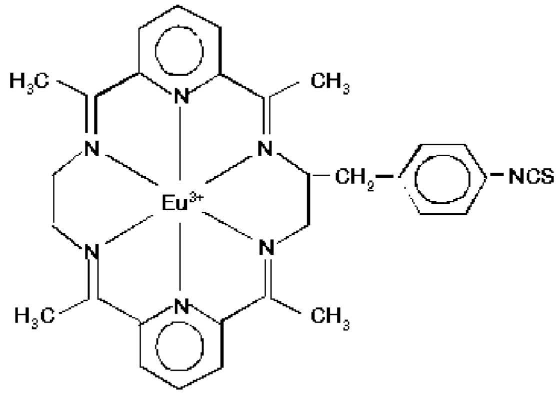

-

- Les chélates de ruthénium, en particulier les complexes constitués d'un ion ruthénium et de plusieurs bipyridines comme le ruthénium(II) tris(2,2'-bipyridine).
- Le chélate de terbium DTPA-cs124 Tb, commercialisé par la société Life technologies de formule ci-dessous (qui peut être couplé au composé à marquer via un groupe réactif R) et dont la synthèse est décrite dans le brevet américain US 5 622 821.

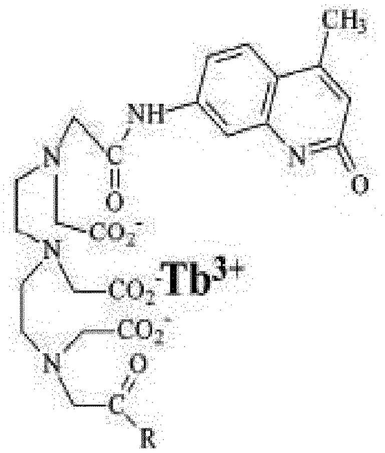

- Le chélate de terbium de formule ci-dessous et décrit par Latva et al (Journal of Luminescence 1997, 75(2): 149-169) :

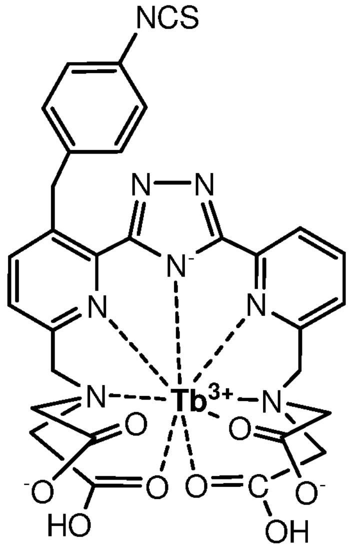

Tb-W14016

Avantageusement, le composé fluorescent donneur est un partenaire de FRET choisi parmi : un cryptate d'europium, un chélate d'europium, un chélate de terbium, un cryptate de terbium, un chélate de ruthénium, un quantum dot, les allophycocyanines, les rhodamines, les cyanines, les squaraines, les coumarines, les proflavines, les acridines, les fluorescéines, les dérivés du boron-dipyrrométhène et le nitrobenzoxadiazole.

De manière particulièrement avantageuse, le composé fluorescent donneur est un partenaire de FRET choisi parmi : un cryptate d'europium; un chélate d'europium ; un chélate de terbium ; un cryptate de terbium ; un chélate de ruthénium ; et un quantum dot ; les chélates et les cryptates d'europium et de terbium étant particulièrement préférés.

*Composés fluorescents accepteurs*

Les composés fluorescents accepteurs peuvent être choisis dans le groupe suivant : les allophycocyanines, en particulier celles connues sous la dénomination commerciale XL665 ; les molécules organiques luminescentes, telles que les rhodamines, les cyanines (comme par exemple Cy5), les squaraines, les coumarines, les proflavines, les acridines, les fluorescéines, les dérivés du boron-dipyrrométhène (commercialisés sous la dénomination « Bodipy »), les fluorophores connus sous la dénomination « Atto », les fluorophores connus sous la dénomination «DY», les composés connus sous la dénomination « Alexa », le nitrobenzoxadiazole. Avantageusement, les composés fluorescents accepteurs sont choisis parmi les allophycocyanines, les rhodamines, les cyanines, les squaraines, les coumarines, les proflavines, les acridines, les fluorescéines, les dérivés du boron-dipyrrométhène, le nitrobenzoxadiazole.

Les expressions « les cyanines » et « les rhodamines » doivent être respectivement comprises comme « les dérivés de cyanine » et « les dérivés de rhodamine ». L'homme du métier connaît ces différents fluorophores, disponibles dans le commerce.

Les composés « Alexa » sont commercialisés par la société Invitrogen; les composés « Atto » sont commercialisés par la société Attotec ; les composés « DY » sont commercialisés par la société Dyomics ; les composés « Cy » sont commercialisés par la société Amersham Biosciences ; les autres composés sont commercialisés par divers fournisseurs de réactifs chimiques, tels que les sociétés Sigma, Aldrich ou Acros.

Les protéines fluorescentes suivantes peuvent également être utilisées en tant que composé fluorescent accepteur : les protéines fluorescentes cyans (AmCyan1, Midori-Ishi Cyan, mTFP1), les protéines fluorescentes vertes (EGFP, AcGFP, TurboGFP, Emerald, Azami Green, ZsGreen), les protéines fluorescentes jaunes (EYFP, Topaz, Venus, mCitrine, YPet, PhiYFP, ZsYellow1, mBanana), les protéines fluorescentes oranges et rouges (Orange kusibari, mOrange, tdtomato, DsRed, DsRed2, DsRed-Express, DsRed-Monomer, mTangerine, AsRed2, mRFP1, JRed, mCherry, mStrawberry, HcRed1, mRaspberry, HcRed-Tandem, mPlim, AQ143), les protéines fluorescentes dans le rouge lointain (mKate, mKate2, tdKatushka2).

Avantageusement, le composé fluorescent accepteur est un partenaire de FRET choisi parmi : les allophycocyanines, les rhodamines, les cyanines, les squaraines, les coumarines, les proflavines, les acridines, les fluorescéines, les dérivés du boron-dipyrrométhène, le nitrobenzoxadiazole et un quantum dot, la GFP, les variants GFP choisis parmi GFP10, GFP2 et eGFP, la YFP, les variants YFP choisis parmi eYFP, YFP topaz, YFP citrine, YFP venus et YPet, le mOrange, le DsRed.

Marquage pour la mise en oeuvre d'un BRET

Dans un mode de réalisation particulier, le premier ligand et le deuxième ligand sont chacun marqués par un membre d'un couple de partenaires de BRET, c'est à dire un composé luminescent donneur ou un composé fluorescent accepteur d'énergie.

Un ligand peut être marqué de manière directe ou indirecte.

Le marquage direct du ligand par un composé luminescent donneur ou un composé fluorescent accepteur de type protéine, membre d'un couple de partenaires de BRET, peut être effectué par les méthodes classiques connues de l'homme du métier et notamment décrites dans l'article de Tarik Issad et Ralf Jockers (Bioluminescence Resonance Energy Transfer to Monitor Protein-Protein Interactions, Transmembrane Signaling Protocols pp 195-209, Part of the Methods in Molecular Biology™ book series MIMB, volume 332) auquel l'homme du métier pourra se référer.

Le marquage direct du ligand par un composé fluorescent accepteur de type molécule organique, membre d'un couple de partenaires de BRET, peut être effectué par les méthodes classiques connues de l'homme du métier, reposant sur la présence de groupes réactifs sur le ligand comme cité ci-dessus. Par exemple, lorsque le ligand est un anticorps ou un fragment d'anticorps, les groupes réactifs suivants peuvent être utilisés : le groupe amino terminal, les groupes carboxylates des acides aspartiques et glutamiques, les groupes amines des lysines, les groupes guanidines des arginines, les groupes thiols des cystéines, les groupes phénols des tyrosines, les cycles indoles des tryptophanes, les groupes thioéthers des méthionines, les groupes imidazoles des histidines.

Les groupes réactifs peuvent former une liaison covalente avec un groupe réactif porté par un membre d'un couple de partenaires de BRET. Les groupes réactifs appropriés, portés par le membre d'un couple de partenaires de BRET, sont bien connus de l'homme du métier, par exemple un composé accepteur fonctionnalisé par un groupe maléimide sera par exemple capable de se lier de manière covalente avec les groupes thiols portés par les cystéines portées par une protéine ou un peptide, par exemple un anticorps ou un fragment d'anticorps. De même, un composé accepteur portant un ester de N-hydroxysuccinimide sera capable de se fixer de manière covalente à une amine présente une protéine ou un peptide.

Le ligand peut également être marqué par un composé bioluminescent ou fluorescent de manière indirecte, par exemple en introduisant dans le milieu de mesure un anticorps ou un fragment d'anticorps, lui-même lié de manière covalente à un composé accepteur/donneur, ce deuxième anticorps ou fragment d'anticorps reconnaissant de manière spécifique le ligand.

Un autre moyen de marquage indirect très classique consiste à fixer de la biotine sur le ligand à marquer, puis d'incuber ce ligand biotinylé en présence de streptavidine marquée par un composé accepteur/donneur. Des ligands biotinylés appropriés peuvent être préparés par des techniques bien connues de l'homme du métier ; la société Cisbio Bioassays commercialise par exemple de la streptavidine marquée avec un fluorophore dont la dénomination commerciale est « d2 » (réf. 610SADLA).

La sélection du couple de partenaires de BRET pour obtenir un signal de BRET est à la portée de l'homme du métier. Par exemple, des couples donneur-accepteur utilisables pour étudier les phénomènes de BRET sont notamment décrits dans l'article de Dasiel O. Borroto-Escuela (BIOLUMINISCENCE RESONANCE ENERGY TRANSFER (BRET)

METHODS TO STUDY G PROTEIN-COUPLED RECEPTOR-RECEPTOR TYROSINE KINASE HETERORECEPTOR COMPLEXES, Methods Cell Biol. 2013 ; 117: 141-164), auquel l'homme du métier pourra se référer.

*Composés luminescents donneurs*

Dans un mode de réalisation particulier, le composé luminescent donneur est un partenaire de BRET choisi parmi : la Luciférase (luc), Renilla Luciférase (Rluc), les variants de Rénilla Luciférase (Rluc8) et la Firefly Luciférase.

*Composés fluorescents accepteur*

Dans un mode de réalisation particulier, le composé fluorescent accepteur est un partenaire de BRET choisi parmi : les allophycocyanines, les rhodamines, les cyanines, les squaraines, les coumarines, les proflavines, les acridines, les fluorescéines, les dérivés du boron-dipyrrométhène, le nitrobenzoxadiazole, un quantum dot, la GFP, les variants GFP (GFP10, GFP2, eGFP), la YFP, les variants YFP (eYFP, YFP topaz, YFP citrine, YFP venus, YPet), le mOrange, le DsRed.

**Kit**

L'invention concerne également un kit pour la mise en oeuvre de la méthode selon l'invention, comprenant :

- un premier ligand de la sous-unité alpha d'une protéine G (protéine Galpha) marqué par un premier membre d'un couple de partenaires de RET et un second ligand de la protéine Galpha marqué par un second membre du couple de partenaires de RET, lesdits ligands étant capables de se lier spécifiquement, soit individuellement soit en association, à la protéine Galpha qui n'est pas liée au GTP ou au GDP ou au GTP non hydrolysable ou lentement hydrolysable (protéine Galpha vide) ; et
- une source de GDP ou une source de GTP ;
- une notice d'instructions ;

caractérisé en ce que le premier ligand est choisi parmi un anticorps, un fragment d'anticorps, un peptide ou un aptamère et le second ligand est choisi parmi un anticorps, un fragment d'anticorps ou un aptamère.

Dans un mode de réalisation particulier, le kit comprend en outre une protéine Galpha et/ou une préparation membranaire portant un ou plusieurs RCPG et une ou plusieurs protéine(s) G.

Le kit peut également comprendre un ou des tampon(s) de dilution pour les réactifs.

## Exemples

**Protocole pour obtenir des anticorps anti-protéine Galphai1**

La protéine TST-Galphai1 recombinantes (protéine Galphai1 de séquence UniProt P63096-1 tagguée en N-terminal avec le tag TwinStreptag (TST) (IBA) via un linker TEV) a été produite dans des cellules d'insectes Sf9 (infection avec un baculovirus codant ladite protéine) puis purifiée sur une colonne d'affinité via le tag TwinStreptag (TST) (Strep-Tactin Superflow high capacity resin (IBA, Catalogue : 2-1208-002)).

Des souris BALB/c ont été immunisées par injection de la protéine TST-Galphai1 préalablement diluée dans du tampon contenant du GTPgS (HEPES 20mM pH8, NaCl 100mM, MgCl2 3mM, CHAPS 11mM, GTPgS 100$\mu$M). La primo-injection a été suivie de trois rappels à intervalle d'un mois.

Quinze jours après chaque injection, des ponctions sanguines sur les souris ont permis de vérifier la présence d'une réponse immunitaire.

Pour cela, un test de type ELISA a été mis en place. La protéine TST-Galphai1 préalablement diluée à 20$\mu$g/mL dans du tampon contenant du GTPgS (Tris HCl 20mM pH8.5, NaCl 140mM, EDTA 2mM, MgCl2 10mM, BSA 0.1%, GTPgS 1$\mu$M) a été adsorbée via le tag TwinStreptag sur des plaques 96 puits contenant de la Strep-Tactin®XT (IBA, Catalogue : 2-4101-001). Pour cela, 100$\mu$l de protéine ont été ajoutés dans chaque puits puis incubées pendant 2h à 37°C suivi de trois lavages en tampon PBS 1X, 0.05% Tween20.

Les dilutions sérielles d'un facteur 10 à 100 millions des ponctions sanguines ont ensuite été ajoutées à hauteur de 100$\mu$L / puits et incubées pendant 2h à 37°C. Les anticorps non fixés à la protéine ont été éliminés par trois étapes de lavages en tampon PBS 1X, 0.05% Tween20 puis la détection des anticorps fixés a été réalisée à l'aide d'un anticorps secondaire anti-Fc de souris lié à la HRP (horseradish peroxidase) (Sigma #A0168 dilué au 1/10 000 dans du PBS, BSA 0.1%). Après 1h d'incubation à 37°C puis trois lavages en tampon PBS 1X, 0.05% Tween20, la révélation de la HRP a été réalisé par dosage colorimétrique à 450nm suite à l'incubation de son substrat TMB (3,3',5,5'-Tétraméthylbenzidine, Sigma #T0440) pendant 20min à température ambiante sous agitation.

Afin de s'assurer que les anticorps détectés par le test ELISA étaient bien dirigés contre la protéine Galphai1 et non contre le tag TwinStrepTag, les mêmes ponctions ont été testés sur le test ELISA après pré-incubation avec un excès d'une autre protéine orthogonale tagguée avec le TwinStrepTag (SNAPTag-TwinStrpeTag). Ainsi, les anticorps anti tag se fixent sur la protéine orthogonale taguée et donc pas sur la protéine Galphai1 attachée au fond des puits ; auquel cas aucun signal HRP ou une diminution du signal HRP est détecté.

Les souris présentant les meilleurs titres d'anticorps et le moins de baisse de signal dans le cas contrôle anti tag ont été sélectionnées pour l'étape suivante d'hybridation lymphocytaire, aussi appelée fusion. La rate des souris a été récupérée et un mélange des lymphocytes et plasmocystes issus de cette rate a été fusionné *in vitro* avec une lignée cellulaire de myélome en présence d'un catalyseur de la fusion cellulaire du type polyéthylène glycol. Une lignée cellulaire de myélome mutante, manquant l'enzyme HGPRT (Hypoxanthine Guanosin Phosphoribosyl Transferase) a été utilisée pour permettre une sélection des cellules hybrides, appelées hybridomes. Ces cellules ont été cultivées dans un milieu contenant de l'hypoxanthine, de l'aminopterine (methotrexate) et de la thyamine (milieu HAT) pour permettre l'élimination des cellules de myélome non fusionnées et ainsi sélectionner les hybridomes d'intérêt. Les cellules de rate non fusionnées quant à elle meurent puisqu'elles sont incapables de proliférer *in vitro.* Ainsi, seuls les hybridomes ont survécu.

Ces hybridomes ont alors été cultivés dans des plaques de culture. Les surnageants de ces hybridomes ont ensuite été testés pour évaluer leur capacité à produire des anticorps anti protéine Galphai1. Pour cela, un test ELISA comme décrit ci-dessus a été réalisé.

Afin d'évaluer la sélectivité des anticorps entre les différentes formes de la protéine Galpahi1 (forme pleine liée au GDP vs forme pleine liée au GTPgS vs forme vide), le test a été réalisé en parallèle sur des conditions de protéine TST-Galphai1 pré-incubée dans le tampon contenant soit du GDP à 1$\mu$M, soit du GTPgS à 1$\mu$M soit sans nucléotide. Les meilleurs hybridomes ont ensuite été clonés avec une étape de dilution limite afin d'obtenir des clones d'hybridome.

Les clones d'hybridomes d'intérêt ont ensuite été injectés dans des souris (injection intra péritonéale) afin de permettre la production des anticorps en grande quantité dans le liquide d'ascite.

Les anticorps ont ensuite été purifiés par chromatographie d'affinité sur des colonnes avec des résines présentant de la protéine A.

Les anticorps monoclonaux ainsi obtenus ont été marqués avec des sondes fluorescentes par exemple, comme expliqué précédemment, pour être utilisés dans la méthode de l'invention.

## Matériel

- les membranes de cellules exprimant les récepteurs étudiés et la protéine Galphai ont été achetées chez Perkin Elmer. Le tableau ci-dessous liste les fonds cellulaires et références des différents échantillons utilisés :

| Récepteur | Fond cellulaire | Fournisseur | Référence |
|---|---|---|---|
| **Delta Opioïde** | HEK293 | Perkin Elmer | 6110549400UA |
| **Delta Opioïde** | CHO-K1 | Perkin Elmer | RBHODM400UA |
| **Galanine R1 (GALR1)** | HEK293 | Perkin Elmer | 6110537400UA |
| **NOP (ORL-1)** | HEK293 | Perkin Elmer | RBHORLM400UA |
| **5HT1A** | HEK293-EBNA | Perkin Elmer | RBHS1AM400UA |

- l'anticorps anti-Galphai SC13533 a été acheté chez Santa Cruz Biotechnology (référence catalogue SC13533). Cet anticorps lie non spécifiquement les trois formes de la protéine Galpha (protéine Galpha vide, protéine Galpha pleine liée au GDP et protéine Galpha pleine liée au GTP ou GTP non hydrolysable ou lentement hydrolysable).
- les anticorps DSV36S, DSV3S, DSV39S, DSV26S ont été générés par Cisbio Bioassays et sont disponibles auprès de Cisbio Bioassays sur demande (sous les références respectives DSV36S, DSV3S, DSV39S, DSV26S). Les anticorps ont été marqués avec les sondes fluorescentes compatibles pour une détection TR-FRET (accepteur rouge - d2 ou donneur Lumi4Tb). Le peptide KB1753 biotinylé a été généré par Cisbio Bioassays.

- l'anticorps DSV36S lie spécifiquement la forme de la protéine Galpha GTP ou GTP non hydrolysable ou lentement hydrolysable par rapport aux formes de la protéine Galpha vide et GDP
- l'anticorps DSV3S lie spécifiquement la forme de la protéine Galpha vide par rapport aux formes GDP et GTP ou GTP non hydrolysable ou lentement hydrolysable.
- l'anticorps DSV26S lie spécifiquement la forme de la protéine Galpha vide par rapport aux formes GDP et GTP ou

GTP non hydrolysable ou lentement hydrolysable.

- l'anticorps DSV39S lie non spécifiquement les trois formes de la protéine Galpha (protéine Galpha vide, protéine Galpha pleine liée au GDP et protéine Galpha pleine liée au GTP ou GTP non hydrolysable ou lentement hydrolysable).
- le peptide KB1753 lie spécifiquement la forme de la protéine Galpha GTP ou GTP non hydrolysable ou lentement hydrolysable par rapport aux formes de la protéine Galpha vide et GDP.
- le couple de ligands peptide KB1753 / anticorps anti-Galphai SC13533 lie spécifiquement la forme de la protéine Galpha GTP ou GTP non hydrolysable ou lentement hydrolysable par rapport aux formes de la protéine Galpha vide et GDP.
- le couple de ligands anticorps anti-Galphai DSV36S / anticorps anti-Galphai SC13533 lie spécifiquement la forme de la protéine Galpha GTP ou GTP non hydrolysable ou lentement hydrolysable par rapport aux formes de la protéine Galpha vide et GDP.
- le couple de ligands anticorps anti-Galphai DSV39S / anticorps anti-Galphai SC13533 lie spécifiquement la forme de la protéine Galpha GTP ou GTP non hydrolysable ou lentement hydrolysable par rapport aux formes de la protéine Galpha vide et GDP.
- le couple de ligands anticorps anti-Galphai DSV3S / anticorps anti-Galphai DSV36S lie spécifiquement la forme de la protéine Galpha GTP ou GTP non hydrolysable ou lentement hydrolysable par rapport aux formes de la protéine Galpha vide et GDP.
- le couple de ligands anticorps anti-Galphai DSV39S / anticorps anti-Galphai DSV26S lie spécifiquement la forme de la protéine Galpha vide par rapport aux formes de la protéine Galpha GDP et GTP ou GTP non hydrolysable ou lentement hydrolysable.

- la streptavidine marquée avec l'accepteur rouge XL665 a été généré par Cisbio Bioassays (référence catalogue 610SAXLB).
- les nucléotides GTP, GDP et GTP$\gamma$S ont été achetés chez Sigma Aldrich (références catalogues respectives G8877, G7127 et G8634).
- l'agoniste du RCPG Delta Opioïde SNC162 et l'antagoniste du RCPG Delta Opioïde Naltrindole ont été achetés chez Tocris (références catalogues respectives 1529 et 0740).
- les plaques 384 puits Low volume, blanches à fond blanc ont été achetées chez Greiner Bio One (référence Catalogue 784075).

**Méthode**

- Préparation des réactifs :

Tous les réactifs ont été dilués dans du tampon TrisHCl 50mM pH 7,4, MgCl2 10mM, NaCl 10mM, BSA 0,1%. Les membranes ont été préparées 4X pour distribuer 1$\mu$g/puits (hormis autres spécifications). Les nucléotides GDP, GTP ou GTP$\gamma$S et les composés à tester (agonistes ou antagonistes) ont été pré-mixés et préparés 4X pour obtenir les concentrations finales dans les puits mentionnées dans les graphiques. L'ensemble des réactifs anti Galphai utilisés pour la détection ont été préparés 4X pour viser les concentrations finales dans les puits suivantes : sdAbs-d2 (50nM) ; anticorps SC13533-d2 (0.1nM) ; anticorps SC13533-Lumi4Tb - pas selon l'invention (0.25nM) ; anticorps DSV36S-d2 (0.1nM); anticorps DSV36S-Lumi4Tb - pas selon l'invention (0.25nM) ; anticorps DSV3S-d2 (10nM); anticorps DSV39S-d2 (10nM) ; anticorps DSV26S-Lumi4Tb (0.25nM) ; peptide KB1753-Biotine (50nM) en association avec streptavidine-XL665 (SA-XL) (25nM) (pré-incubés 30 minutes à température ambiante avant distribution dans les plaques).

- Distribution des réactifs dans les plaques 384 puits :

  ◦ Membranes exprimant le RCPG et la protéine G : 5$\mu$L
  ◦ Nucléotides (GDP ou GTP ou GTP$\gamma$S) +/- composés à tester (agonistes et/ou antagonistes) : 5$\mu$L
  ◦ Ligand anti Galphai-accepteur (d2 ou XL665) : 5$\mu$L
  ◦ Ligand anti Galphai-donneur (Lumi4Tb) : 5$\mu$L

Le signal non spécifique (bruit de fond de fluorescence) a été mesuré avec des puits ne contenant que les deux réactifs de détection (marqués avec le donneur et l'accepteur), les autres composants ayant été remplacés par leur tampon de dilution.

- Lecture du signal HTRF :

Les plaques ont été incubées à 21°C pendant 3h ou 20h puis le signal HTRF a été mesuré sur le lecteur PHERAstar (BMG Labtech) avec la configuration suivante :

◦ Module : HTRF (Excitation 337nm, Emission 665nm et 620nm)
◦ Excitation : laser, 40 flashs
◦ Fenêtre de lecture : délais : 60μs - Intégration : 400μs

- Traitement du signal :
  A partir des signaux bruts à 665nm et 620nm, le Ratio HTRF a été calculé selon la formule suivante :

$$\text{Ratio HTRF} = \text{Signal à 665nm} / \text{Signal à 620nm} * 10{,}000$$

**Formats d'essais**

La figure 3A illustre la discrimination de la forme protéine Galpha vide de la forme protéine Galpha pleine liée au GDP **(format 1A).** Après activation du RCPG et de la protéine Galpha par un agoniste des RCPG, l'augmentation de la proportion de la forme protéine Galpha vide est détectée spécifiquement à l'aide de deux ligands marqués avec des partenaires de RET. L'activation du RCPG entraine une augmentation de signal de RET.
La Figure 3B illustre la discrimination de la forme protéine Galpha pleine liée GDP de la forme protéine Galpha vide **(format 1B** - qui n'est pas selon l'invention). Après activation du RCPG et de la protéine Galpha par un agoniste des RCPG, la diminution de la proportion de la protéine forme Galpha pleine liée au GDP (due à une augmentation de la proportion de la protéine Galpha vide) est détectée spécifiquement à l'aide de deux ligands marqués avec des partenaires de RET. L'activation du RCPG entraine une diminution de signal de RET.
La figure 3C illustre la discrimination de la forme protéine Galpha vide de la forme protéine Galpha pleine liée au GTP ou liée au GTPγS **(format 2A**). Après activation du RCPG et de la protéine Galpha par un agoniste des RCPG, l'augmentation de la proportion de la forme protéine Galpha vide est détectée spécifiquement à l'aide de deux ligands marqués avec des partenaires de RET. L'activation du RCPG entraine une augmentation de signal de RET.
La figure 3D illustre la discrimination de la forme protéine Galpha pleine liée au GTP ou liée au GTPγS de la forme protéine Galpha vide **(format 2B** - qui n'est pas selon l'invention). Après activation du RCPG et de la protéine Galpha par un agoniste des RCPG, la diminution de la proportion de la forme protéine Galpha pleine liée au GTP ou liée au GTPγS (due à une augmentation de la proportion de la forme protéine Galpha vide) est détectée spécifiquement à l'aide de deux ligands marqués avec des partenaires de RET. L'activation du RCPG entraine une diminution de signal de RET.

**Exemples 1 à 4 - Test d'activation selon le format 2B, qui n'est pas selon l'invention : discrimination de la forme GTP ou GTPvS de la forme vide avec des couples de ligands de détection marqués avec des partenaires de TR-FRET**

Dans un premier temps, la capacité des couples de ligands de détection à détecter spécifiquement la protéine Galpha pleine liée au GTP ou liée au GTPγS vis-à-vis de la protéine Galpha vide a été mise en évidence en utilisant des membranes de cellules HEK293 exprimant le RCPG Delta Opioïde et la protéine Galphai. Les membranes ont été incubées avec un excès de GTPγS (10μM), permettant de charger la protéine G avec le nucléotide (forme protéine Galpha pleine liée au GTPyS), ou avec du tampon seul (forme protéine Galpha vide). La différence de signal TR-FRET (Ratio HTRF) observée entre ces deux conditions montre que les 4 paires de ligands de détection utilisées (Peptide KB1753-biotine/SA-XL / anticorps anti-Galphai SC13533-Lumi4Tb - Exemple 1 / Figure 4A ; anticorps anti-Galphai DSV36S-d2 / anticorps anti-Galphai SC13533-Lumi4Tb - Exemple 2 / Figure 5A; anticorps anti-Galphai DSV39S-d2 / anticorps anti-Galphai SC13533-Lumi4Tb - exemple 3 / Figure 6A ; anticorps anti-Galphai DSV3S-d2 / anticorps anti-Galphai DSV36S -Lumi4Tb - Exemple 4 / Figure 7A) permettent de discriminer la forme protéine Galpha liée au GTPγS de la forme protéine Galpha vide.
Dans un second temps, la capacité d'un agoniste du RCPG à moduler la proportion de protéine Galpha sous forme vide a été testée avec les mêmes membranes et paires de ligands de détection. Les membranes de cellules HEK293 exprimant le RCPG Delta Opioïde et la protéine Galphai ont été incubées avec du GTPγS (10nM) et de l'agoniste du RCPG (SNC162) en concentration croissante. La diminution du signal TR-FRET (Ratio HTRF) engendrée par la stimulation avec l'agoniste signifie que la proportion de forme protéine Galpha vide augmente. Ainsi, le récepteur RCPG activé par son agoniste entraine la sortie du GTPgS de la protéine G qui passe alors sous forme vide et entraîne la diminution du signal TR-FRET. Dans une deuxième condition, l'activation par une concentration fixe d'agoniste du RCPG SNC162 (10nM) a été inhibée par une concentration croissante d'antagoniste du RCPG (Naltrindole). Cette inhibition

d'activation est observée par l'augmentation du signal TR-FRET (Ratio HTRF). Ces résultats sont représentés sur les figures 4B (Exemple 1), 5B (Exemple 2), 6B (Exemple 3) et 7B (Exemple 4, dans lequel la deuxième condition n'a pas été mise en œuvre).

**Exemple 5 - Test d'activation selon le format 1A: discrimination de la forme protéine Galpha vide de la forme protéine Galpha pleine liée au GDP) avec un couple de ligands de détection marqués avec des partenaires de TR-FRET**

Dans un premier temps, la capacité du couple de ligands de détection à détecter spécifiquement la forme protéine Galpha vide vis-à-vis de la forme protéine Galpha pleine liée au GDP a été mise en évidence en utilisant des membranes de cellules HEK293 exprimant le RCPG Delta Opioïde et la protéine Galphai. Les membranes ont été incubées avec un excès de GDP (10μM), permettant de charger la protéine G avec le nucléotide (forme protéine Galpha pleine liée au GDP), ou avec du tampon seul (forme protéine Galpha vide). La différence de signal TR-FRET (Ratio HTRF) observée entre ces deux conditions montre que la paire de ligands de détection utilisée (anticorps anti-Galphai DSV39S-d2 + anticorps anti-Galphai DSV26S-Lumi4Tb -Figure 8A) permet de discriminer la forme protéine Galpha vide de la forme protéine Galpha pleine liée au GDP.

Dans un second temps, la capacité d'un agoniste du RCPG à moduler la proportion de protéine G sous forme vide a été testée avec les mêmes membranes et la même paire de ligands de détection. Les membranes de cellules HEK293 exprimant le RCPG Delta Opioïde et la protéine Galphai ont été incubées avec du GDP (10μM) et de l'agoniste du RCPG (SNC162) en concentration croissante. L'augmentation du signal TR-FRET (Ratio HTRF) engendrée par la stimulation avec l'agoniste signifie que la proportion de protéine G sous forme vide augmente. Ainsi, le récepteur RCPG activé par son agoniste entraine la sortie du GDP de la protéine G qui passe alors sous forme vide et entraine l'augmentation du signal TR-FRET. Dans une deuxième condition, l'activation par une concentration fixe d'agoniste du RCPG SNC162 (10nM) a été inhibée par une concentration croissante d'antagoniste du RCPG (Naltrindole). Cette inhibition d'activation est observée par la diminution du signal TR-FRET (Ratio HTRF). Ces résultats sont représentés sur la figure 8B.

**Exemple 6 - Test d'activation selon le format 2A : discrimination de la forme vide de la forme GTP ou GTPγS) avec un couple de ligands de détection marqués avec des partenaires de TR-FRET**

Dans un premier temps, la capacité du couple de ligands de détection à détecter spécifiquement la forme protéine Galpha vide vis-à-vis de la forme protéine Galpha pleine liée au GTP ou liée au GTPγS a été mise en évidence en utilisant des membranes de cellules HEK293 exprimant le RCPG Delta Opioïde et la protéine Galphai. Les membranes ont été incubées avec un excès de GTPγS (10μM), permettant de charger la protéine G avec le nucléotide (forme protéine Galpha pleine liée au GTPyS), ou avec du tampon seul (forme protéine Galpha vide). La différence de signal TR-FRET (Ratio HTRF) observée entre ces deux conditions montre que la paire de ligands de détection utilisée (anticorps anti-Galphai DSV39S-d2 + anticorps anti-Galphai DSV26S-Lumi4Tb -Figure 9A) permet de discriminer la forme protéine Galpha vide de la forme protéine Galpha pleine liée au GTPγS.

Dans un second temps, la capacité d'un agoniste du RCPG à moduler la proportion de protéine G sous forme vide a été testée avec les mêmes membranes et la même paire de ligands de détection. Les membranes de cellules HEK293 exprimant le RCPG Delta Opioïde et la protéine Galphai ont été incubées avec du GTPγS (10nM) et de l'agoniste du RCPG (SNC162) en concentration croissante. L'augmentation du signal TR-FRET (Ratio HTRF) engendrée par la stimulation avec l'agoniste signifie que la proportion de protéine G sous forme vide augmente. Ainsi, le récepteur RCPG activé par son agoniste entraine la sortie du GTPgS de la protéine G qui passe alors sous forme vide et entraine l'augmentation du signal TR-FRET. Dans une deuxième condition, l'activation par une concentration fixe d'agoniste SNC162 (10nM) est inhibée par une concentration croissante d'antagoniste du RCPG (Naltrindole). Cette inhibition d'activation est observée par la diminution du signal TR-FRET (Ratio HTRF). Ces résultats sont représentés sur la figure 9B.

**Exemple 7 - Validation du test d'activation sur différents récepteurs RCPG et différents fonds cellulaires (sur le format 2B, qui n'est pas selon l'invention : discrimination de la forme protéine Galoha pleine liée au GTP ou liée au GTPγS de la forme protéine Galpha vide avec le couple de ligands de détection marqués avec des partenaires de TR-FRET: Peptide KB1753-biotine/SA-XL / anticorps anti-Galphai SC13533-Lumi4Tb)**

Le principe de détection de l'activation a été validé sur quatre récepteurs RCPG différents (Delta Opioïde, GalanineR1, NOP, 5HT1A) et sur deux fonds cellulaires différents (HEK293 et CHO-K1).

D'une part, des membranes (10μg/puits) de cellules HEK293 exprimant le RCPG Delta Opioïde ou GALR1 (récepteur à la Galanine) ou NOP (Récepteur à la Nociceptine) ou 5HT1A et la protéine Galphai ont été incubées avec

du GTPγS (100nM) seul ou en combinaison avec une concentration fixe saturante (1μM) de l'agoniste du récepteur (SNC162, Galanine, Nociceptine et sérotonine respectivement). Pour les quatre RCPG, la diminution du signal TR-FRET (Ratio HTRF) engendrée par la stimulation avec l'agoniste du récepteur signifie que la proportion de protéine G sous forme vide augmente. Ainsi, le récepteur RCPG activé par son agoniste entraine la sortie du GTPgS de la protéine G qui passe alors sous forme vide et entraine la diminution du signal TR-FRET. Les différences de signaux d'un récepteur à l'autre sur la condition des membranes incubées avec le GTPγS seul peuvent être expliquées par des différences de niveaux d'expression des protéines Galphai dans les différentes préparations membranaires. De même les différences d'amplitude de modulation de signaux observées entre les différents RCPG peuvent être dues à des différences de niveaux d'expression des protéines Galphai ou des récepteurs RCPG.

D'autre part, des membranes (10μg/puits) de cellules HEK293 ou CHO-K1 exprimant le RCPG Delta Opioïde et la protéine Galphai ont été incubées avec du GTPγS (100nM) seul ou en combinaison avec une concentration fixe saturante (1μM) de l'agoniste du récepteur (SNC162). Pour les deux fonds cellulaires, la diminution du signal TR-FRET (Ratio HTRF) engendrée par la stimulation avec l'agoniste signifie que la proportion de protéine G sous forme vide augmente. Ainsi, le récepteur RCPG activé par son agoniste active à son tour la protéine G qui passe alors sous forme vide et entraine la diminution du signal TR-FRET.

Ces résultats sont représentés sur la figure 10.

**Exemple 8 - Comparaison du GTPγS et du GTP pour le test d'activation selon le format 2A (discrimination de la forme protéine Galpha vide de la forme protéine Galoha pleine liée au GTP ou liée au GTPvS) avec le couple de ligands de détection: anticorps anti-Galphai DSV39S-d2 + anticorps anti-Galphai DSV26S-Lumi4Tb**

Des membranes (1μg/puits) de cellules HEK293 exprimant le RCPG Delta Opioïde et la protéine Galphai ont été incubées avec du GTPγS ou du GTP (50, 1000 ou 20000 nM) seuls ou en combinaison avec une concentration fixe saturante (1μM) de l'agoniste du RCPG (SNC162). Pour les deux nucléotides, GTPγS et GTP, l'augmentation du signal TR-FRET (Ratio HTRF) engendrée par la stimulation avec l'agoniste signifie que la proportion de protéine G sous forme vide augmente. Ainsi, le récepteur RCPG activé par son agoniste entraine la sortie du GTP ou du GTPgS de la protéine G qui passe alors sous forme vide et entraine l'augmentation du signal TR-FRET. Si les deux conditions (GTPγS et GTP) fonctionnent pour détecter une activation du RCPG par l'agoniste, l'amplitude de modulation de signal observée avec le GTPγS est significativement meilleures qu'avec le GTP. Ces différences d'amplitude de modulation de signaux peuvent être expliquées par une instabilité de la forme protéine Galpha pleine liée au GTP (i.e hydrolyse du GTP en GDP).

Ces résultats sont représentés sur la figure 11.

**Exemple 9 - Test d'activation selon le format 2B, qui n'est pas selon l'invention : discrimination de la forme Galphai pleine (GTP ou GTPγS) de la forme Galphai vide avec un couple d'anticorps marqués avec des partenaires de TR-FRET (anticorps anti-Galphai SC13533-d2 / anticorps anti-Galphai DSV36S-Lumi4Tb)**

Dans un premier temps, la capacité des couples de ligands de détection à détecter spécifiquement la protéine Galpha pleine liée au GTP ou liée au GTPγS vis-à-vis de la protéine Galpha vide a été mise en évidence en utilisant des membranes de cellules HEK293 exprimant le RCPG Delta Opioïde et la protéine Galphai. Les membranes ont été incubées avec un excès de GTPγS (10μM), permettant de charger la protéine Galpha avec le nucléotide (forme protéine Galpha pleine liée au GTPγS), ou avec du tampon seul (forme protéine Galpha vide). La différence de signal TR-FRET (Ratio HTRF) observée entre ces deux conditions montre que la paire de ligands de détection utilisées (anticorps anti-Galphai DSV36S-Lumi4Tb / anticorps anti-Galphai SC13533-d2) permettent de discriminer la forme protéine Galpha liée au GTPγS de la forme protéine Galpha vide (Figure 12A).

Dans un second temps, la capacité d'un agoniste du RCPG à moduler la proportion de protéine Galpha sous forme vide a été testée avec les mêmes membranes et paires de ligands de détection.

Dans une première condition, les membranes de cellules HEK293 exprimant le RCPG Delta Opioïde et la protéine Galphai ont été incubées avec du GTPγS (10nM) et un agoniste du RCPG (SNC162) en concentration croissante. Une diminution du signal TR-FRET (Ratio HTRF) engendrée par la stimulation avec l'agoniste signifie que la proportion de forme protéine Galpha vide augmente. Ainsi, le récepteur RCPG activé par son agoniste entraine la sortie du GTPgS de la protéine G qui passe alors sous sa forme vide et entraîne la diminution du signal TR-FRET.

Dans une deuxième condition, les membranes de cellules HEK293 exprimant le RCPG Delta Opioïde et la protéine Galphai ont été incubées avec du GTPγS (10nM), une concentration fixe d'un agoniste du RCPG SNC162 (10nM) et une concentration croissante d'un antagoniste du RCPG (Naltrindole). L'activation par l'agoniste est donc inhibée quand la concentration en antagoniste augmente. Cette inhibition d'activation a été observée par l'augmentation du signal TR-FRET (Ratio HTRF).

Les résultats sont représentés sur la Figure 12B.

**Exemples 10 - Test d'activation selon le format 2B, qui n'est pas selon l'invention: discrimination de la forme Galphai pleine (GTP ou GTPvS) de la forme Galohai vide avec des couples de ligands marqués avec des partenaires de TR-FRET.**

Huit couples de ligands ont été utilisés :

- sdAb anti-Galphai F11 (SEQ ID NO : 2) / anticorps anti-Galphai SC13533;
- sdAb anti-Galphai F4 (SEQ ID NO : 3) / anticorps anti-Galphai SC13533;
- sdAb anti-Galphai G6 (SEQ ID NO : 4) / anticorps anti-Galphai SC13533;
- sdAb anti-Galphai B11 (SEQ ID NO : 5) / anticorps anti-Galphai SC13533;
- sdAb anti-Galphai F9 (SEQ ID NO : 6) / anticorps anti-Galphai SC13533;
- sdAb anti-Galphai G7 (SEQ ID NO : 7) / anticorps anti-Galphai SC13533;
- sdAb anti-Galphai A10 (SEQ ID NO : 8) / anticorps anti-Galphai DSV36S;
- sdAb anti-Galphai E2 (SEQ ID NO : 9) / anticorps anti-Galphai SC13533.

Dans un premier temps, la capacité des couples de ligands à détecter spécifiquement la protéine Galphai pleine liée au GTP ou liée au GTPγS vis-à-vis de la protéine Galphai vide a été mise en évidence en utilisant des membranes de cellules HEK293 exprimant le RCPG Delta Opioïde et la protéine Galphai. Les membranes ont été incubées avec un excès de GTPγS (10μM), permettant de charger la protéine Galpha avec le nucléotide (forme protéine Galpha pleine liée au GTPγS), ou avec du tampon seul (forme protéine Galpha vide). La différence de signal TR-FRET (Ratio HTRF) observée entre ces deux conditions montre que les 8 couples de ligands de détection utilisées permettent de discriminer la forme protéine Galpha pleine liée au GTPγS de la forme protéine Galpha vide (sdAb anti-Galphai F11-d2 / anticorps anti-Galphai SC13533-Lumi4Tb - Figure 13A ; sdAb anti-Galphai F4-d2 / anticorps anti-Galphai SC13533-Lumi4Tb - Figure 14A ; sdAb anti-Galphai G6-d2 / anticorps anti-Galphai SC13533-Lumi4Tb - Figure 15A ; sdAb anti-Galphai B11-d2 / anticorps anti-Galphai SC13533-Lumi4Tb - Figure 16A ; sdAb anti-Galphai F9-d2 / anticorps anti-Galphai SC13533-Lumi4Tb - Figure 17A ; sdAb anti-Galphai G7-d2 / anticorps anti-Galphai SC13533-Lumi4Tb - Figure 18A ; sdAb anti-Galphai A10-d2 / anticorps anti-Galphai DSV36S-Lumi4Tb - Figure 19A ; sdAb anti-Galphai E2-d2 / anticorps anti-Galphai SC13533-Lumi4Tb - Figure 20A).

Dans un second temps, la capacité d'un agoniste du RCPG à moduler l'activation d'un RCPG, en mesurant la proportion de protéine Galpha sous forme vide, a été testée avec les membranes de cellules HEK293 exprimant le RCPG Delta Opioïde et la protéine Galphai et les huit couples de ligands précités.

Dans une première condition, les membranes ont été incubées avec du GTPγS (10nM) et un agoniste du RCPG (SNC162) en concentration croissante. La diminution du signal TR-FRET (Ratio HTRF) engendrée par la stimulation avec l'agoniste signifie que la proportion de forme protéine Galpha vide augmente. Le récepteur RCPG activé par son agoniste entraine la sortie du GTPgS de la protéine Galpha qui passe alors sous sa forme vide et entraîne la diminution du signal TR-FRET.

Dans une deuxième condition, les membranes ont été incubées avec du GTPγS (10nM), une concentration fixe d'un agoniste du RCPG SNC162 (10nM) et une concentration croissante d'un antagoniste du RCPG (Naltrindole). L'activation par l'agoniste est donc inhibée quand la concentration en antagoniste augmente. Cette inhibition d'activation a été observée par l'augmentation du signal TR-FRET (Ratio HTRF).

Les résultats sont représentés sur les Figures 13B, 14B, 15B, 16B, 17B, 18B et 19B (La deuxième condition n'a pas été mise en oeuvre à la Figure 19B).

## Revendications

1. Méthode pour déterminer la capacité d'une molécule à moduler l'activation d'un récepteur couplé à une protéine G (RCPG), ladite méthode comprenant les étapes suivantes :

   a) introduction, dans un premier contenant :

      - d'une préparation membranaire portant un ou plusieurs RCPG et une ou plusieurs protéine(s) G,
      - d'une source de GDP ou d'une source de GTP,
      - d'un premier ligand de la sous-unité alpha d'une protéine G (protéine Galpha) marqué par un premier membre d'un couple de partenaires de RET, et d'un second ligand de la protéine Galpha marqué par un second membre du couple de partenaires de RET, lesdits ligands étant capables de se lier spécifiquement, soit individuellement soit en association, à la protéine Galpha qui n'est pas liée au GTP ou au GDP ou au GTP non hydrolysable ou lentement hydrolysable (protéine Galpha vide) ;

b) mesure du signal de RET émis dans le premier contenant ;
c) introduction (i) dans un second contenant, des mêmes réactifs qu'à l'étape a) et de la molécule à tester ou (ii) dans le premier contenant, de la molécule à tester ;
d) mesure du signal de RET émis dans le second contenant ou dans le premier contenant obtenu à l'étape c) ;
e) comparaison des signaux obtenus aux étapes b) et d);

- une augmentation du signal quand on utilise une source de GDP et des ligands capables de se lier spécifiquement à la protéine Galpha vide indiquant que la molécule est un agoniste du RCPG ;
- une diminution du signal quand on utilise une source de GDP et des ligands capables de se lier spécifiquement à la protéine Galpha vide indiquant que la molécule est un agoniste inverse du RCPG ;
- une augmentation du signal quand on utilise une source de GTP et des ligands capables de se lier spécifiquement à la protéine Galpha vide indiquant que la molécule est un agoniste du RCPG ;
- une diminution du signal quand on utilise une source de GTP et des ligands capables de se lier spécifiquement à la protéine Galpha vide indiquant que la molécule est un agoniste inverse du RCPG ;

**caractérisée en ce que** le premier ligand est choisi parmi un anticorps, un fragment d'anticorps, un peptide ou un aptamère et le second ligand est choisi parmi un anticorps, un fragment d'anticorps ou un aptamère.

**2.** Méthode pour déterminer la capacité d'une molécule à moduler l'activation d'un récepteur couplé à une protéine G (RCPG), ladite méthode comprenant les étapes suivantes :

a) introduction, dans un premier contenant :

- d'une préparation membranaire portant un ou plusieurs RCPG et une ou plusieurs protéine(s) G,
- d'une source de GDP ou d'une source de GTP,
- d'un premier ligand de la sous-unité alpha d'une protéine G (protéine Galpha) marqué par un premier membre d'un couple de partenaires de RET, et d'un second ligand de la protéine Galpha marqué par un second membre du couple de partenaires de RET, lesdits ligands étant capables de se lier spécifiquement, soit individuellement soit en association, à la protéine Galpha qui n'est pas liée au GTP ou au GDP ou au GTP non hydrolysable ou lentement hydrolysable (protéine Galpha vide) ;
- d'un agoniste du RCPG ;

b) mesure du signal de RET émis dans le premier contenant ;
c) introduction dans un second contenant, des mêmes réactifs qu'à l'étape a) et de la molécule à tester;
d) mesure du signal de RET émis dans le second contenant obtenu à l'étape c) ;
e) comparaison des signaux obtenus aux étapes b) et d);

- une augmentation du signal quand on utilise une source de GDP et des ligands capables de se lier spécifiquement à la protéine Galpha vide indiquant que la molécule est un agoniste ou un modulateur allostérique positif du RCPG ;
- une augmentation du signal quand on utilise une source de GTP et des ligands capables de se lier spécifiquement à la protéine Galpha vide indiquant que la molécule est un agoniste ou un modulateur allostérique positif du RCPG ;
- une diminution du signal quand on utilise une source de GDP et des ligands capables de se lier spécifiquement à la protéine Galpha vide indiquant que la molécule est un antagoniste ou un modulateur allostérique négatif du RCPG ;
- une diminution du signal quand on utilise une source de GTP et des ligands capables de se lier spécifiquement à la protéine Galpha vide indiquant que la molécule est un antagoniste ou un modulateur allostérique négatif du RCPG ;

**caractérisée en ce que** le premier ligand est choisi parmi un anticorps, un fragment d'anticorps, un peptide ou un aptamère et le second ligand est choisi parmi un anticorps, un fragment d'anticorps ou un aptamère.

**3.** Méthode selon l'une quelconque des revendications 1 ou 2, dans laquelle ladite protéine Galpha est choisie parmi la protéine Galphai1, Galphai2, Galphai3, Galphao1, Galphao2, Galphaq, Galpha12, Galpha13, Galphas, Galphaz, Galphat1, Galphat2, Galpha11, Galpha14, Galpha15, Galpha16 et Galphagus, de préférence choisie parmi la protéine Galphai1, Galphai2 et Galphai3.

**4.** Méthode selon l'une quelconque des revendications 1 à 3, dans laquelle le premier ligand est un peptide et le second ligand est un anticorps ou un fragment d'anticorps.

**5.** Méthode selon l'une quelconque des revendications 1 à 4, dans laquelle le premier ligand et le second ligand sont des anticorps ou des fragments d'anticorps.

**6.** Méthode selon l'une quelconque des revendications 1 à 5, dans laquelle la source de GTP est un GTP non-hydrolysable ou lentement hydrolysable, de préférence choisi parmi le GTPgammaS (GTPγS), GppNHp et GppCp.

**7.** Méthode selon l'une quelconque des revendications 1 à 6, dans laquelle le premier ligand et le second ligand sont chacun marqués par un membre d'un couple de partenaires de RET, l'un des membres du couple étant un composé fluorescent donneur ou luminescent donneur et l'autre membre du couple étant un composé fluorescent accepteur ou un composé accepteur non fluorescent (quencher).

**8.** Méthode selon la revendication 7, dans laquelle le composé fluorescent donneur est un partenaire de FRET choisi parmi : un cryptate d'europium, un chélate d'europium, un chélate de terbium, un cryptate de terbium, un chélate de ruthénium, un quantum dot, les allophycocyanines, les rhodamines, les cyanines, les squaraines, les coumarines, les proflavines, les acridines, les fluorescéines, les dérivés du boron-dipyrrométhène et le nitrobenzoxadiazole.

**9.** Méthode selon l'une quelconque des revendications 7 ou 8, dans laquelle le composé fluorescent accepteur est un partenaire de FRET choisi parmi : les allophycocyanines, les rhodamines, les cyanines, les squaraines, les coumarines, les proflavines, les acridines, les fluorescéines, les dérivés du boron-dipyrrométhène, le nitrobenzoxadiazole, un quantum dot, la GFP, les variants GFP choisis parmi GFP10, GFP2 et eGFP, la YFP, les variants YFP choisis parmi eYFP, YFP topaz, YFP citrine, YFP venus et YPet, le mOrange, le DsRed.

**10.** Méthode selon la revendication 7, dans laquelle le composé luminescent donneur est un partenaire de BRET choisi parmi : la Luciférase (luc), Renilla Luciférase (Rluc), les variants de Rénilla Luciférase (Rluc8) et la Firefly Luciférase.

**11.** Méthode selon l'une quelconque des revendications 7 ou 10, dans laquelle le composé fluorescent accepteur est un partenaire de BRET choisi parmi : les allophycocyanines, les rhodamines, les cyanines, les squaraines, les coumarines, les proflavines, les acridines, les fluorescéines, les dérivés du boron-dipyrrométhène, le nitrobenzoxadiazole, un quantum dot, la GFP, les variants GFP choisis parmi GFP10, GFP2 et eGFP, la YFP, les variants YFP choisis parmi eYFP, YFP topaz, YFP citrine, YFP venus et YPet, le mOrange, le DsRed.

**12.** Kit pour la mise en œuvre de la méthode selon l'une quelconque des revendications 1 à 11 comprenant :

- un premier ligand de la sous-unité alpha d'une protéine G (protéine Galpha) marqué par un premier membre d'un couple de partenaires de RET et un second ligand de la protéine Galpha marqué par un second membre du couple de partenaires de RET, lesdits ligands étant capables de se lier spécifiquement, soit individuellement soit en association, à la protéine Galpha qui n'est pas liée au GTP ou au GDP ou au GTP non hydrolysable ou lentement hydrolysable (protéine Galpha vide) ;
- une notice d'instructions ;
- éventuellement un ou des tampon(s) de dilution pour les réactifs ;
- une source de GDP ou une source de GTP ;
- éventuellement une préparation membranaire portant un ou plusieurs RCPG et/ou une ou plusieurs protéine(s) ; et
- éventuellement une protéine Galpha ;

**caractérisé en ce que** le premier ligand est choisi parmi un anticorps, un fragment d'anticorps, un peptide ou un aptamère et le second ligand est choisi parmi un anticorps, un fragment d'anticorps ou un aptamère.

## Patentansprüche

**1.** Verfahren zum Bestimmen der Fähigkeit eines Moleküls, die Aktivierung eines G-Protein-gekoppelten Rezeptors (GPCR) zu modulieren, wobei das Verfahren die folgenden Schritte umfasst:

a) Einbringen, in einen ersten Behälter:

- eines Membranpräparats, das einen oder mehrere GPCR und ein oder mehrere G-Protein(e) trägt,
- einer GDP-Quelle oder einer GTP-Quelle,
- eines ersten Liganden der Alpha-Untereinheit eines G-Proteins (Galpha-Protein), markiert durch ein erstes Mitglied eines Paares von RET-Partnern, und eines zweiten Liganden des Galpha-Proteins, markiert durch ein zweites Mitglied des Paares von RET-Partnern, wobei die Liganden in der Lage sind, spezifisch entweder einzeln oder in Verbindung an das Galpha-Protein zu binden, das nicht an das GTP oder das GDP oder das GTP, das nicht hydrolisierbar oder langsam hydrolisierbar ist, gebunden ist (leeres Galpha-Protein),

b) Messen des in dem ersten Behälter ausgesendeten RET-Signals,
c) Einbringen (i), in einen zweiten Behälter, der gleichen Reagenzien wie in Schritt a) und des zu testenden Moleküls oder (ii) des zu testenden Moleküls in den ersten Behälter,
d) Messen des in Schritt c) erhaltenen RET-Signals, das in dem zweiten Behälter oder in dem ersten Behälter ausgesendet wird,
e) Vergleichen der in den Schritten b) und d) erhaltenen Signale,

- wobei ein Anstieg des Signals bei Verwendung einer GDP-Quelle und der Liganden, die in der Lage sind, spezifisch an das leere Galpha-Protein zu binden, angibt, dass das Molekül ein GPCR-Agonist ist,
- wobei eine Verringerung des Signals bei Verwendung einer GDP-Quelle und der Liganden, die in der Lage sind, spezifisch an das leere Galpha-Protein zu binden, angibt, dass das Molekül ein inverser GPCR-Agonist ist,
- wobei ein Anstieg des Signals bei Verwendung einer GTP-Quelle und der Liganden, die in der Lage sind, spezifisch an das leere Galpha-Protein zu binden, angibt, dass das Molekül ein GPCR-Agonist ist,
- wobei eine Verringerung des Signals bei Verwendung einer GTP-Quelle und der Liganden, die in der Lage sind, spezifisch an das leere Galpha-Protein zu binden, angibt, dass das Molekül ein inverser GPCR-Agonist ist,

**dadurch gekennzeichnet, dass** der erste Ligand aus einem Antikörper, einem Antikörperfragment, einem Peptid oder einem Aptamer ausgewählt ist und der zweite Ligand aus einem Antikörper, einem Antikörperfragment oder einem Aptamer ausgewählt ist.

**2.** Verfahren zum Bestimmen der Fähigkeit eines Moleküls, die Aktivierung eines G-Protein-gekoppelten Rezeptors (GPCR) zu modulieren, wobei das Verfahren die folgenden Schritte umfasst:

a) Einbringen, in einen ersten Behälter:

- eines Membranpräparats, das einen oder mehrere GPCR und ein oder mehrere G-Protein(e) trägt,
- einer GDP-Quelle oder einer GTP-Quelle,
- eines ersten Liganden der Alpha-Untereinheit eines G-Proteins (Galpha-Protein), markiert durch ein erstes Mitglied eines Paares von RET-Partnern, und eines zweiten Liganden des Galpha-Proteins, markiert durch ein zweites Mitglied des Paares von RET-Partnern, wobei die Liganden in der Lage sind, spezifisch entweder einzeln oder in Verbindung an das Galpha-Protein zu binden, das nicht an das GTP oder das GDP oder das GTP, das nicht hydrolisierbar oder langsam hydrolisierbar ist, gebunden ist (leeres Galpha-Protein),
- eines GPCR-Agonisten,

b) Messen des in dem ersten Behälter ausgesendeten RET-Signals,
c) Einbringen, in einen zweiten Behälter, der gleichen Reagenzien wie in Schritt a) und des zu testenden Moleküls,
d) Messen des in Schritt c) erhaltenen RET-Signals, das in dem zweiten Behälter ausgesendet wird,
e) Vergleichen der in den Schritten b) und d) erhaltenen Signale,

- wobei ein Anstieg des Signals bei Verwendung einer GDP-Quelle und der Liganden, die in der Lage sind, spezifisch an das leere Galpha-Protein zu binden, angibt, dass das Molekül ein GPCR-Agonist oder ein positiver allosterischer GPCR-Modulator ist,
- wobei ein Anstieg des Signals bei Verwendung einer GTP-Quelle und der Liganden, die in der Lage sind, spezifisch an das leere Galpha-Protein zu binden, angibt, dass das Molekül ein GPCR-Agonist oder ein positiver allosterischer GPCR-Modulator ist,
- wobei eine Verringerung des Signals bei Verwendung einer GDP-Quelle und der Liganden, die in der Lage sind, spezifisch an das leere Galpha-Protein zu binden, angibt, dass das Molekül ein GPCR-Antagonist

oder ein negativer allosterischer GPCR-Modulator ist,
- wobei eine Verringerung des Signals bei Verwendung einer GTP-Quelle und der Liganden, die in der Lage sind, spezifisch an das leere Galpha-Protein zu binden, angibt, dass das Molekül ein GPCR-Antagonist oder ein negativer allosterischer GPCR-Modulator ist,

**dadurch gekennzeichnet, dass** der erste Ligand aus einem Antikörper, einem Antikörperfragment, einem Peptid oder einem Aptamer ausgewählt ist und der zweite Ligand aus einem Antikörper, einem Antikörperfragment oder einem Aptamer ausgewählt ist.

**3.** Verfahren nach einem der Ansprüche 1 oder 2, wobei das Galpha-Protein aus dem Protein Galphai1, Galphai2, Galphai3, Galphao1, Galphao2, Galphaq, Galpha12, Galpha13, Galphas, Galphaz, Galphat1, Galphat2, Galpha11, Galpha14, Galpha15, Galpha16 und Galphagus ausgewählt ist, bevorzugt aus dem Protein Galphai1, Galphai2 und Galphai3 ausgewählt ist.

**4.** Verfahren nach einem der Ansprüche 1 bis 3, wobei der erste Ligand ein Peptid ist und der zweite Ligand ein Antikörper oder ein Antikörperfragment ist.

**5.** Verfahren nach einem der Ansprüche 1 bis 4, wobei der erste Ligand und der zweite Ligand Antikörper oder Antikörperfragmente sind.

**6.** Verfahren nach einem der Ansprüche 1 bis 5, wobei die GTP-Quelle ein nicht hydrolisierbares oder langsam hydrolisierbares GTP ist, bevorzugt aus GTPgammaS (GTP$\gamma$S), GppNHp und GppCp ausgewählt.

**7.** Verfahren nach einem der Ansprüche 1 bis 6, wobei der erste Ligand und der zweite Ligand jeweils durch ein Mitglied eines Paares von RET-Partnern markiert sind, wobei eines der Mitglieder des Paares eine fluoreszierende Donatorverbindung oder eine lumineszierende Donatorverbindung ist und das andere Mitglied des Paares eine fluoreszierende Aktzeptorverbindung oder eine nicht-fluoreszierende Akzeptorverbindung (Quencher) ist.

**8.** Verfahren nach Anspruch 7, wobei die fluoreszierende Donatorverbindung ein FRET-Partner ist, der ausgewählt ist aus: einem Europiumkryptat, einem Europiumchelat, einem Terbiumchelat, einem Terbiumkryptat, einem Rutheniumchelat, einem Quantenpunkt, Allophycocyaninen, Rhodaminen, Cyaninen, Squarainen, Cumarinen, Proflavinen, Acridinen, Fluoresceinen, Bor-Dipyrromethen-Derivaten und Nitrobenzoxadiazol.

**9.** Verfahren nach einem der Ansprüche 7 oder 8, wobei die fluoreszierende Akzeptorverbindung ein FRET-Partner ist, der ausgewählt ist aus: Allophycocyaninen, Rhodaminen, Cyaninen, Squarainen, Cumarinen, Proflavinen, Acridinen, Fluoresceinen, Bor-Dipyrromethen-Derivaten, Nitrobenzoxadiazol, einem Quantenpunkt, GFP, GFP-Varianten ausgewählt aus GFP10, GFP2 und eGFP, YFP, YFP-Varianten ausgewählt aus eYFP, YFP-Topas, YFP Citrin, YFP Venus und YPet, mOrange, DsRed.

**10.** Verfahren nach Anspruch 7, wobei die lumineszierende Donatorverbindung ein BRET-Partner ist, der ausgewählt ist aus: Luciferase (luc), Renilla Luciferase (Rluc), Varianten von Renilla Luciferase (Rluc8) und Firefly-Luciferase.

**11.** Verfahren nach einem der Ansprüche 7 oder 10, wobei die fluoreszierende Akzeptorverbindung ein BRET-Partner ist, der ausgewählt ist aus: Allophycocyaninen, Rhodaminen, Cyaninen, Squarainen, Cumarinen, Proflavinen, Acridinen, Fluoresceinen, Bor-Dipyrromethen-Derivaten, Nitrobenzoxadiazol, einem Quantenpunkt, GFP, GFP-Varianten ausgewählt aus GFP10, GFP2 und eGFP, YFP, YFP-Varianten ausgewählt aus eYFP, YFP-Topas, YFP Citrin, YFP Venus und YPet, mOrange, DsRed.

**12.** Kit zur Umsetzung des Verfahrens nach einem der Ansprüche 1 bis 11, umfassend:

- einen ersten Liganden der Alpha-Untereinheit eines G-Proteins (Galpha-Protein), markiert durch ein erstes Mitglied eines Paares von RET-Partnern, und einen zweiten Liganden des Galpha-Proteins, markiert durch ein zweites Mitglied des Paares von RET-Partnern, wobei die Liganden in der Lage sind, spezifisch entweder einzeln oder in Verbindung an das Galpha-Protein zu binden, das nicht an das GTP oder das GDP oder das GTP, das nicht hydrolisierbar oder langsam hydrolisierbar ist, gebunden ist (leeres Galpha-Protein),
- eine Bedienungsanleitung,
- optional einen oder mehrere Verdünnungspuffer für die Reagenzien,
- eine GDP-Quelle oder eine GTP-Quelle,

- optional ein Membranpräparat, das einen oder mehrere GPCR und/oder ein oder mehrere Protein(e) trägt, und
- optional ein Galpha-Protein,

**dadurch gekennzeichnet, dass** der erste Ligand aus einem Antikörper, einem Antikörperfragment, einem Peptid oder einem Aptamer ausgewählt ist und der zweite Ligand aus einem Antikörper, einem Antikörperfragment oder einem Aptamer ausgewählt ist.

## Claims

1. Method for determining the ability of a molecule to modulate the activation of a G protein coupled receptor (GPCR), said molecule comprising the following steps:

   a) introducing, into a first container:

      - a membrane preparation carrying one or more GPCR and one or more G protein(s),
      - a GDP source or a GTP source,
      - a first ligand of the alpha subunit of a G protein (Galpha protein) labelled by a first member of a pair of RET partners, and of a second ligand of the Galpha protein labelled by a second member of the pair of RET partners, said ligands being able to bind specifically, either individually or in association, to the Galpha protein which is not bound to GTP or GDP or to non-hydrolysable or slowly hydrolysable GTP (empty G alpha protein);

   b) measurement of the RET signal emitted in the first container;
   c) introduction (i) into a second container, of the same reagents as in step a) and of the molecule to be tested or (ii) into the first container, of the molecule to be tested;
   d) measurement of the RET signal emitted in the second container or in the first container obtained in step c);
   e) comparing the signals obtained in steps b) and d);

      - an increase in the signal when using a GDP source and ligands able to bind specifically to the empty Galpha protein indicating that the molecule is a GPCR agonist;
      - a decrease in the signal when using a GDP source and ligands able to bind specifically to the empty Galpha protein indicating that the molecule is an inverse GPCR agonist;
      - an increase in the signal when using a GTP source and ligands able to bind specifically to the empty Galpha protein indicating that the molecule is a GPCR agonist;
      - a decrease in the signal when using a GTP source and ligands able to bind specifically to the empty Galpha protein indicating that the molecule is an inverse GPCR agonist;

   **characterised in that** the first ligand is selected from an antibody, an antibody fragment, a peptide or an aptamer and the second ligand is selected from an antibody, an antibody fragment or an aptamer.

2. Method for determining the ability of a molecule to modulate the activation of a G protein-coupled receptor (GPCR), said method comprising the following steps:

   a) introduction, into a first container:

      - a membrane preparation carrying one or more GPCR and one or more G protein(s),
      - a GDP source or a GTP source,
      - a first ligand of the alpha subunit of a G protein (Galpha protein) labelled by a first member of a pair of RET partners, and a second ligand of the Galpha protein labelled by a second member of the pair of RET partners, said ligands being able to bind specifically, either individually or in association, to the Galpha protein which is not bound to GTP or GDP or to non-hydrolysable or slowly hydrolysable GTP (empty Galpha protein);
      - a GPCR agonist;

   b) measurement of the RET signal emitted in the first container;
   c) introduction into a second container of the same reagents as in step a) and the molecule to be tested;
   d) measurement of the RET signal emitted in the second container obtained in step c);

e) comparison of the signals obtained in steps b) and d);

- an increase in the signal when using a GDP source and ligands able to bind specifically to the empty Galpha protein indicating that the moleucle is a positive allosteric agonist or modulator of the GPCR;
- an increase in the signal when using a GTP source and ligands able to bind specifically to the empty Galpha protein indicating that the molecule is a positive allosteric agonist or modulator of the GPCR;
- a decrease in the signal when using a GDP source and ligands able to bind specifically to the empty Galpha protein indicating that the molecule is an agonist or a negative allosteric modulator of the GPCR;
- a decrease in the signal when using a GTP source and ligands able to bind specifically to the empty Galpha protein indicating that the molecule is an antagonist or a negative allosteric modulator of the GPCR;

**characterised in that** the first ligand is selected from an antibody, an antibody fragment, a peptide or an aptamer and the second ligand is selected from an antibody, an antibody fragment or an aptamer.

**3.** Method according to anyone of claims 1 or 2, wherein said Galpha protein is selected from the protein Galphai1, Galphai2, Galphai3, Galphao1, Galphao2, Galphaoq, Galpha12, Galpha13, Galphas, Galpaz, Galphat1, Galphat2, Galpha11, Galpha14, Galpha15, Galpha16 and Galphagus, preferably selected from the protein Galphai1, Galphai2 and Galphai3.

**4.** Method according to anyone of claims 1 to 3, wherein the first ligand is a peptide and the second ligand is an antibody or an antibody fragment.

**5.** Method according to anyone of claims 1 to 4, wherein the first ligand and the second ligand are antibodies or antibody fragments.

**6.** Method according to anyone of claims 1 to 5, wherein the GTP source is a non-hydrolysable or slowly hydrolysable GTP, preferably selected from the GTPgammaS (GTP$\gamma$S), GppNHp and GppCp.

**7.** Method according to anyone of claims 1 to 6, wherein the first ligand and the second ligand are each labelled with one member of a pair of RET partners, one member of the pair being a fluorescent donor or luminescent donor compound and the other member of the pair being a fluorescent acceptor compound or a non-fluorescent acceptor compound (quencher).

**8.** Method according to claim 7, wherein the fluorescent donor compound is a FRET partner selected from: a europium cryptate, a europium chelate, a terbium chelate, a terbium cryptate, a ruthenium chelate, a quantum dot, allophycocyanines, rhodamines, cyanines, squaraines, coumarines, proflavines, acridines, fluoresceins, boron-dipyrromethene derivatives and nitrobenzoxadiazole.

**9.** Method according to anyone of claims 7 or 8, wherein the fluorescent acceptor compound is a FRET partner chosen from: allophycocianins, rhodamines, cyanines, squarines, coumarins, proflavines, acridines, fluoresceins, boron-dipyrromethene derivatives, nitrobenzoxadiazole, a quantum dot, GFP, GFP variants selected from GFP10, GFP2 and eGFP, YFP, YFP variants selected from eYFP, YFP topaz, YFP citrine, YFP venus and YPet, mOrange, DsRed.

**10.** Method according to claim 7, wherein the luminescent acceptor compound is a BRET partner selected from: Luciferase (luc), Renilla Luciferase (Rluc), variants of Renilla Luciferase (Rluc8) and Firefly Luciferase.

**11.** Method according to anyone of claims 7 or 10, wherein the fluorescent acceptor compound is a BRET partner chosen from : allophycocyanines, rhodamines, cyanines, squarines, coumarins, proflavines, acridines, fluoresceins, boron-dipyrromethene derivatives, nitrobenzoxadiazole, a quantum dot, GFP, GFP variants selected from GFP10, GFP2 and eGFP, YFP, YFP variants selected from eYFP, YFP topaz, YFP citrine, YFP venus and YPet, mOrange, DsRed.

**12.** Kit for implementing the method according to anyone of claims 1 to 11 comprising:

- a first ligand of the alpha subunit of a G protein (Galpha protein) labelled by a first member of a pair of RET partners and a second ligand of the Galpha protein labelled by a second member of the pair of RET partners, said ligands being able to bind specifically, either individually or in association, to the Galpha protein which is not bound to the GTP or GDP or non-hydrolysable or slowly hydrolysable GTP (empty Galpha protein);

- an instruction leaflet;
- optionnaly one or more dilution buffer(s) for the reagents;
- a GDP source or a GTP source;
- optionnaly a membrane preparation carrying one or more GPCR and/or one or more protein(s); and
- optionnaly a Galpha protein;

**characterised in that** the first ligand is selected from an antibody, an antibody fragment, a peptide or an aptamer and the second ligand is selected from an antibody, an antibody fragment or an aptamer.

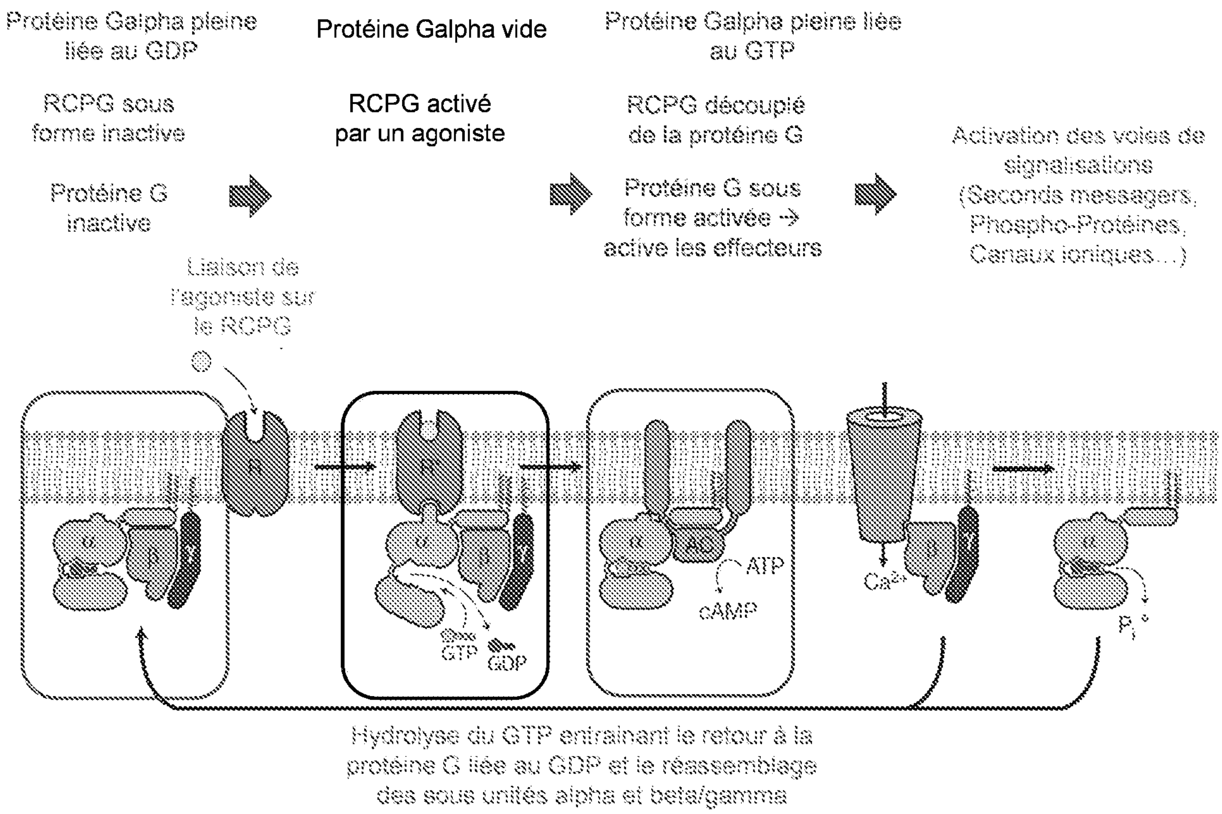
Protéine Galpha pleine liée au GDP
Protéine Galpha vide
Protéine Galpha pleine liée au GTP
RCPG sous forme inactive
RCPG activé par un agoniste
RCPG découplé de la protéine G
Protéine G inactive
Protéine G sous forme activée → active les effecteurs
Activation des voies de signalisations (Seconds messagers, Phospho-Protéines, Canaux ioniques…)
Liaison de l'agoniste sur le RCPG
AC
ATP
cAMP
GTP
GDP
Hydrolyse du GTP entraînant le retour à la protéine G liée au GDP et le réassemblage des sous unités alpha et beta/gamma

**FIG.1**

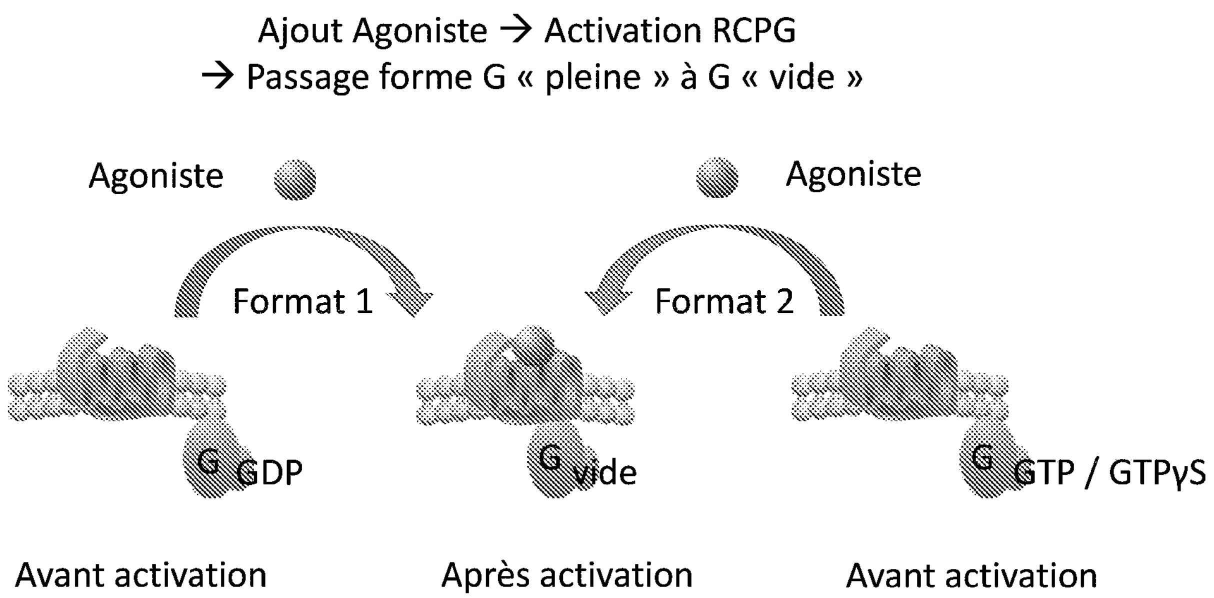
Ajout Agoniste → Activation RCPG
→ Passage forme G « pleine » à G « vide »
Agoniste
Agoniste
Format 1
Format 2
G GDP
G vide
G GTP / GTPγS
Avant activation
Après activation
Avant activation

**FIG.2**

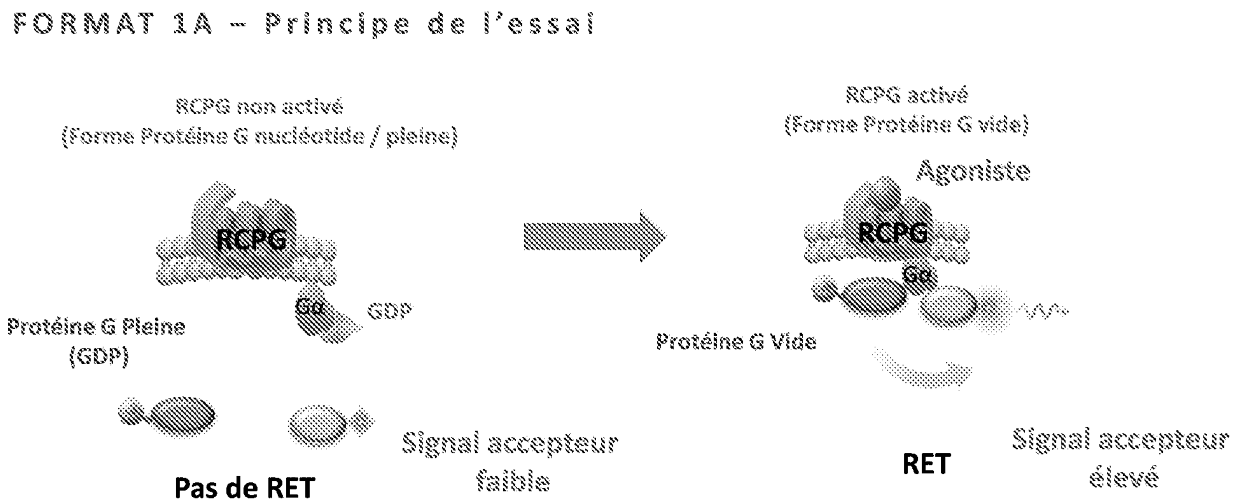
FORMAT 1A – Principe de l'essai
RCPG non activé
(Forme Protéine G nucléotide / pleine)
RCPG
Gα
GDP
Protéine G Pleine
(GDP)
Pas de RET
Signal accepteur
faible
RCPG activé
(Forme Protéine G vide)
Agoniste
RCPG
Gα
Protéine G Vide
RET
Signal accepteur
élevé

Fig.3A

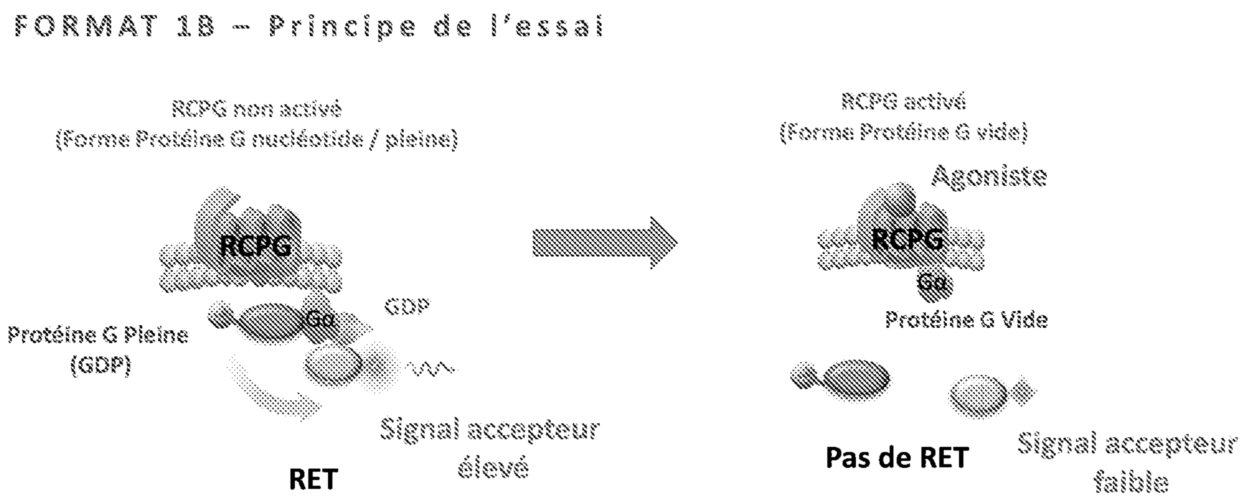
FORMAT 1B – Principe de l'essai
RCPG non activé
(Forme Protéine G nucléotide / pleine)
RCPG
Gα
GDP
Protéine G Pleine
(GDP)
RET
Signal accepteur
élevé
RCPG activé
(Forme Protéine G vide)
Agoniste
RCPG
Gα
Protéine G Vide
Pas de RET
Signal accepteur
faible

Fig.3B

FORMAT 2A – Principe de l'essai

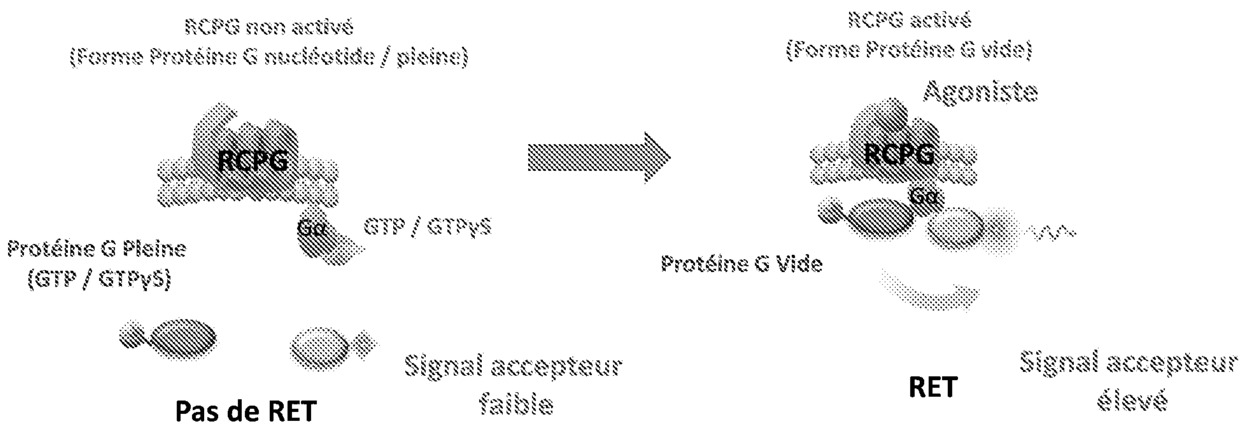


Fig.3C

FORMAT 2B – Principe de l'essai

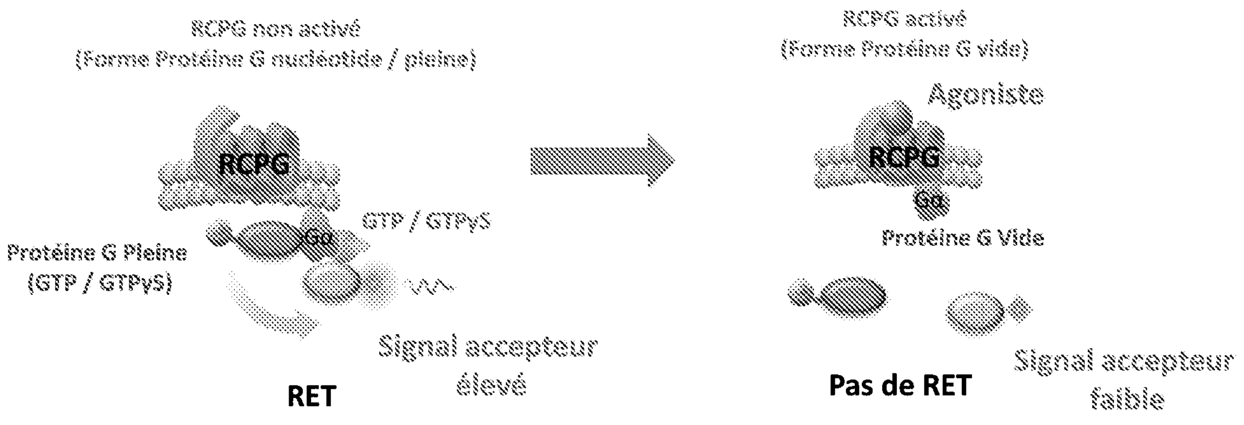


Fig.3D

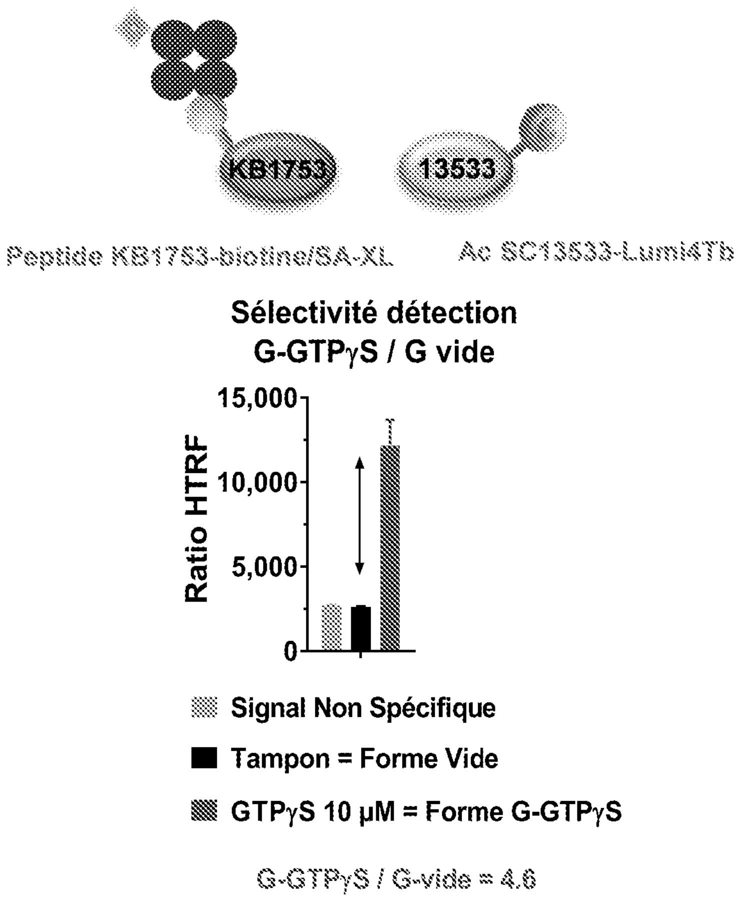
KB1753
13533
Peptide KB1753-biotine/SA-XL
Ac SC13533-Lumi4Tb
Sélectivité détection
G-GTPγS / G vide
Ratio HTRF
15,000
10,000
5,000
0
Signal Non Spécifique
Tampon = Forme Vide
GTPγS 10 µM = Forme G-GTPγS
G-GTPγS / G-vide = 4.6

Fig.4A

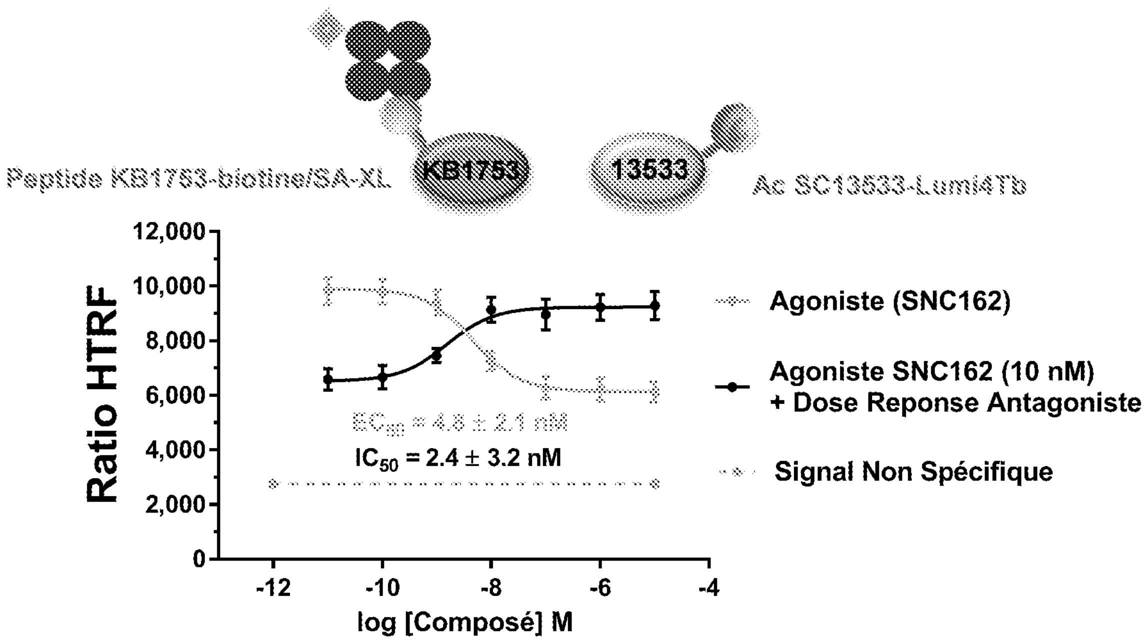
Peptide KB1753-biotine/SA-XL
KB1753
13533
Ac SC13533-Lumi4Tb
Ratio HTRF
12,000
10,000
8,000
6,000
4,000
2,000
0
-12
-10
-8
-6
-4
log [Composé] M
EC50 = 4.8 ± 2.1 nM
IC50 = 2.4 ± 3.2 nM
Agoniste (SNC162)
Agoniste SNC162 (10 nM)
+ Dose Reponse Antagoniste
Signal Non Spécifique

Fig.4B

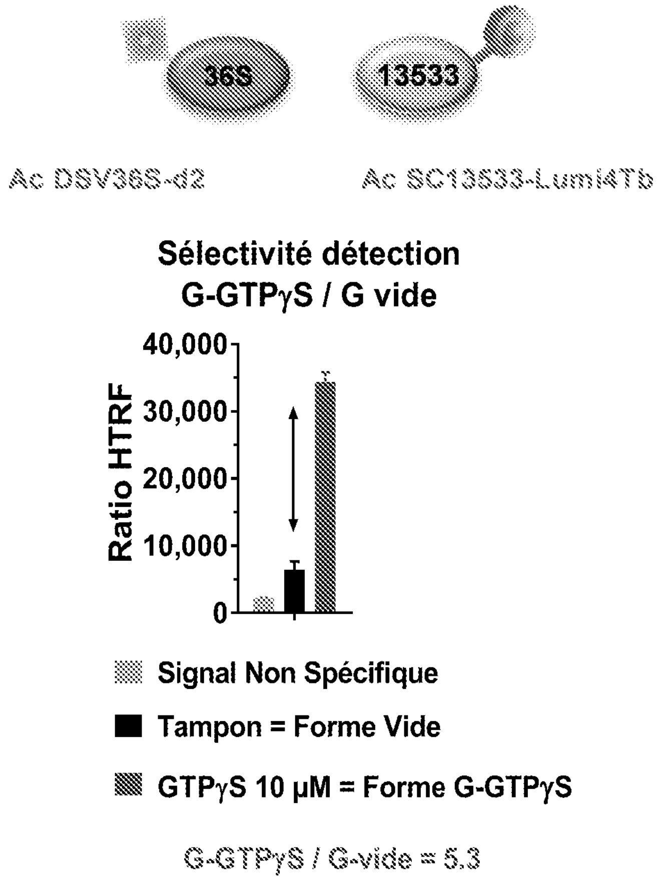
36S
13533
Ac DSV36S-d2
Ac SC13533-Lumi4Tb
Sélectivité détection
G-GTPγS / G vide
Ratio HTRF
40,000
30,000
20,000
10,000
0
Signal Non Spécifique
Tampon = Forme Vide
GTPγS 10 µM = Forme G-GTPγS
G-GTPγS / G-vide = 5.3

Fig.5A

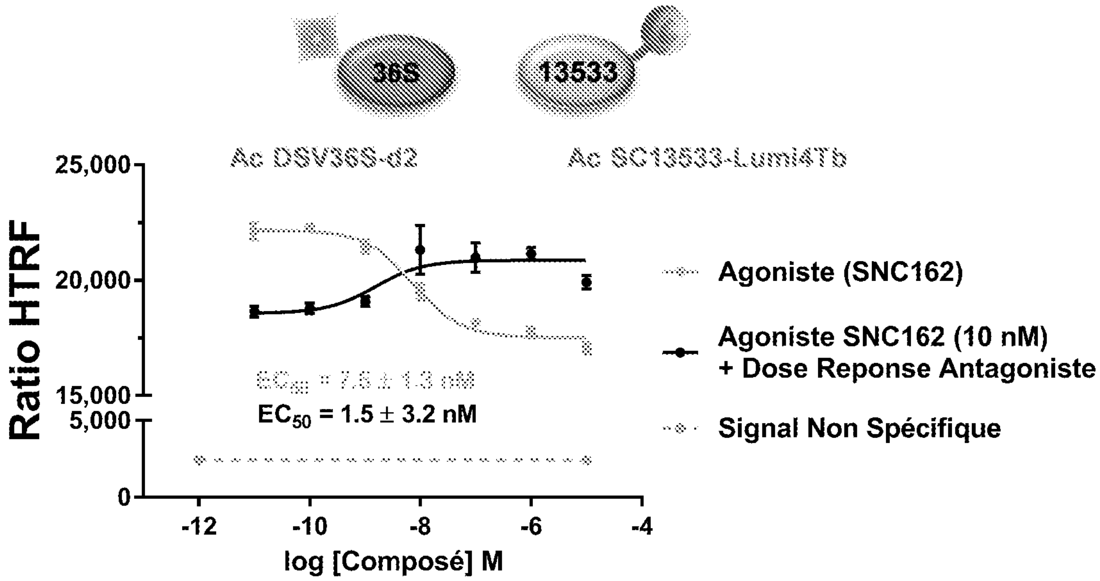
36S
13533
Ac DSV36S-d2
Ac SC13533-Lumi4Tb
Ratio HTRF
25,000
20,000
15,000
5,000
0
EC50 = 7.6 ± 1.3 nM
EC50 = 1.5 ± 3.2 nM
-12
-10
-8
-6
-4
log [Composé] M
Agoniste (SNC162)
Agoniste SNC162 (10 nM)
+ Dose Reponse Antagoniste
Signal Non Spécifique

Fig.5B

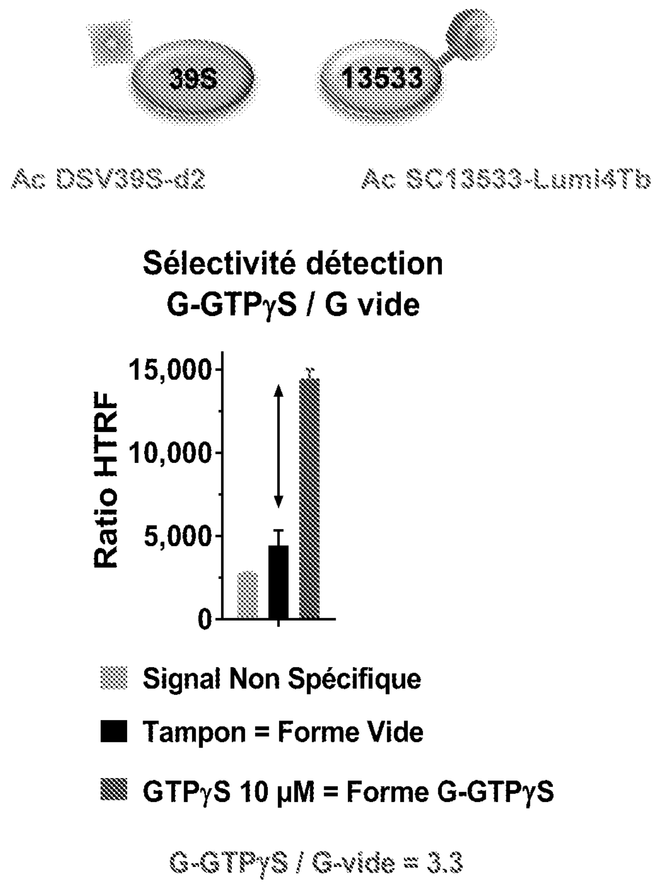
39S
13533
Ac DSV39S-d2
Ac SC13533-Lumi4Tb
Sélectivité détection
G-GTPγS / G vide
Ratio HTRF
15,000
10,000
5,000
0
Signal Non Spécifique
Tampon = Forme Vide
GTPγS 10 µM = Forme G-GTPγS
G-GTPγS / G-vide = 3.3

Fig.6A

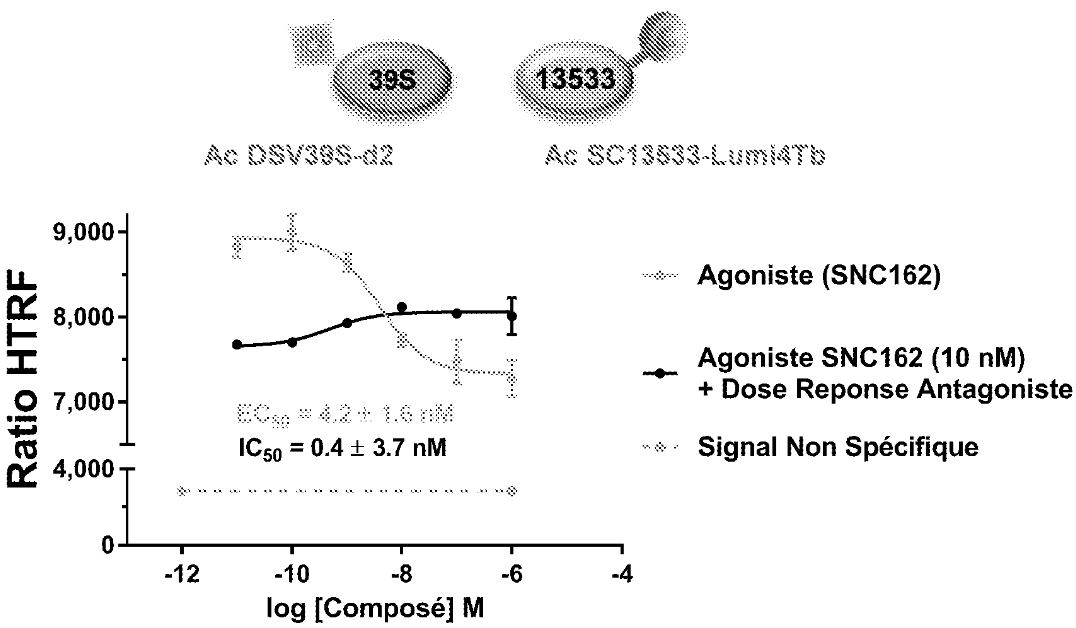
39S
13533
Ac DSV39S-d2
Ac SC13533-Lumi4Tb
Ratio HTRF
9,000
8,000
7,000
4,000
0
-12
-10
-8
-6
-4
log [Composé] M
EC50 = 4.2 ± 1.6 nM
IC50 = 0.4 ± 3.7 nM
Agoniste (SNC162)
Agoniste SNC162 (10 nM)
+ Dose Reponse Antagoniste
Signal Non Spécifique

Fig.6B

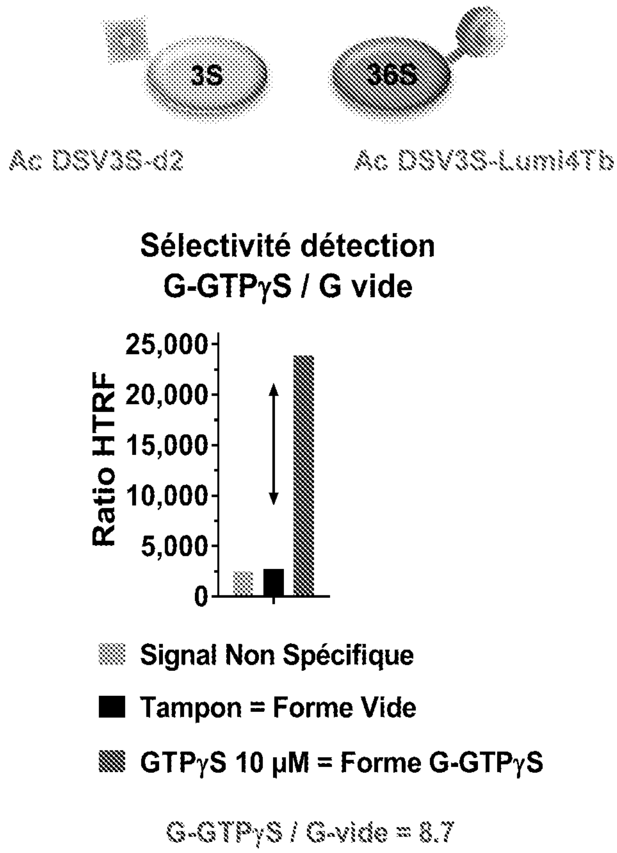
3S
36S
Ac DSV3S-d2
Ac DSV3S-Lumi4Tb
Sélectivité détection
G-GTPγS / G vide
Ratio HTRF
25,000
20,000
15,000
10,000
5,000
0
Signal Non Spécifique
Tampon = Forme Vide
GTPγS 10 µM = Forme G-GTPγS
G-GTPγS / G-vide = 8.7

**Fig.7A**

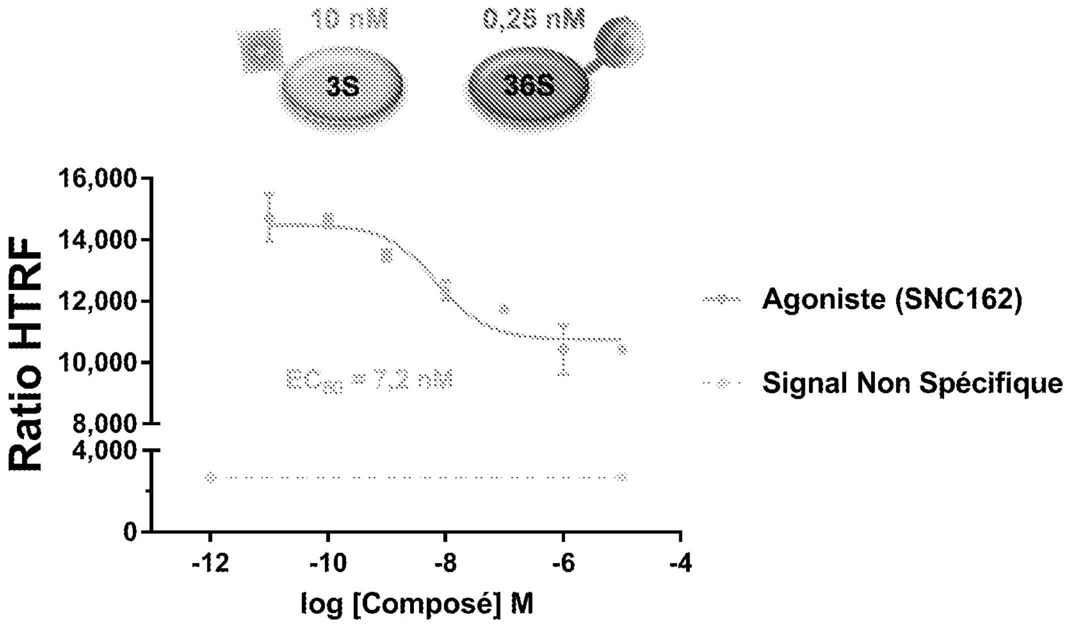
10 nM
0,25 nM
3S
36S
Ratio HTRF
16,000
14,000
12,000
10,000
8,000
4,000
0
$EC_{50}$ = 7,2 nM
-12
-10
-8
-6
-4
log [Composé] M
Agoniste (SNC162)
Signal Non Spécifique

**Fig.7B**

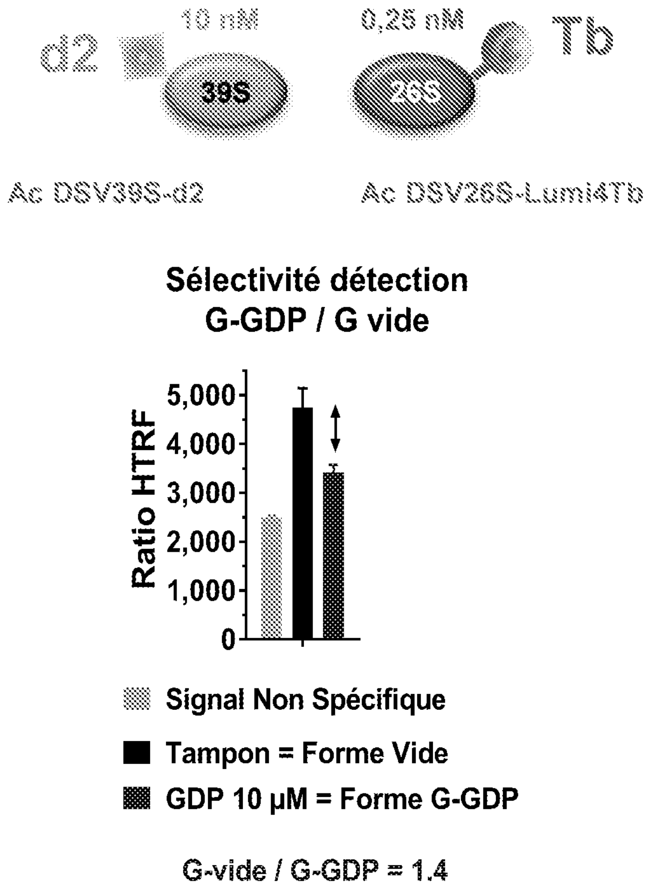
10 nM
0,25 nM
d2
39S
26S
Tb
Ac DSV39S-d2
Ac DSV26S-Lumi4Tb
Sélectivité détection
G-GDP / G vide
Ratio HTRF
5,000
4,000
3,000
2,000
1,000
0
Signal Non Spécifique
Tampon = Forme Vide
GDP 10 µM = Forme G-GDP
G-vide / G-GDP = 1.4

Fig.8A

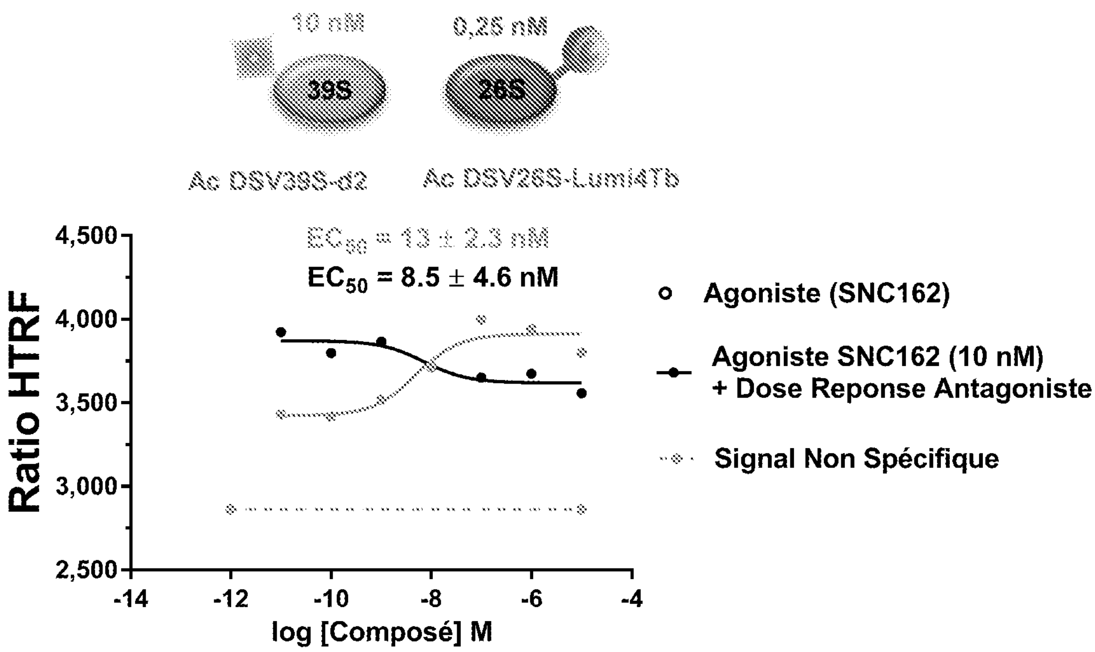
10 nM
0,25 nM
39S
26S
Ac DSV39S-d2
Ac DSV26S-Lumi4Tb
EC50 = 13 ± 2.3 nM
EC50 = 8.5 ± 4.6 nM
Ratio HTRF
4,500
4,000
3,500
3,000
2,500
-14
-12
-10
-8
-6
-4
log [Composé] M
Agoniste (SNC162)
Agoniste SNC162 (10 nM)
+ Dose Reponse Antagoniste
Signal Non Spécifique

Fig.8B

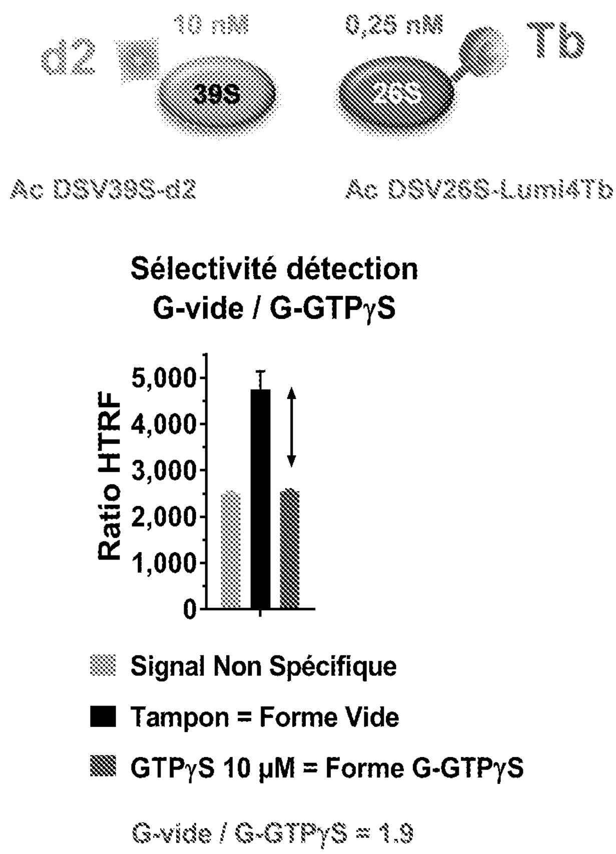
d2
10 nM
39S
0,25 nM
26S
Tb
Ac DSV39S-d2
Ac DSV26S-Lumi4Tb
Sélectivité détection
G-vide / G-GTPγS
Ratio HTRF
5,000
4,000
3,000
2,000
1,000
0
Signal Non Spécifique
Tampon = Forme Vide
GTPγS 10 µM = Forme G-GTPγS
G-vide / G-GTPγS = 1.9

Fig.9A

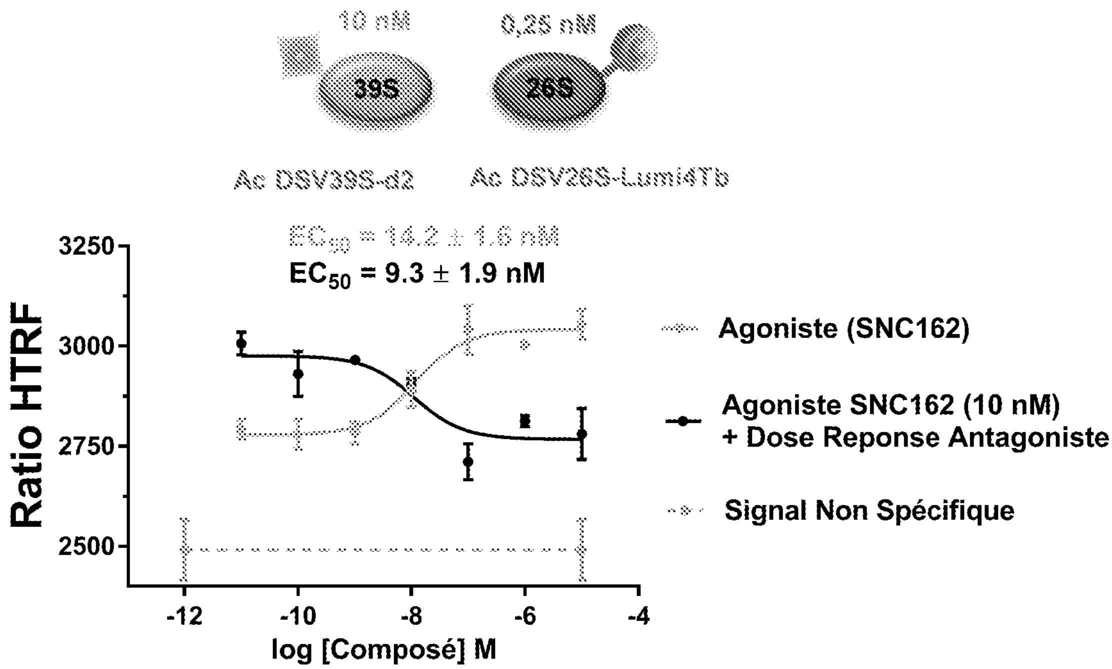
10 nM
39S
0,25 nM
26S
Ac DSV39S-d2
Ac DSV26S-Lumi4Tb
EC50 = 14.2 ± 1.6 nM
EC50 = 9.3 ± 1.9 nM
Ratio HTRF
3250
3000
2750
2500
-12
-10
-8
-6
-4
log [Composé] M
Agoniste (SNC162)
Agoniste SNC162 (10 nM)
+ Dose Reponse Antagoniste
Signal Non Spécifique

Fig.9B

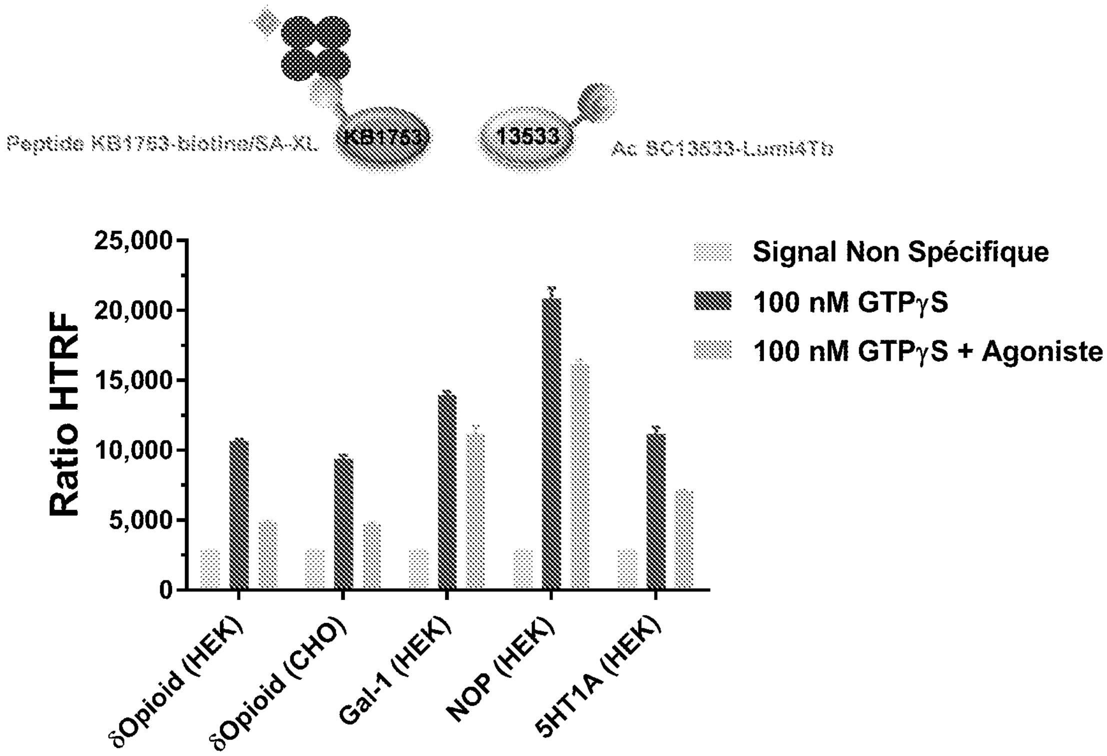
Peptide KB1753-biotine/SA-XL
KB1753
13533
Ac SC13533-Lumi4Tb
Ratio HTRF
25,000
20,000
15,000
10,000
5,000
0
Signal Non Spécifique
100 nM GTPγS
100 nM GTPγS + Agoniste
δOpioid (HEK)
δOpioid (CHO)
Gal-1 (HEK)
NOP (HEK)
5HT1A (HEK)

**Fig.10**

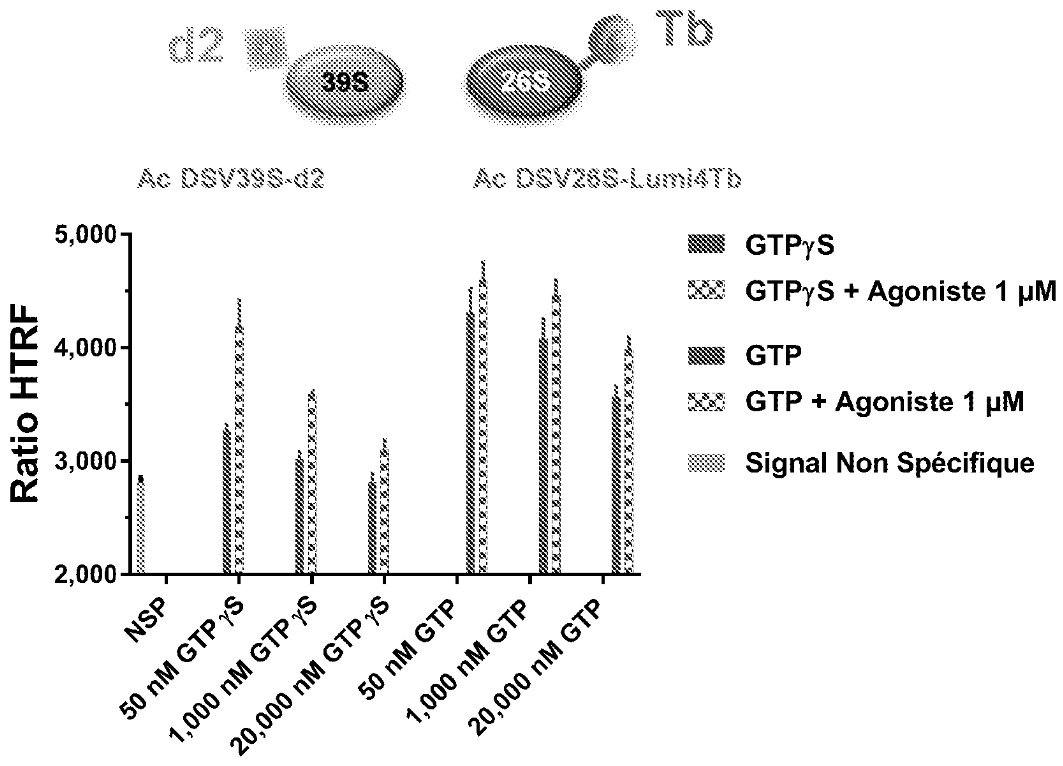
d2
39S
26S
Tb
Ac DSV39S-d2
Ac DSV26S-Lumi4Tb
Ratio HTRF
5,000
4,000
3,000
2,000
GTPγS
GTPγS + Agoniste 1 µM
GTP
GTP + Agoniste 1 µM
Signal Non Spécifique
NSP
50 nM GTPγS
1,000 nM GTPγS
20,000 nM GTPγS
50 nM GTP
1,000 nM GTP
20,000 nM GTP

**Fig.11**

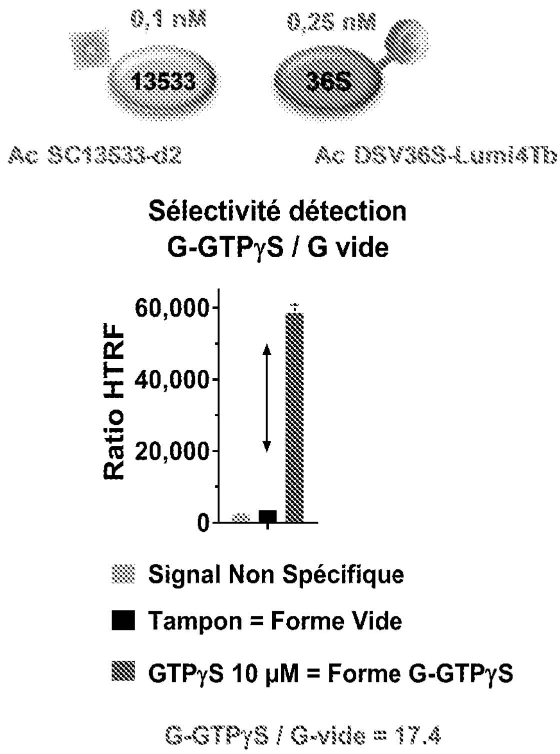


**Figure 12A**

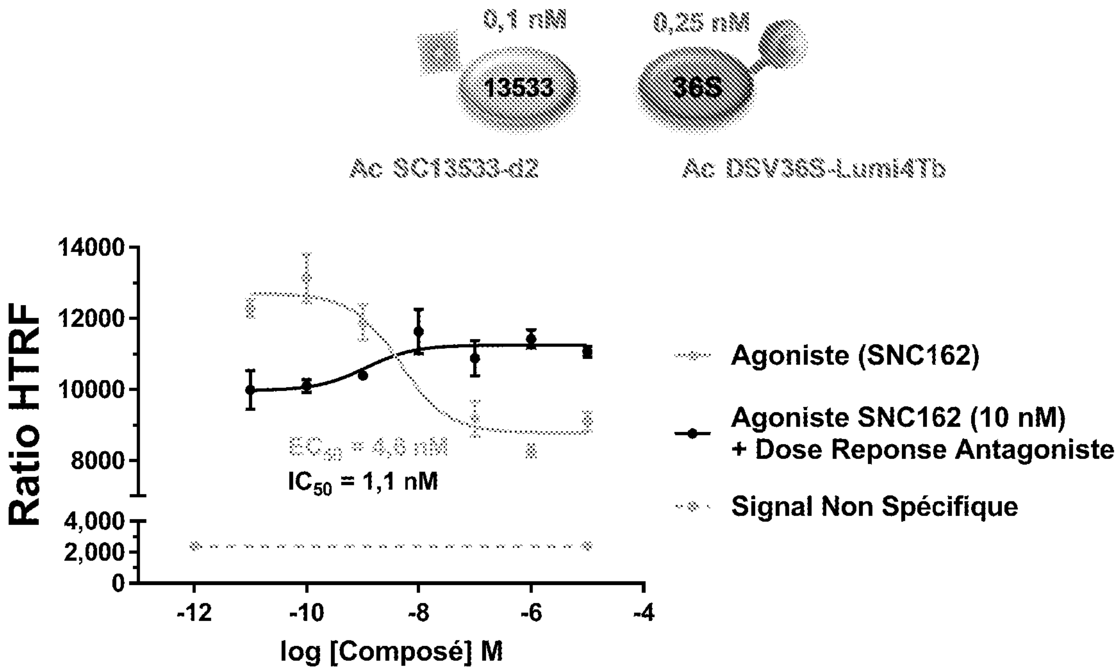


**Figure 12B**

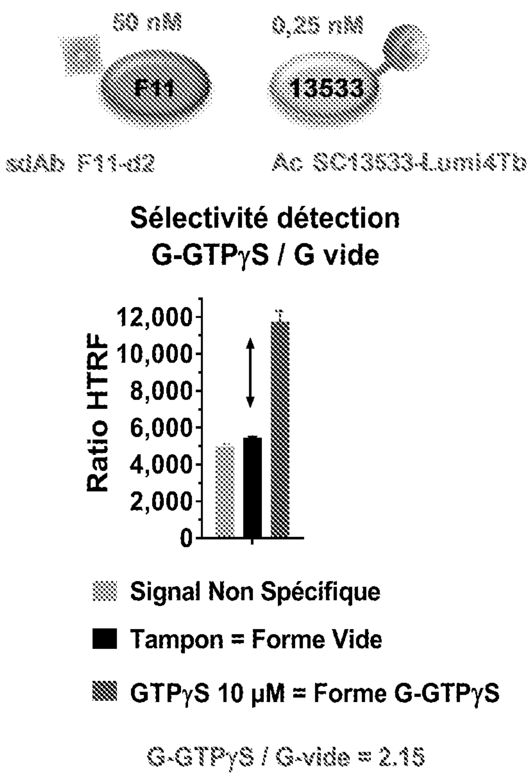


**Figure 13A**

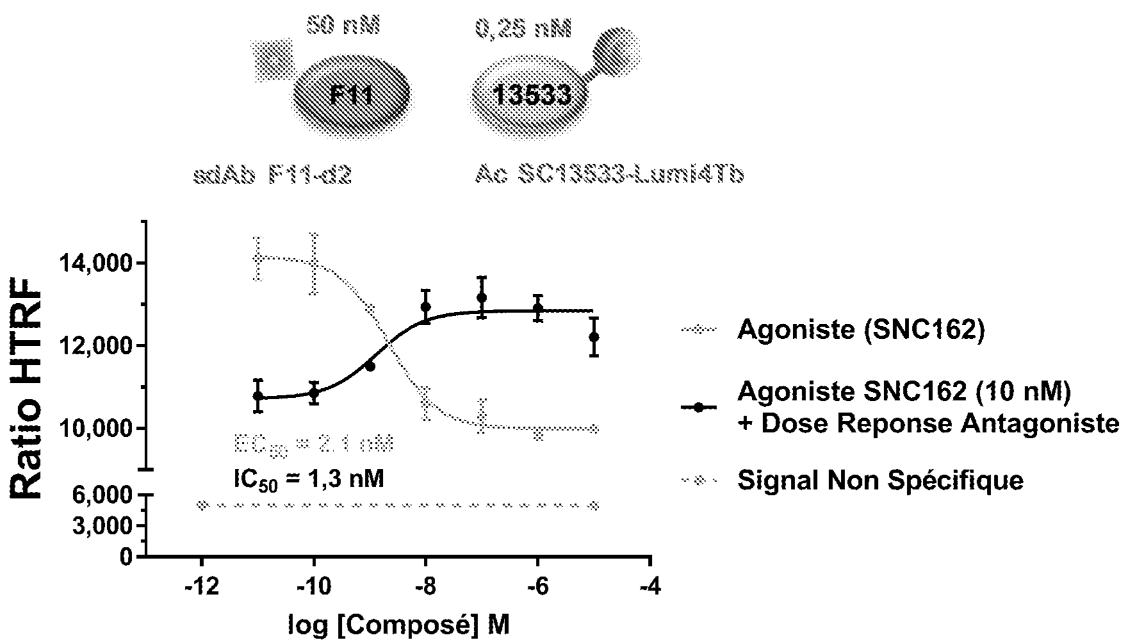


**Figure 13B**

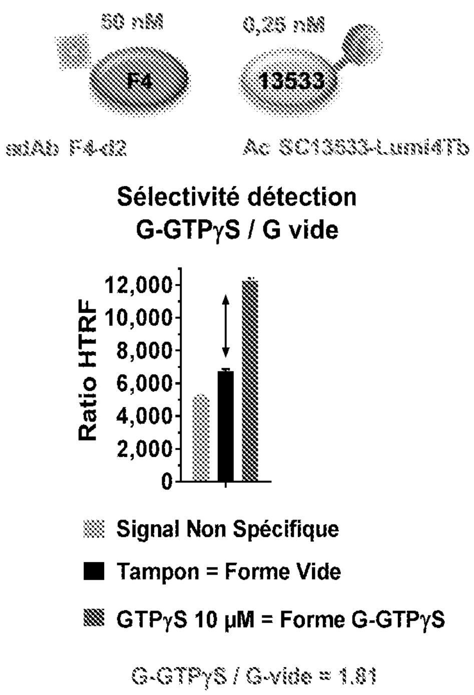


**Figure 14A**

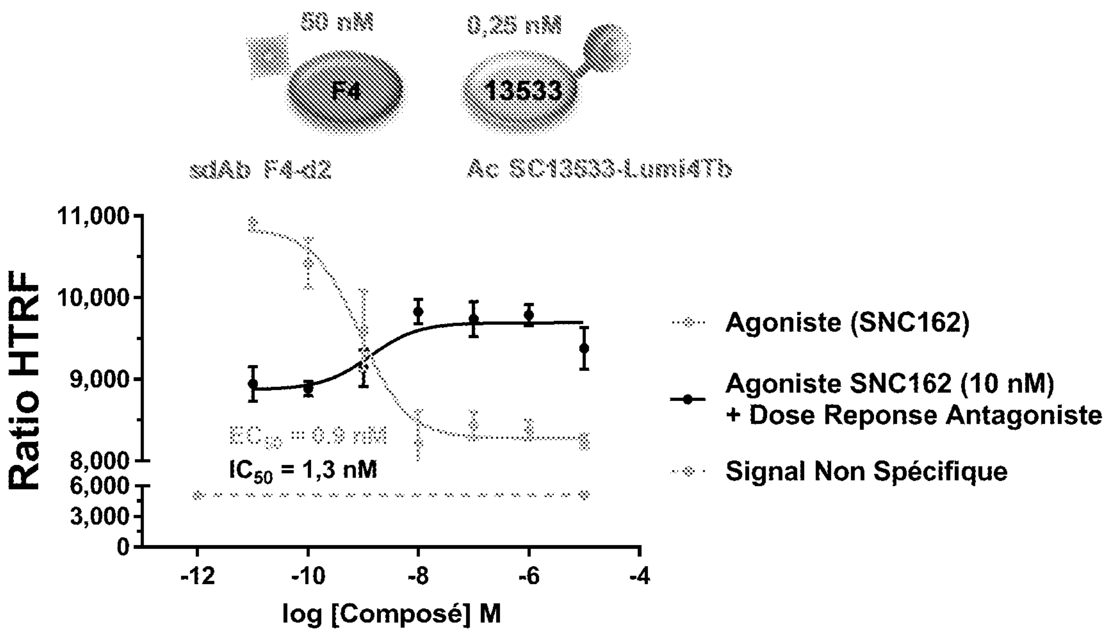


**Figure 14B**

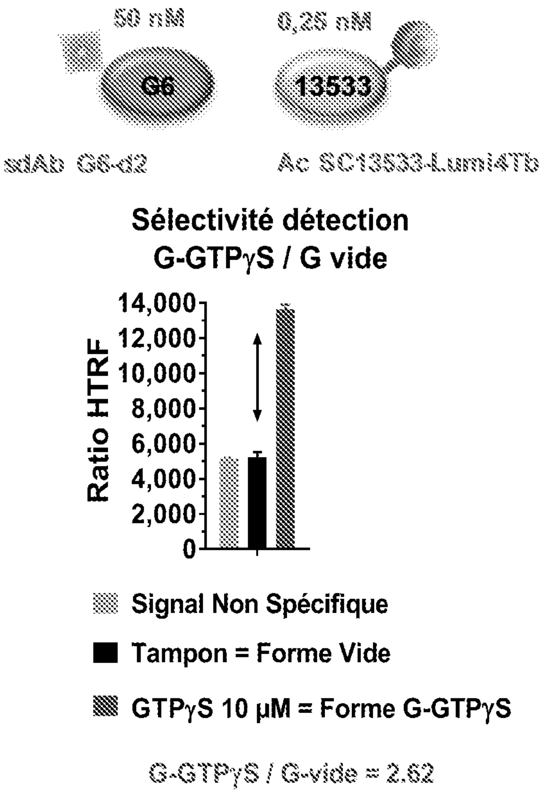


Figure 15A

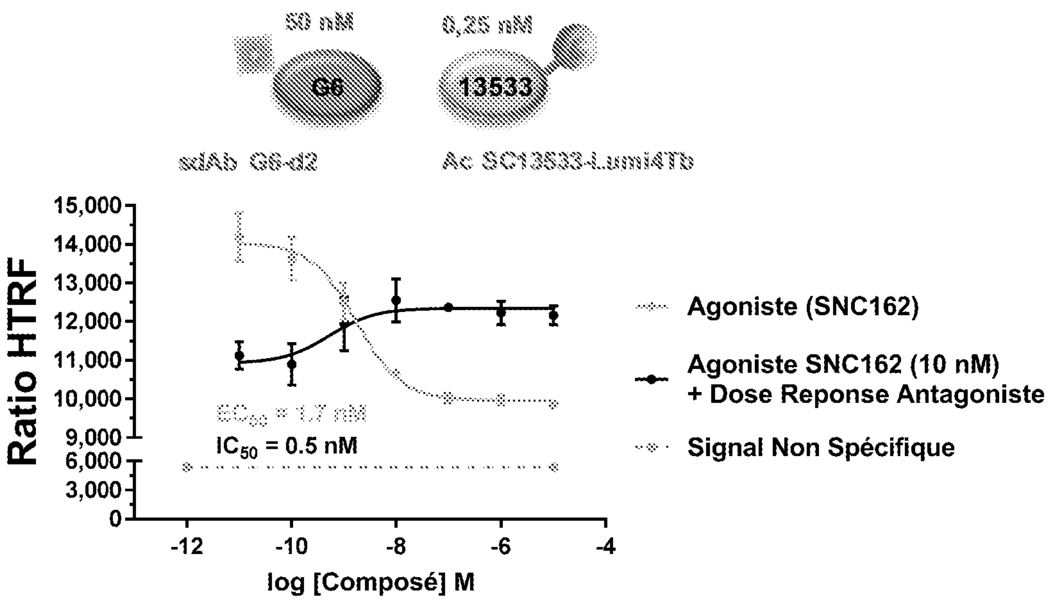


Figure 15B

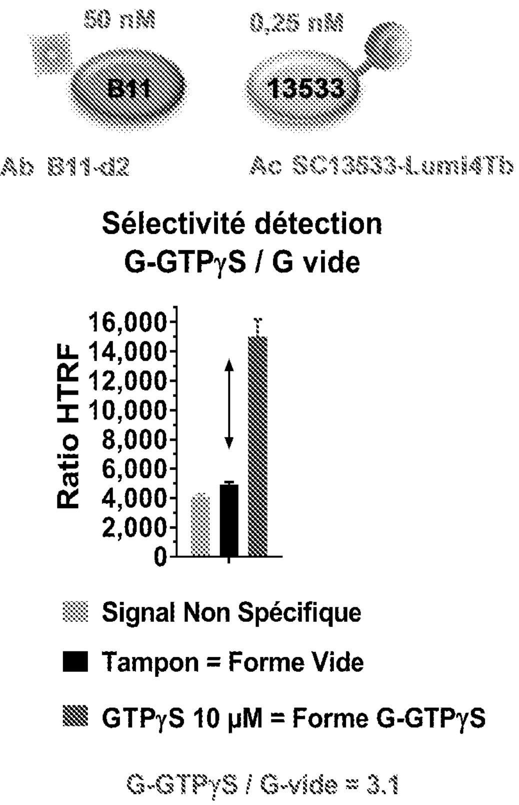


**Figure 16A**

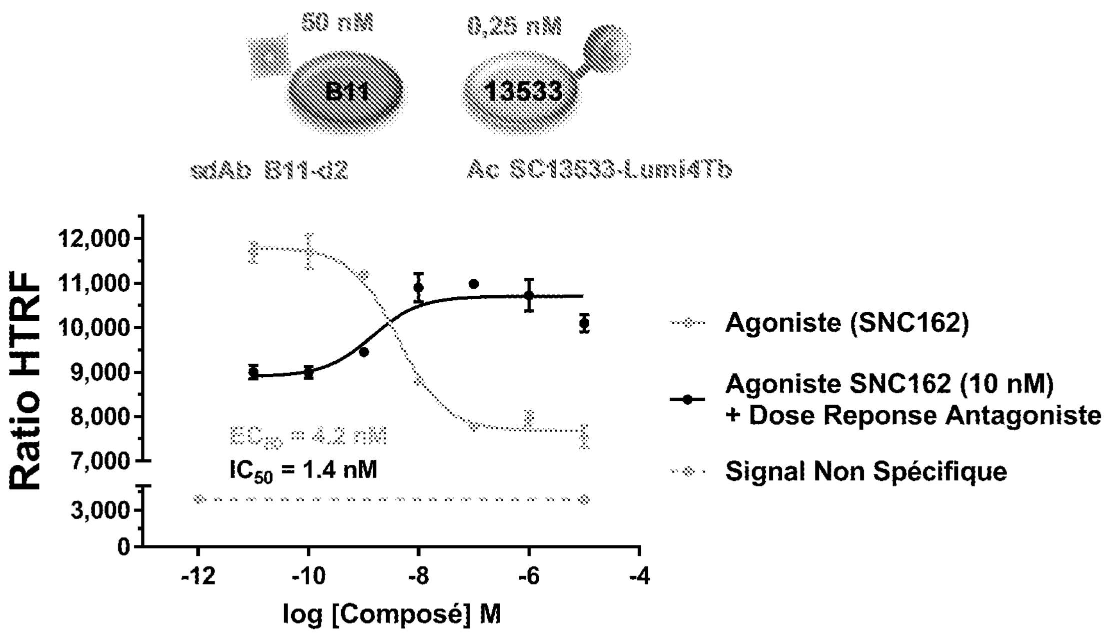


**Figure 16B**

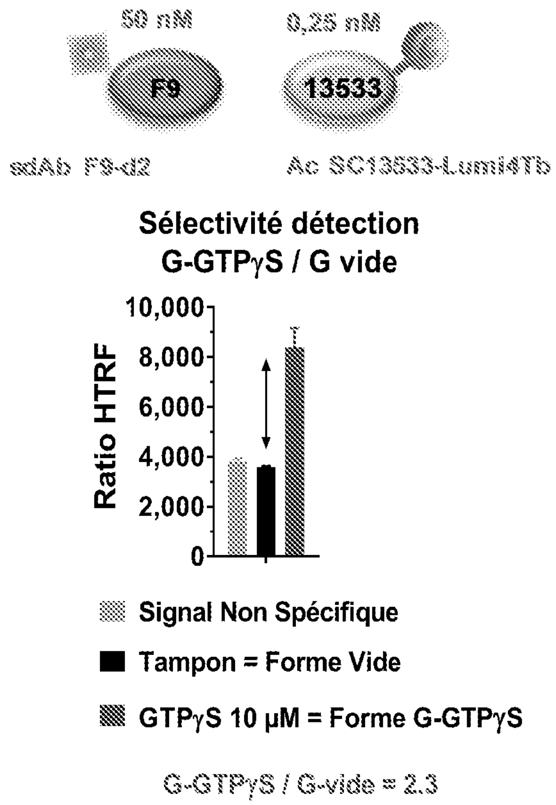


Figure 17A

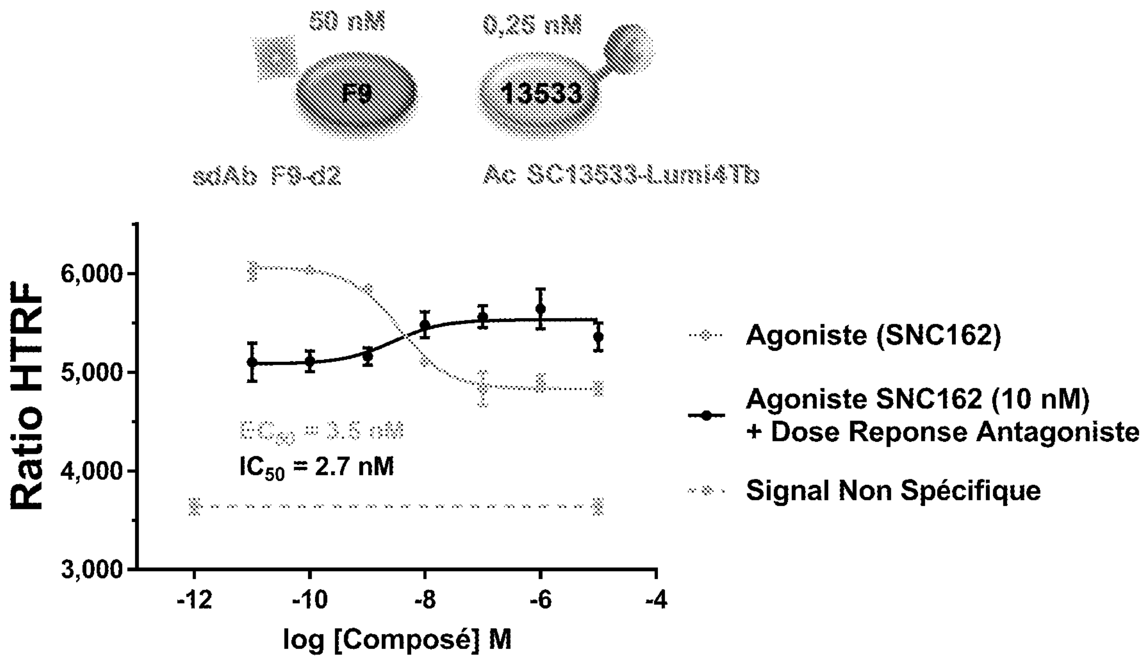


Figure 17B

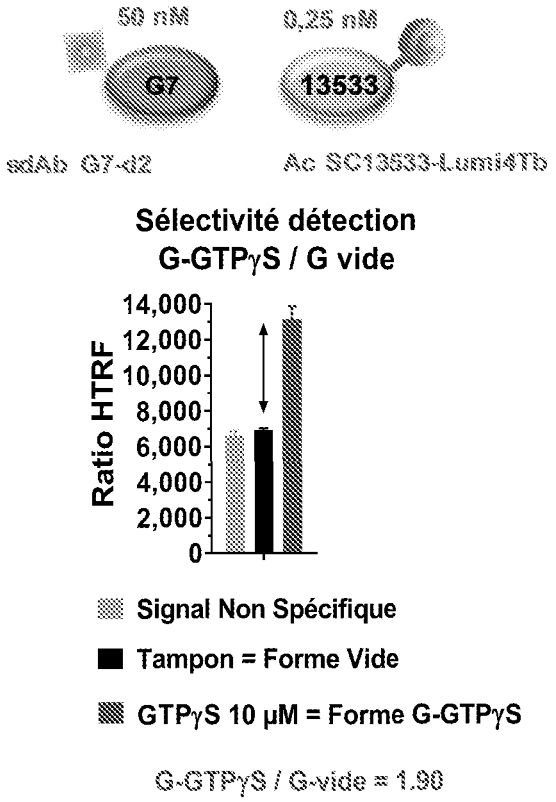


**Figure 18A**

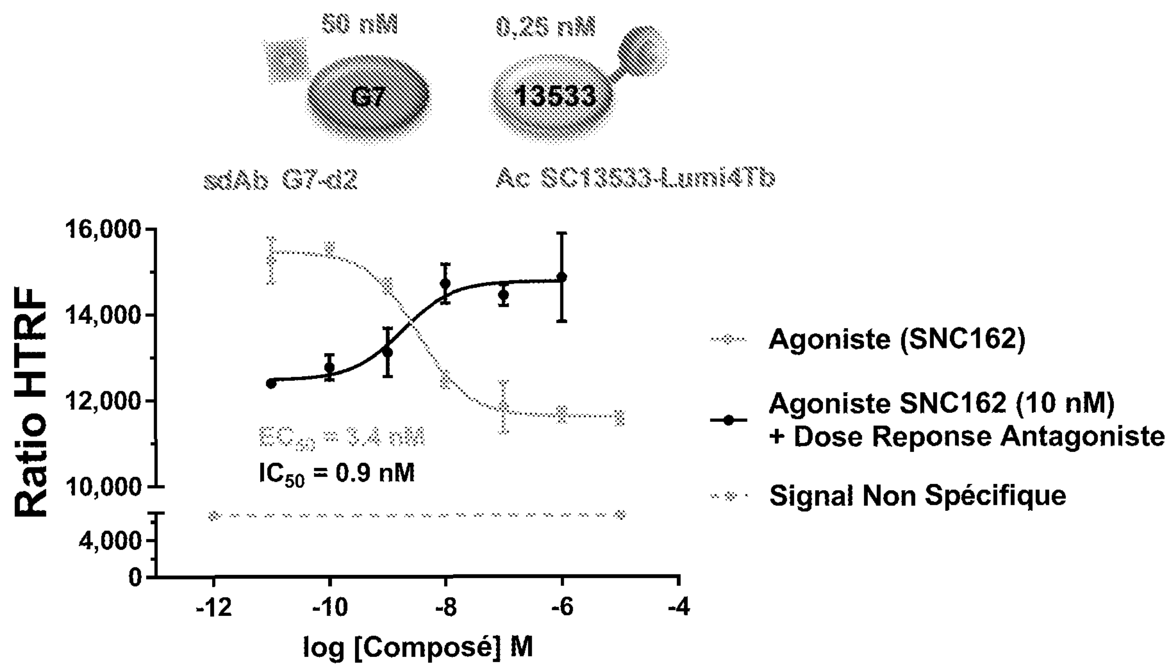


**Figure 18B**

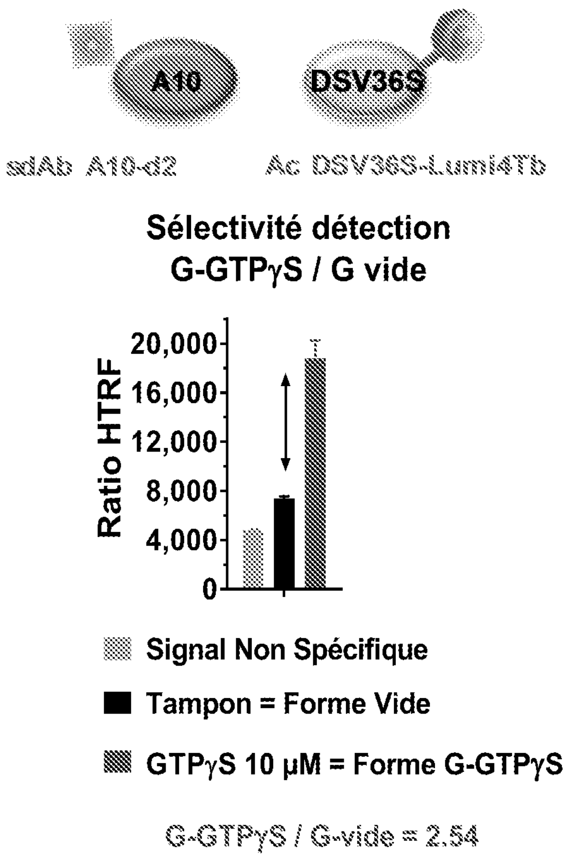


**Figure 19A**

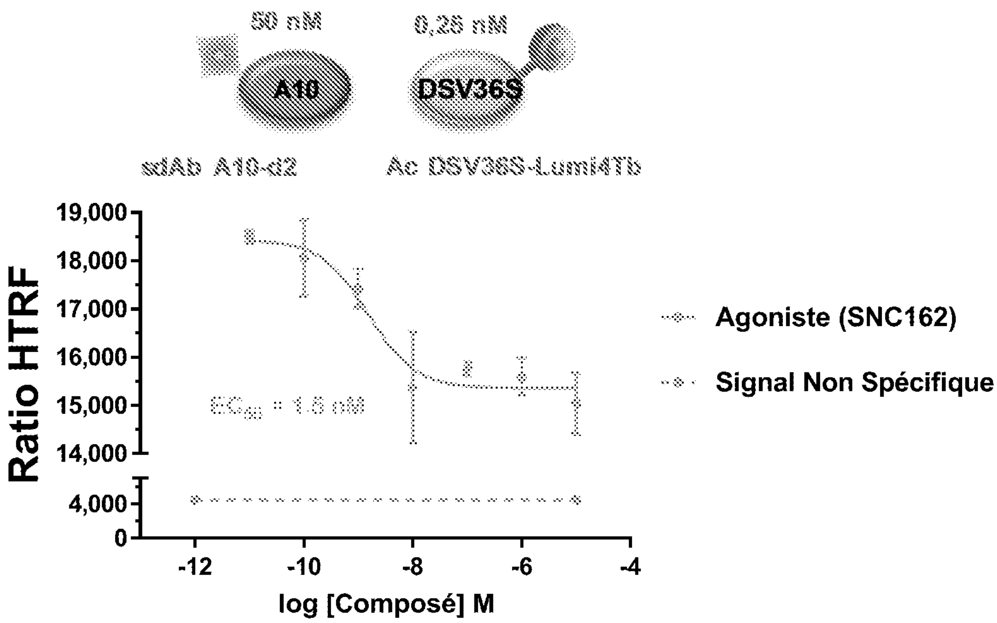


**Figure 19B**

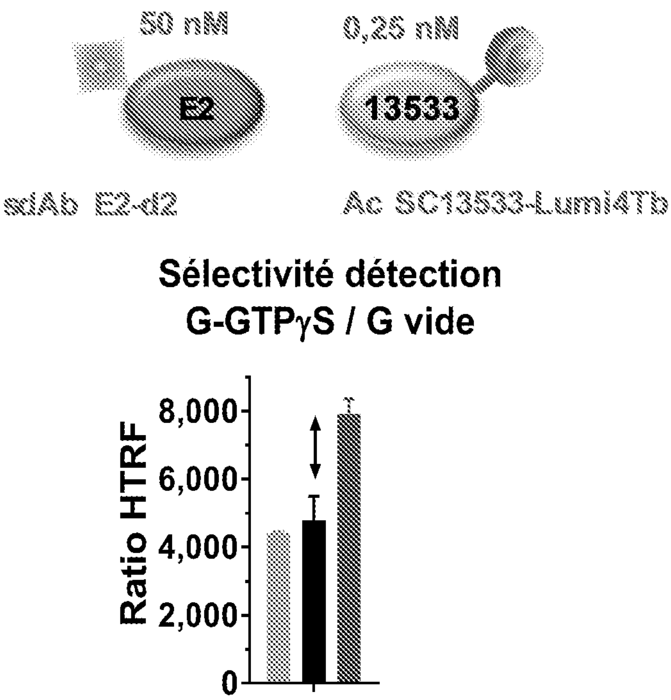


Signal Non Spécifique

Tampon = Forme Vide

GTPγS 10 µM = Forme G-GTPγS

G-GTPγS / G-vide = 1.65

**Figure 20A**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2006086883 A
- WO 2003008435 A
- WO 2006035208 A
- WO 2004035614 A
- EP 0180492 A
- EP 0321353 A
- EP 0601113 A
- WO 0196877 A
- US 4761481 A
- US 5032677 A
- US 5055578 A
- US 5106957 A
- US 5116989 A
- US 4801722 A
- US 4794191 A
- US 4637988 A
- US 4670572 A
- US 4837169 A
- US 4859777 A
- EP 0403593 A
- US 5324825 A
- US 5202423 A
- US 5316909 A
- WO 200910580 A
- EP 1154991 A
- EP 1154990 A
- WO 2008063721 A
- US 5622821 A


### Littérature non-brevet citée dans la description


- **ZHANG et al.** Tools for GPCR drug discovery. *Acta Pharmacologica Sinica,* 2012
- **BUNEMANN et al.** *Proc. Natl. Acad. Sci.,* 2003, vol. 26, 16077-16082
- *CHEMICAL ABSTRACTS,* 37589-80-3
- *CHEMICAL ABSTRACTS,* 148892-91-5
- *CHEMICAL ABSTRACTS,* 10470-57-2
- **POOLE R. et al.** Synthesis and characterisation of highly emissive and kinetically stable lanthanide complexes suitable for usage in cellulo. *Biomol. Chem,* 2005, vol. 3, 1013-1024
- **LATVA et al.** *Journal of Luminescence,* 1997, vol. 75 (2), 149-169
- Bioluminescence Resonance Energy Transfer to Monitor Protein-Protein Interactions, Transmembrane Signaling Protocols. Part of the Methods in Molecular Biology. MIMB, vol. 332, 195-209
- BIOLUMINISCENCE RESONANCE ENERGY TRANSFER (BRET) METHODS TO STUDY G PROTEIN-COUPLED RECEPTOR-RECEPTOR TYROSINE KINASE HETERORECEPTOR COMPLEXES. *Methods Cell Biol.,* 2013, vol. 117, 141-164